(19) Europäisches Patentamt
European Patent Office
Office européen des brevets

(11) Veröffentlichungsnummer: **0 065 479**
A2

# (12) EUROPÄISCHE PATENTANMELDUNG

(21) Anmeldenummer: **82710025.6**

(22) Anmeldetag: **03.05.82**

(51) Int. Cl.³: **C 09 B 62/20**, D 06 P 3/66, D 06 P 3/10, C 07 D 239/28

(30) Priorität: **12.05.81 DE 3118699**

(43) Veröffentlichungstag der Anmeldung: **24.11.82**
**Patentblatt 82/47**

(84) Benannte Vertragsstaaten: **CH DE FR GB IT LI**

(71) Anmelder: **BAYER AG, Zentralbereich Patente, Marken und Lizenzen, D-5090 Leverkusen 1, Bayerwerk (DE)**

(72) Erfinder: **Schallner, Otto, Dr., Jakob-Boehme-Strasse 11, D-5000 Köln 80 (DE)**
Erfinder: **Schündehütte, Karl-Heinz, Prof. Dr., Klief 75, D-5090 Leverkusen 3 (DE)**
Erfinder: **Klauke, Erich, Dr., Eichendorffweg 8, D-5068 Odenthal (DE)**

(54) **Reaktivfarbstoffe, Verfahren zu ihrer Herstellung und ihre Verwendung zum Färben und Bedrucken von hydroxylgruppen- oder stickstoffhaltigen Materialien.**

(57) Die Erfindung betrifft neue Reaktivfarbstoffe der Formel

$$D \left[ -W-\underset{\displaystyle R}{\underset{|}{N}}-A \right]_n \qquad (I)$$

worin

D, W, R und n die im Anmeldungstext angegebene Bedeutung besitzen und

A für den Rest

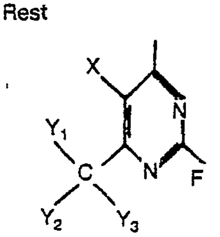

steht, wobei

X = H oder Substituent

$Y_1$ und $Y_2$ gleich oder verschieden sind und für H, Cl oder Br stehen und

$Y_3$ Cl, Br oder F.

Die Erfindung betrifft weiterhin ein Verfahren zur Herstellung der Reaktivfarbstoffe (I) sowie ihre Verwendung zum Färben und Bedrucken von hydroxylgruppen- oder stickstoffhaltigen Materialien.

EP 0 065 479 A2

BAYER AKTIENGESELLSCHAFT 5090 Leverkusen, Bayerwerk
Zentralbereich
Patente, Marken und Lizenzen Er/Klu/kl-c

# Reaktivfarbstoffe, Verfahren zu ihrer Herstellung und ihre Verwendung zum Färben und Bedrucken von hydroxylgruppen- oder stickstoffhaltigen Materialien

Die Erfindung betrifft neue Reaktivfarbstoffe der Formel

$$D\left[W-\underset{R}{\overset{|}{N}}-A\right]_n \qquad (I)$$

worin D für einen organischen Farbstoffrest steht,

W = direkte Bindung oder ein Brückenglied zu einem aromatischen, carbocyclischen oder aromatisch-heterocyclischen Kohlenstoffatom des Restes D,

R = Wasserstoff oder $C_1$-$C_4$-Alkyl

A =

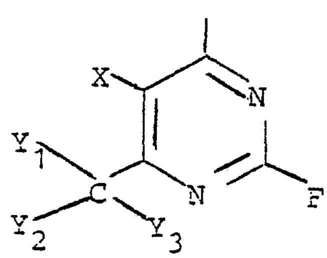

wobei X = Wasserstoff oder Substituent $Y_1$ und $Y_2$ gleich oder verschieden sind und für Wasserstoff, Chlor oder Brom stehen,

und $Y_3$ = Cl, Br oder F

und n = 1 oder 2.

In der Formel für A steht X bevorzugt für Wasserstoff oder Halogen (wie Fluor, Chlor, Brom und Jod) und $Y_1$, $Y_2$ und $Y_3$ haben die oben angegebene Bedeutung.

Insbesondere bevorzugt sind folgende Reste A:

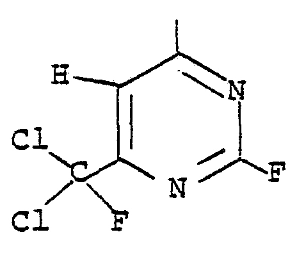

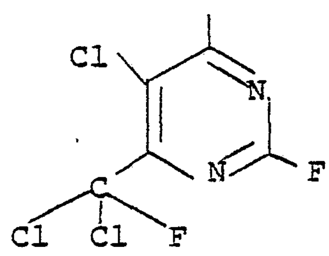

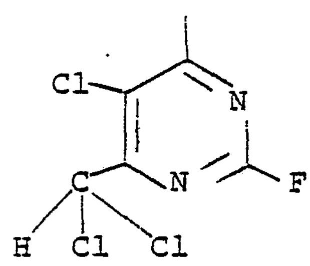

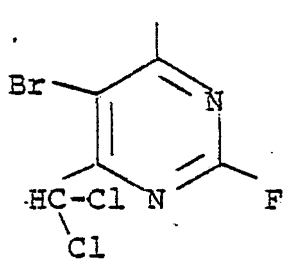

und

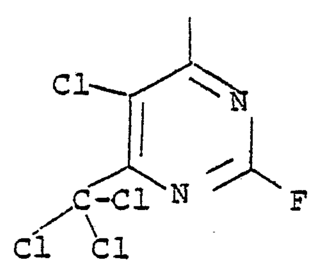

Für die im vorliegenden Anmeldungstext und insbesondere unter den Formeln I bis XXVI aufgeführten Alkyl-, Aryl-, Aralkyl- und hetarylreste gilt:

Unter Alkylgruppen werden dabei insbesondere solche mit 1 - 4 C-Atomen verstanden, die gegebenenfalls Substituenten aufweisen können, beispielsweise Halogen wie Cl und Br, OH, CN.

Unter Arylgruppen werden insbesondere Phenylreste verstanden, die gegebenenfalls Substituenten aufweisen, beispielsweise Halogen wie Cl und Br, $C_1$-$C_4$-Alkyl, $C_1$-$C_4$-Alkoxy, $C_1$-$C_4$-Alkylsulfonyl, $NO_2$, CN, $CF_3$, $SO_3H$, COOH.

Unter Aralkylresten werden insbesondere Benzylreste verstanden, die gegebenenfalls die für die Phenylreste genannten Substituenten aufweisen können.

Geeignete Hetarylreste sind beispielsweise Thiophenyl, Benzthiophenyl, Benzthiazolyl, Benzimidazolyl, Pyridyl, Pyrimidyl, Benzofuranyl, Indolyl, Chinolyl, Carbazyl, Chinoxalyl, Benztriazolyl.

Geeignete Brückenglieder W sind beispielsweise:

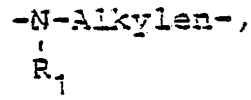

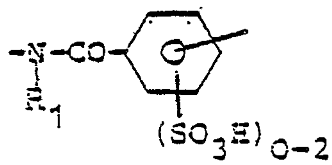

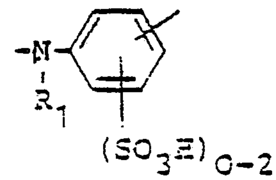

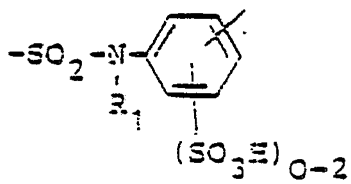

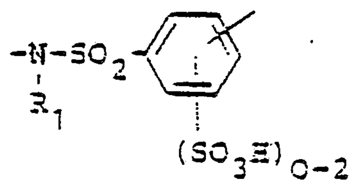

-O-Alkylen-,

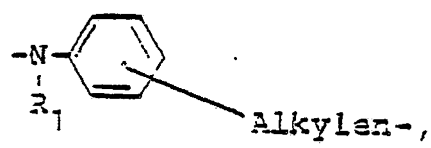

-S-Alkylen-, -Alkylen-, -Alkylen-CO-, -Alkylen-$SO_2$-,

wobei $R_1$ für Wasserstoff oder Alkyl, insbesondere $C_1$-$C_4$-Alkyl steht und Alkylen einen Alkylenrest mit 1 - 4 C-Atomen bedeutet.

Die vorliegende Anmeldung betrifft weiterhin neue Verbindungen der allgemeinen Formel

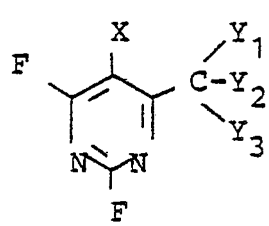

(Ia)

in welcher

X H, Cl oder Br,

$Y_1$,$Y_2$ H, Cl, Br und

$Y_3$ Cl, Br, F

bedeuten.

Die Erfindung betrifft weiterhin ein Verfahren zur Herstellung der Verbindungen der Formel (Ia), welches dadurch gekennzeichnet ist, daß Chlorpyrimidine der Formel

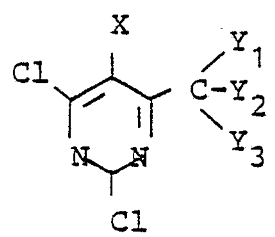

(IIa)

in welcher

X,$Y_1$,$Y_2$ und $Y_3$ die oben angegebene Bedeutung besitzen,

entweder mit HF in wasserfreier Flußsäure unter Druck umgesetzt oder in einem polaren organischen Lösungsmittel mit einem Alkalifluorid zur Reaktion gebracht werden.

Von besonderer Bedeutung sind Verbindungen der Formel (Ia), bei denen die Gruppierung der Formel

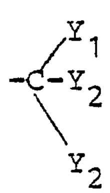

die folgende Bedeutung besitzt:

$-CCl_3$, $-CHCl_2$, $-CHBr_2$ und $-CCl_2F$.

Die für die Herstellung der Verbindungen der Formel (Ia) einzusetzenden Chlorpyrimidin-Derivate der Formel (IIa) sind zum Teil bekannt. Sie werden nach an sich bekannten Methoden hergestellt (Literatur: Siehe z.B. Journal of Het. Chem. 16, 1575 (1979)). Folgende Verbindungen der Formel (IIa) sind als Ausgangsverbindungen für (Ia) von besonderem Interesse:

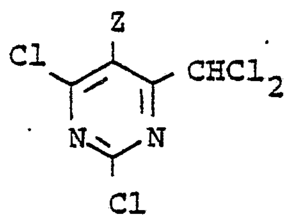

(IIb)

wobei

Z H, Cl oder Br

bedeutet.

Die Verbindungen der Formel (IIb) werden hergestellt, indem man die entsprechenden in 5-Stellung des Pyrimidinmoleküls H, Cl oder Br enthaltende 6-Methyluracile zunächst mit $POCl_3$ in die 2,4-Dichlor-5-(H,Cl,Br)-6-methyluracile überführt und anschließend ohne Zwischenisolierung mit elementarem Chlor bei Temperaturen von ca. 60 bis ca. 160°C behandelt. Von besonderem Wert als Ausgangsverbindung der Formel (IIb) ist

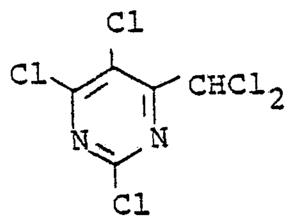

Im Pyrimidinkern sind Chlor oder Chloratome in 2,4- und 6-Stellung durch den Einfluß der Ringstickstoffatome aktiviert. Ihr Austausch gegen Fluor erfolgt im allgemeinen durch Kaliumfluorid in polaren Lösungsmitteln wie z.B. Tetramethylensulfon, Dimethylsulfoxid, N-Methylpyrrolidon oder anderer (vgl. z.B. M. Hudlicky, Chemistry of Organic Flourine Compounds, 2. Aufl., J. Wiley and Sons 1976, S. 123-124). In einigen Fällen ist auch die Verwendung von Natriumfluorid beschrieben worden (s.o., S. 129). B.A. Ivin et al beschreiben in J.Gen.Chem. USSR, Vol. 34, S. 4283 (engl. Übers) die Herstellung von 2,4-Difluor-6-methylpyrimidin und von 2,4-Difluor-5-brom-6-methylpyrimidin durch einen Austausch des Chlors gegen ein Fluoratom mit Kaliumfluorid. Wegen extremer Schwierigkeiten schlagen die Autoren Maßnahmen vor, wie Einsatz bestimmter Katalysatoren und Lösungsmittel die Ausbeute auf schließlich 40 % der Theorie bringen.

Weiterhin ist bekannt, daß man Trichlormethyl und auch Dichlormethylgruppen in der Seitenkette von aromatischen Verbindungen durch Cl/F-Austausch herstellen kann (s. u.a. Houben-Weyl, Bd. 5/3, S. 122; M. Hudlicky, s.o. S. 96, 105).

Wie oben beschrieben wurde nun gefunden, daß man die neuen in 2- und 4-Stellung fluorierten Pyrimidine der allgemeinen Formel (Ia) durch einen Austausch von Chlor gegen Fluoratome erhalten kann. Als Fluorierungsmittel kann man wie oben erwähnt, sowohl wasserfreie Fluorwasserstoffsäure als auch Alkalifluorid (insbesondere Natriumfluorid) im Lösungsmittel verwenden. Das ist überraschend, denn durch die bisher bekannt gewordenen Fluorierungen mit den vorgenannten Fluorierungsmitteln ist eine selektive Chlor gegen Fluor-Austauschreaktion am Kern des Pyrimidinringes weder offenbart noch nahegelegt worden.

Zur Fluorierung mit wasserfreier Fluorwasserstoffsäure verfährt man im allgemeinen so, daß die Fluorwasserstoffsäure in einem Druckgefäß mit Rührer, Rückflußkühler und Thermometer vorgelegt wird. Dem Rückflußkühler nachgeschaltet ist ein Entspannungsventil das es erlaubt, die bei der Reaktion freiwerdende Chlorwasserstoffsäure bei einem bestimmten konstanten Druck in eine Abgasleitung zu entspannen. Bei einer Temperatur unterhalb des Siedepunktes der wasserfreien HF (Kp 19°C) gibt man das Ausgangsmaterial zu und verschließt dann den Autoklaven. Nun wird hochgeheizt bis zu einer Temperatur bei der Chlor-Fluor-Austauschreaktion einsetzt. Je nach Art des Substituenten in 5-Stellung des Pyrimidinringes und der Zahl der Halogenatome in der Seitenkette setzt die Reaktion zwischen ca. 40 bis ca. 80°C ein. Im Maß der nachlassenden Chlorwasserstoffentwicklung heizt man höher, gegebenenfalls bis auf 180°C.

Im allgemeinen ist die Temperatur von 120 bis 150°C für einen hohen Umsatz ausreichend. Der Entspannungsdruck wird mit steigender Temperatur nachgeregelt; und zwar in der Weise daß der Entspannungsdruck deutlich (2 bis 10 bar) über dem Dampfdruck dem Flußsäure liegt. Gegen Ende der Reaktion entspannt man den Chlorwasserstoffdruck über der Reaktionslösung soweit, daß diese am Rückfluß siedet. Ist auch dann keine weitere HCl-Entwicklung mehr zu beobachten, dann ist die Reaktion beendet und man kühlt ab. Die Aufarbeitung erfolgt zunächst in der Weise, daß der überschüssige Fluorwasserstoff abdestilliert wird. Anschließend kann aus dem gleichen Reaktor weiterdestilliert und die gewünschte Fraktion isoliert werden. Andererseits kann man aber auch durch eine Wäsche mit kaltem Wasser alle anhaftende restliche Fluorwasserstoffsäure entfernen und danach die fraktionierte Destillation in einer Glasapparatur durchführen. Außer durch die Temperatur und die Zeit läßt sich die Reaktion auch durch das Molverhältnis von Ausgangspyrimidin und Fluorwasserstoff beeinflussen. Im Ansatz muß mindestens die molare Menge HF vorhanden sein. Es ist aber zweckmäßig, einen Überschuß HF einzusetzen. Es hat sich als zweckmäßig erwiesen, pro Mol Ausgangspyrimidin wenigstens 10 Mol HF einzusetzen. Die Ausbeuten sind jedoch höher und die Reaktion verläuft unter milderen Bedingungen, wenn der Überschuß größer ist. Ab 40 Mol HF/pro Pyrimidin ist kein weiterer Einfluß der HF-Menge auf den Reaktionsablauf mehr feststellbar. Die oben beschriebene erfindungsgemäße Verwendung von wasser-

freier Fluorwasserstoffsäure als Fluorierungsmittel ist immer dann zweckmäßig, wenn Ausgangsmaterial und Reaktionsprodukt empfindlich gegen thermische Belastung oder gegen Alkali sind.

Setzt man als Ausgangskomponente die Verbindungen der Formel

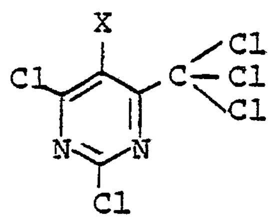

(IIc)

in welcher X = H, Cl oder Br,

mit flüssigem Fluorwasserstoff im Autoklaven entsprechend dem Vorerwähnten ein, erhöht die Temperatur bei dieser Umsetzung auf einen Bereich von ca. 120 bis ca. 180°C, so findet neben dem Austausch der Cl-Atome in Stellung 2 und 4 des Pyrimidinringes auch der Austausch eines Chloratoms in der $CCl_3$-Seitenkette statt und man erhält in hoher Ausbeute Verbindungen der Formel

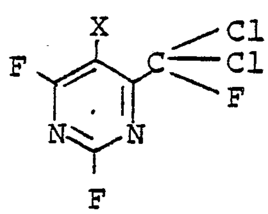

(Ic)

wobei X die oben angegebene Bedeutung hat.

Die Verbindungen der allgemeinen Formel (Ia) sind wertvolle Zwischenprodukte für die Herstellung der neuen Reaktivfarbstoffe der Formel (I). Bei der Herstellung dieser Reaktivfarbstoffe werden Farbstoffe der Formel

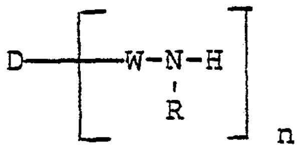

worin

D Rest eines organischen Farbstoffs,

W direkte Bindung oder Brückenglied,

R Wasserstoff oder $C_1$-$C_4$-Alkyl und

n 1 oder 2

mit n Mol der Verbindung der Formel (Ia) unter Zusatz eines Säurebindemittels umgesetzt, wobei man Reaktivfarbstoffe der Formel (I) erhält.

Die neuen Farbstoffe der Formel (I) können den verschiedensten Klassen angehören, z.B. der Reihe der metallfreien oder metallhaltigen Mono- oder Polyazofarbstoffe, metallfreien oder metallhaltigen Azaporphinfarbstoffe, wie Kupfer- oder Nickelphthalocyaninfarbstoffe, der Anthrachinon-, Oxazin-, Dioxazin-, Triphenylmethan-, Nitro-, Azomethin-, metallfreien oder metallhaltigen Formazanfarbstoffe.

Bevorzugte Farbstoffe sind solche mit 1 bis 6 Sulfogruppen.

Geeignete Farbstoffreste D bzw. den Farbstoffen (I) zugrunde liegende aminogruppenhaltige Farbstoffe sind in der Literatur in sehr großer Zahl beschrieben. Beispielhaft erwähnt seien hier:
Belgische Patentschriften: 606 947, 617 435; 567 435, 701 273, 683 734; 683 573; 693 749; 708 003; 707 307;

Deutsche Auslegeschriften: 1 242 553; 1 212 655, 1 225 322;

Deutsche Offenlegungsschriften: 2 633 255; 2 103 299; 2 305 206; 2 418 283; 2 623 224; 1 809 388; 1 912 178; 2 057 867; 1 769 205; 2 107 427; 2 303 601; 2 600 490.

Schweizer Patentschriften: 536 353; 521 403;
Britische Patentschriften: 1 299 881; 1 300 742 sowie
Venkataraman: The Chemistry of Synthetic Dyes, Band VI, Kapitel II, Seite 211 bis 325
New York, London; 1972.

Besonders wertvolle Farbstoffe dieser Reihe sind wasserlösliche Azofarbstoffe, und insbesondere solche, die Sulfonsäure- und/oder Carbonsäuregruppen aufweisen. Dabei besitzen aber auch sulfon- und carbonsäuregruppen-freie reaktive Dispersionsfarbstoffe der

Azoreihe Interesse. Die Azofarbstoffe können sowohl metallfrei als auch metallhaltig sein, wobei unter den Metallkomplexen die Kupfer-, Chrom- und Kobaltkomplexe bevorzugtes Interesse haben.

Wichtige Azofarbstoffe sind beispielsweise solche der Benzol-azo-naphthalinreihe, der Benzol-azo-1-phenyl-5-pyrazolonreihe, der Benzol-azo-benzolreihe, der Naphthalin-azo-benzolreihe, der Benzol-azo-aminonaphthalinreihe, der Naphthalin-azo-naphthalinreihe, der Naphthalin-azo-1-phenylpyrazolon-5-Reihe, der Benzol-azo-pyridonreihe, der Benzol-azo-aminopyridin-reihe, der Napthalin-azo-pyridonreihe, der Napthalin-azo-aminopyridinreihe, und der Stilben-azo-benzolreihe, wobei auch hier die sulfonsäuregruppenhaltigen Farbstoffe bevorzugt sind. Im Falle von Metallkomplexazofarbstoffen befinden sich die metallkomplexgebundenen Gruppen vorzugsweise in den o-Stellungen zur Azogruppe, z.B. in Form von o,o'-Dihydroxy-, o-Hydroxy-o'-carboxy-, o-Carboxy-o'-amino- und o-Hydroxy-o'-amino-azogruppierungen.

Folgende Farbstoffe der Struktur

$$D\left[-W-\underset{\displaystyle R}{\underset{|}{N}}-A\right]_n$$

kommen insbesonders in betracht, wobei W, R, n und A die in Formel (I) angegebene Bedeutung besitzen:

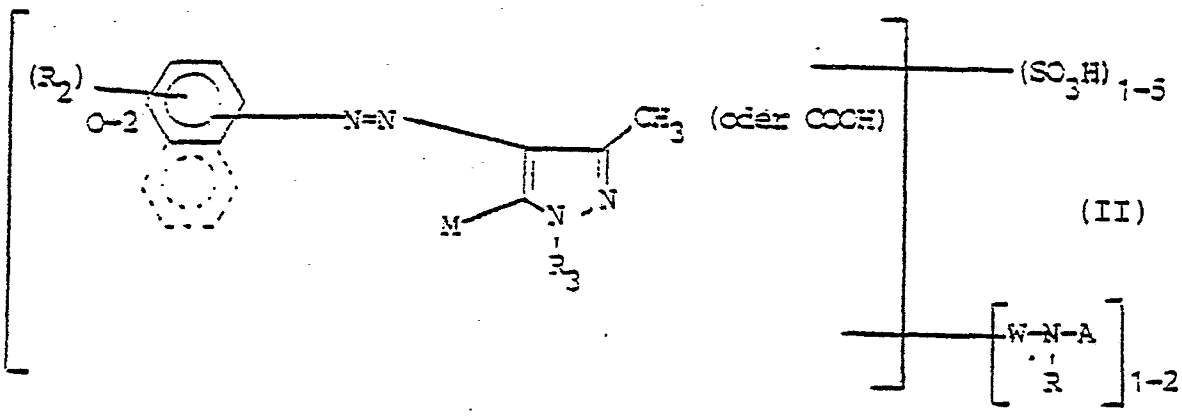

(II)

$M = OH, NH_2, NR_3R_4, OR_3$

$R_2$ = Alkyl, Alkoxy, Halogen, insbesondere Cl,

$R_3$ und $R_4$ = Wasserstoff, Alkyl, Aryl, Hetaryl, Aralkyl, oder wobei $R_3$ und $R_4$ zusammen für gegenenfalls durch NH oder O unterbrochenes $C_4$-$C_6$-Alkylen stehen.

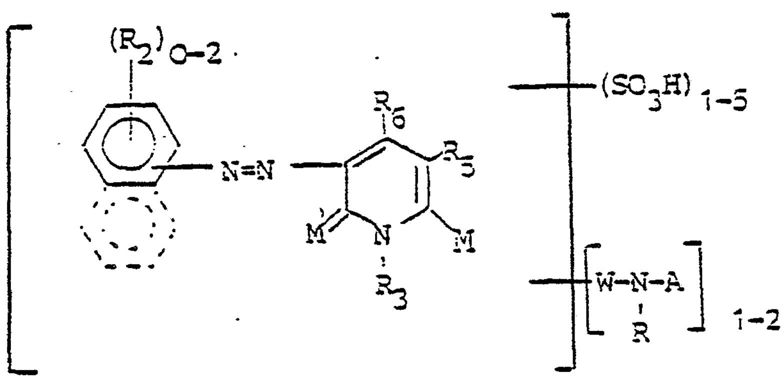

(III)

$R_5 = -COOR_3, -CONR_3R_4, -CN, -CH_2-SO_3H$ oder $-CH_2-NR_3R_4$

$R_6 = -M, -R_3$ oder $-R_5$

$M' = O, NR_3$

wobei $R_3$ und $R_4$ = Wasserstoff, Alkyl, Aryl, Hetaryl, Aralkyl oder wobei $R_3$ und $R_4$ zusammen für gegebenenfalls durch NH oder O unterbrochenes $C_4$-$C_6$Alkylen stehen.

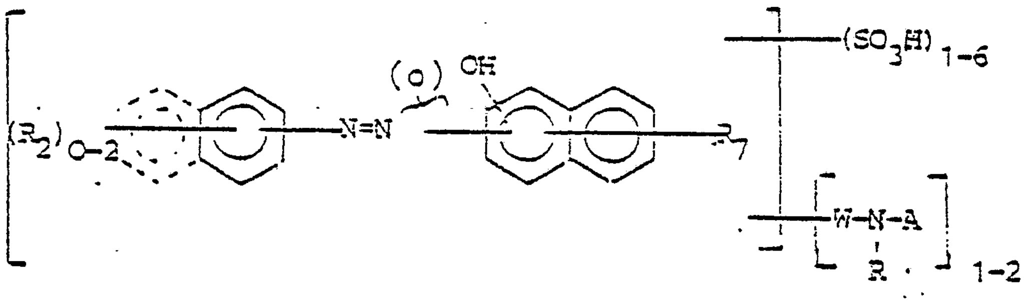

(IV)

$R_7$ = Wasserstoff, $-NR_3-CO-R_8$, $-NR_3-SO_2-R_8$
o = ortho-ständige Stellung der jeweiligen Reste

wobei $R_3$ = Wasserstoff, Alkyl, Aryl, Hetaryl, Aralkyl,
$R_8$ = Alkyl, Aryl, Hetaryl oder Aralkyl

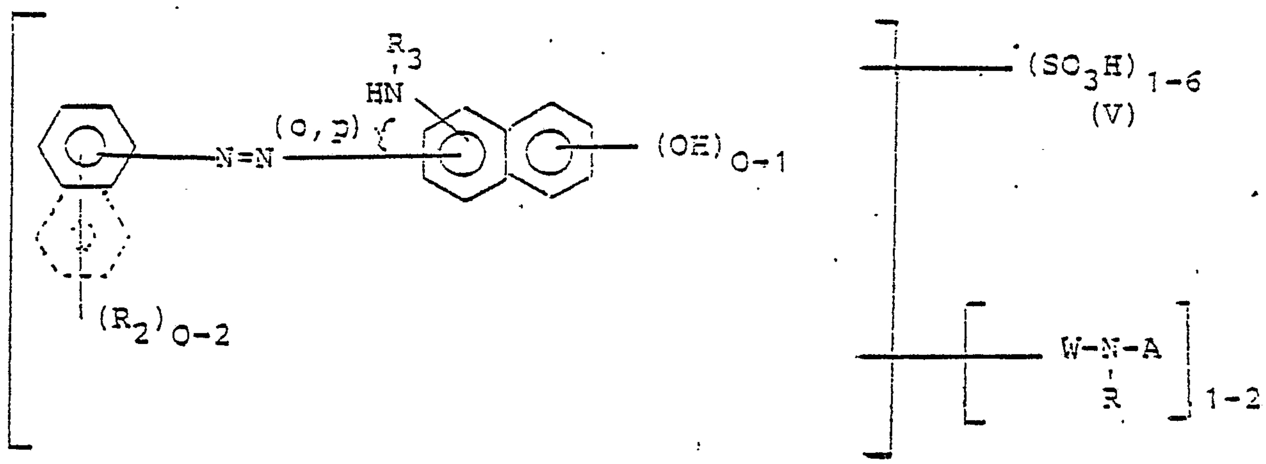

(V)

(o,p) = ortho- oder paraständige Stellung der jeweiligen Reste

wobei $R_2$ = Alkyl, Alkoxy, Halogen (insbesondere Cl)
$R_3$ = Wasserstoff, Alkyl, Aryl, Hetaryl oder Aralkyl.

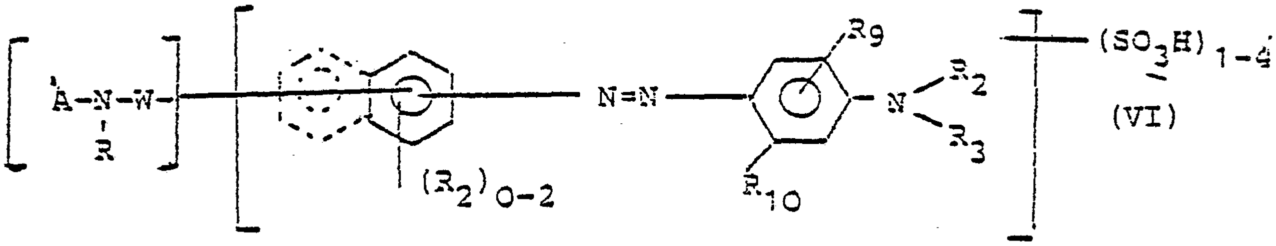

(VI)

$R_9$ und $R_{10}$ = Wasserstoff, $R_8$, $-OR_3$, Halogen, $-NR_3R_4$, $-COOR_3$, $-NR_3-CO-R_4$, $-O-CO-R_4$, $-NR_3-SO_2-R_4$, $-O-SO_2-R_4$, $-NR_3-CO-NR_3R_4$

wobei $R_3$ und $R_4$ = Wasserstoff, Alkyl, Aryl, Hetaryl, Aralkyl oder wobei $R_3$ und $R_4$ zusammen für gegebenenfalls durch NH oder O unterbrochenes $C_4$-$C_6$-Alkylen stehen

und $R_8$ = Alkyl, Aryl, Hetaryl oder Aralkyl.

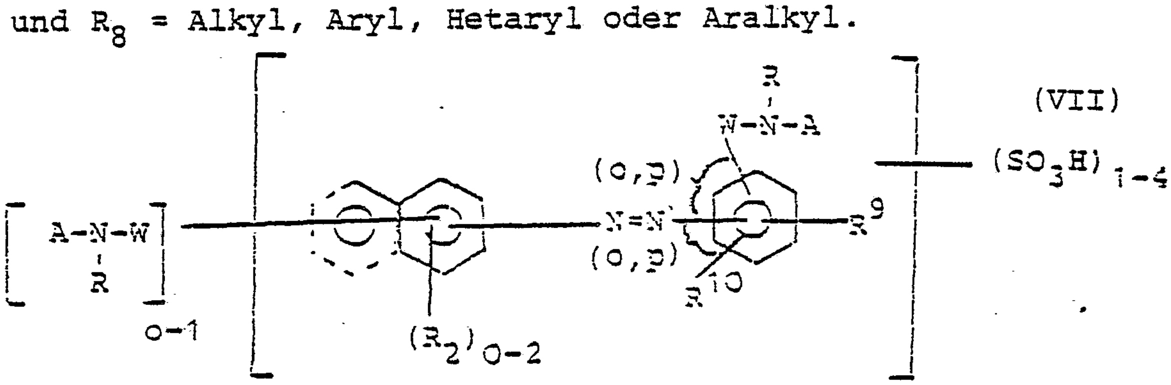

(VII)

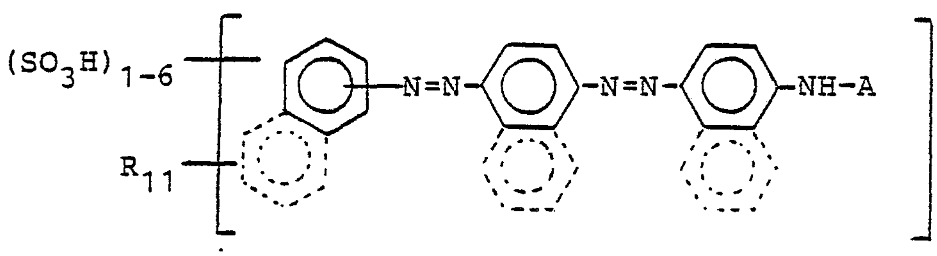

(VIIa)

$R_{11}$ = $R_9$, $C_1$-$C_4$-Alkyl

wobei (o,p) = ortho- oder paraständige Stellung der jeweiligen Reste

$R_2$ = Alkyl, Alkoxy, Halogen, und wobei $R_9$ und $R_{10}$ die unter Formel VI angegebene Bedeutung haben.

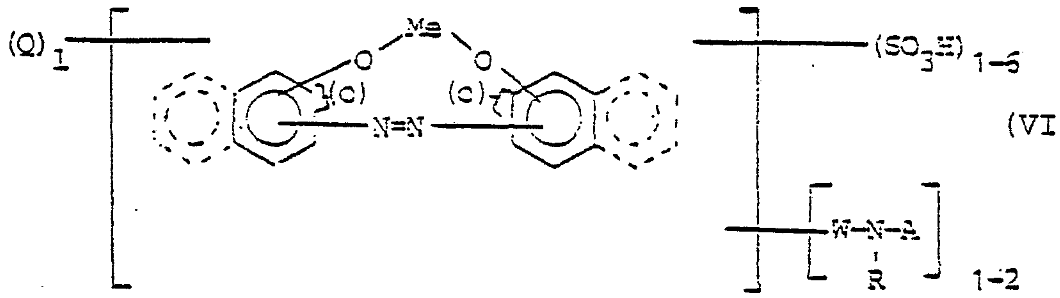

(VIII)

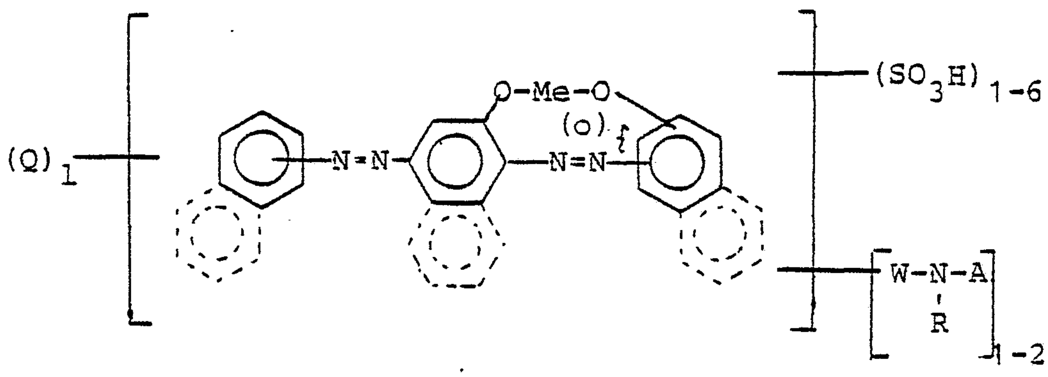

(VIIIa)

wobei (o) = orthoständige Stellung der jeweiligen Reste

Me = Cu, Cr

Q = Alkyl, Alkoxy, Halogen, $-NO_2$, Acylamino wie $CH_3-CO-NR_3-$, $-COOR_3$, $-CONR_3R_4$, $-SO_2-NR_3R_4$

l = 0-4

und wobei $R_3$ und $R_4$ die unter Formel (VI) angegebene Bedeutung haben

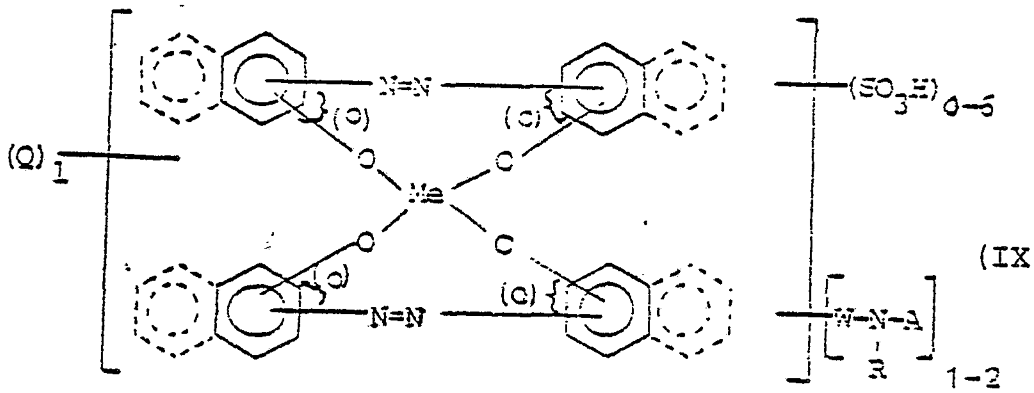

(IX).

Me = Co, Cr

l = 0-8

(O) = orthoständige Stellung der Substituenten

Q = Alkyl, Alkoxy, Halogen, $NO_2$, Acylamino wie $CH_3$-CO-$NR_3$-, -$COOR_3$, -$CONR_3R_4$, -$SO_2$-$NR_3R_4$

und wobei $R_3$ und $R_4$ die unter Formel (VI) angegebene Bedeutung haben.

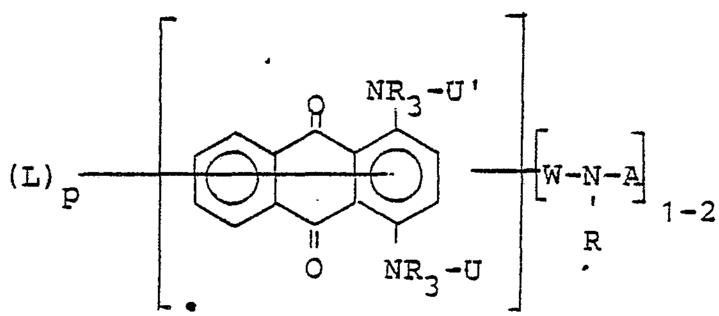

(X)

p = 0 - 4,

L = Substituent, wie Sulfo oder Carboxy,

U = gegebenenfalls durch O oder S unterbrochener, aliphatischer, cycloaliphatischer, araliphatischer oder aromatischer Rest

U' = Wasserstoff oder U

$R_3$ = Wasserstoff, Alkyl, Aryl, Hetaryl oder Aralkyl

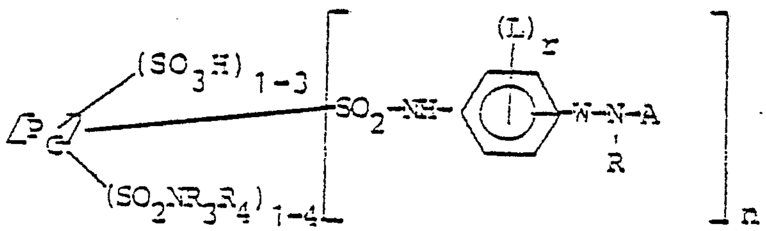

(XI)

$P_C$ = Cu/Ni-Phthalocyanin-Rest

L = Substituent, insbesondere Sulfo oder Carboxy

r = 0 - 4

und wobei

$R_3$ und $R_4$ die unter Formel (VI) angegebene Bedeutung haben.

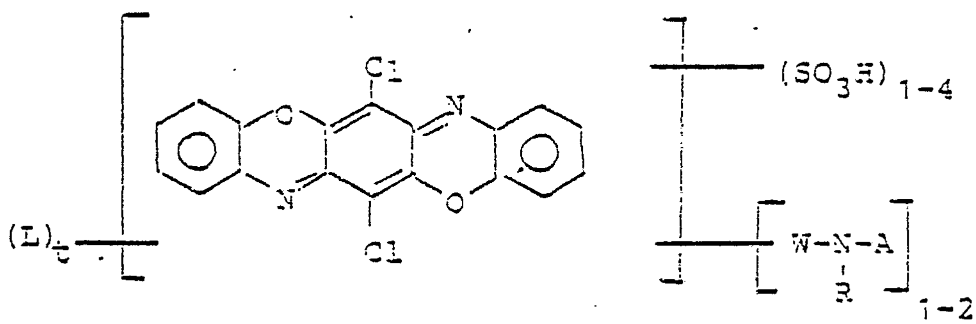

(XII)

L = Substituent, insbesondere Halogen, $C_{1-4}$-Alkyl, $C_{1-4}$-Alkoxy oder Carboxy

t = 0 bis 3

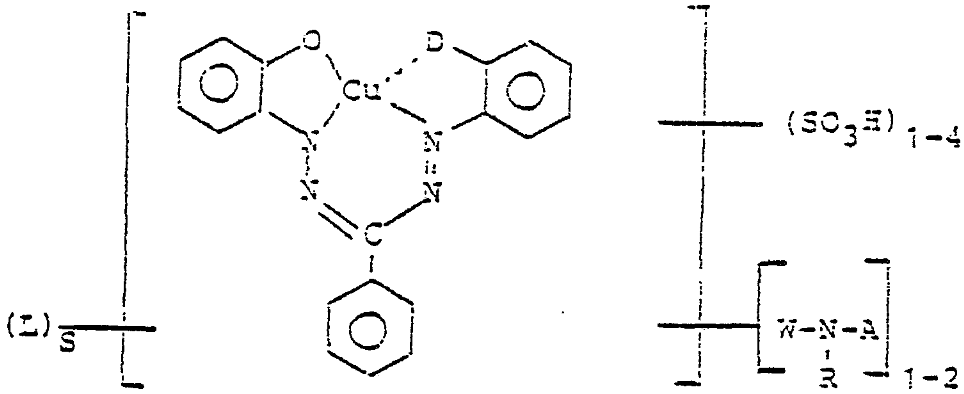

(XIII)

L = Substituent, insbesondere Halogen, $C_{1-4}$-Alkyl, $C_{1-4}$-Alkoxy, Alkylsulfonyl, Aminosulfonyl, unkondensierte Phenylreste oder Carboxy

S = 0-6

D = zur Komplexbildung befähigte Gruppe wie -OH, -COOH oder $-SO_3H$.

Aus der großen Zahl der durch die Formel I - XI beschriebenen Farbstoffe sind folgende Gruppen ganz besonders bevorzugt:

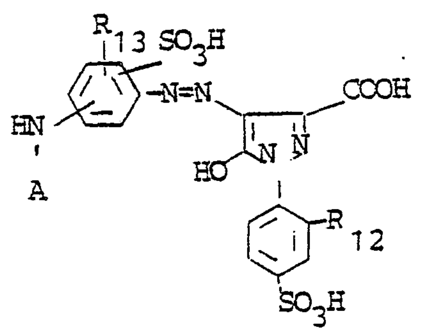

XIV

wobei

$R_{12}$ = H, $CH_3$, Cl

$R_{13}$ = H, $CH_3$, Cl

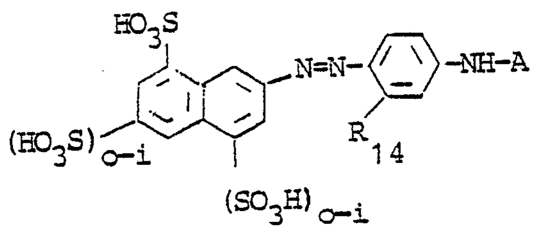

(XV)

wobei $R_{14}$ = H, $CH_3$, $OCH_3$, $NHCOCH_3$, $NH-CONH_2$

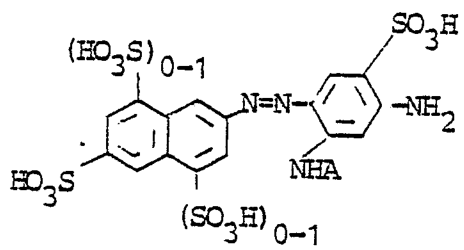

(XVI)

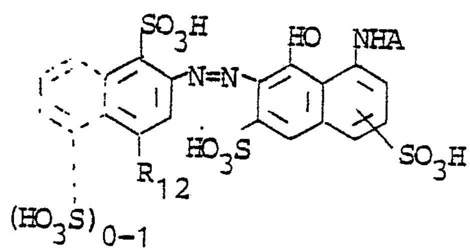

(XVII)

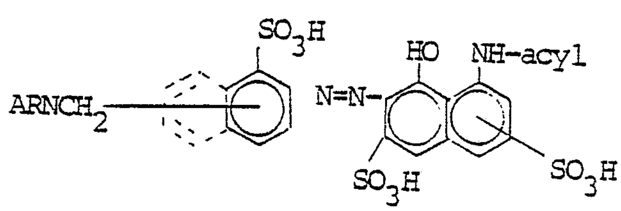

(XVIII)

worin acyl = Acylrest, insbesondere Formyl, $C_1$-$C_4$-Alkylcarbonyl, Arylcarbonyl und bevorzugt gegebenenfalls durch $C_1$-$C_4$-Alkyl, OH, $SO_3H$, COOH substituiertes Phenylcarbonyl.

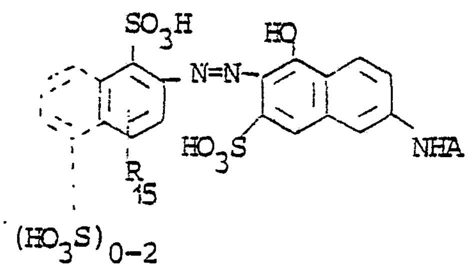

(XIX)

$R_{15}$ = $R_{13}$, $CH_3$, $C_1$-$C_4$-Alkoxy, Acylamino

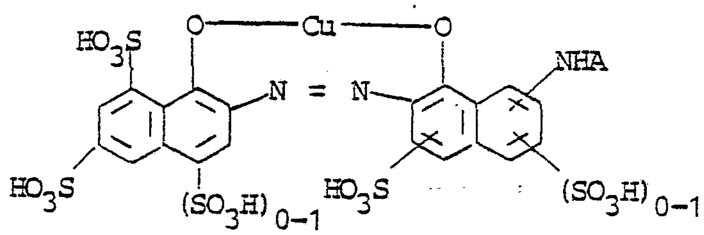

(XX)

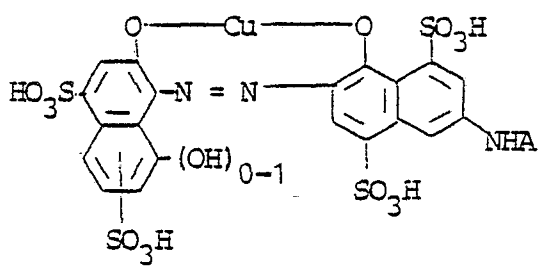

(XXI)

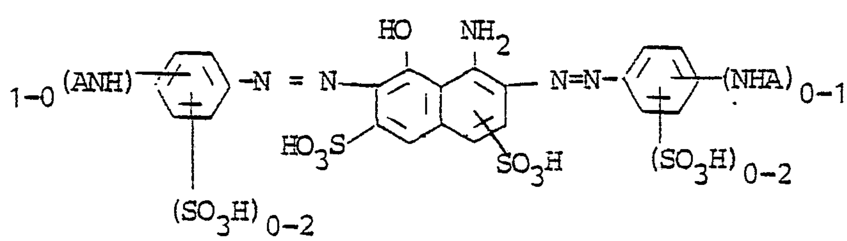

(XXII)

wobei in der Formel XXII der Rest ANH- im Molekül nur einmal vorhanden sein soll,

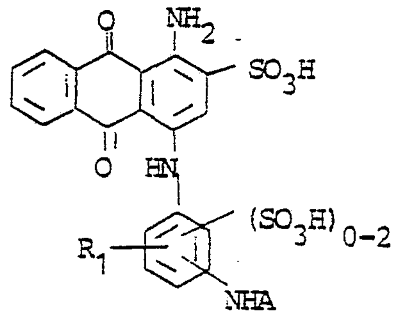

(XXIII)

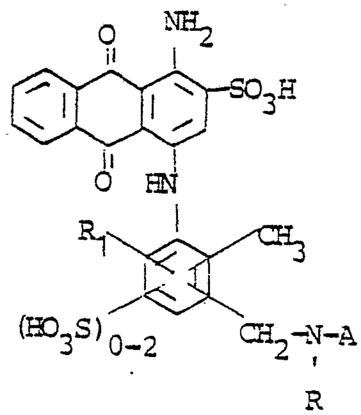

(XXIV)

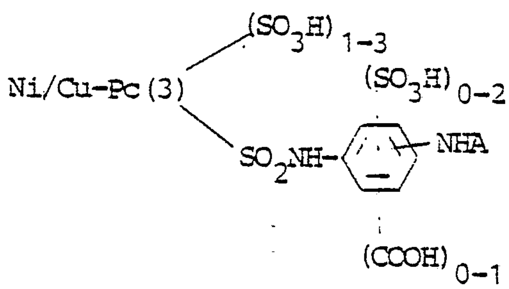

(XXV)

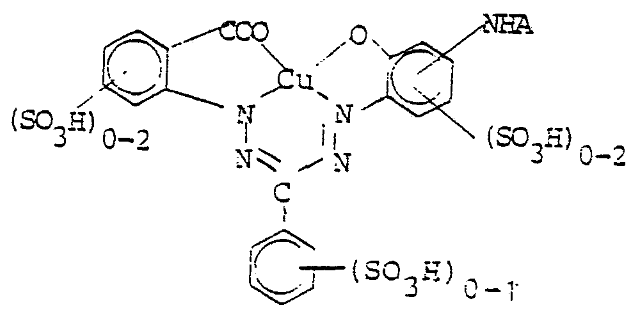

(XXVI)

Die neuen Farbstoffe können im übrigen beliebige in Farbstoffen übliche Substituenten aufweisen, wie Sulfonsäure-, Carbonsäure-, Sulfonamid- und Carbonamidgruppen, die am Amidstickstoffatom weiter substituiert sein können, Sulfonsäureester- und Carbonsäureestergruppen, Alkyl-, Aralkyl- und Arylreste, Alkylamino-, Aralkylamino-, Arylamino-, Acylamino-, Nitro-, Cyan-, Halogen-, wie Cl, Br und F, Hydroxy-, Alkoxy-, Thioether-, Azo-gruppierungen und dergleichen.

Die Farbstoffe der Formeln I bis XXVI eignen sich in hervorragender Weise zum Färben und Bedrucken von textilen Gebilden aus nativer und regenerierter Cellulose sowie von natürlichen oder synthetischen Polypeptiden.

Die mit diesen Farbstoffen in hohen Ausbeuten erhältlichen Färbungen zeichnen sich durch eine hohe Faser-Farbstoff-Bindungsstabilität aus sowie durch eine hervorragende Stabilität gegenüber Oxydationsmitteln wie peroxyd- oder chlorhaltige Waschmittel. Die Auswaschbarkeit der bei Färben oder Drucken entstehenden Hydrolyseprodukte ist ausgezeichnet.

Die neuen Farbstoffe der allgemeinen Formel I sind nach den bei der Synthese von Reaktivfarbstoffen üblichen Herstellungsverfahren zugänglich. So können beispielsweise die entsprechenden Farbstoffe der Formel

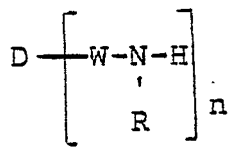

die aus der Literatur in großer Zahl bekannt sind mit der äquimolaren Menge eines substituierten Pyrimidins der Formel

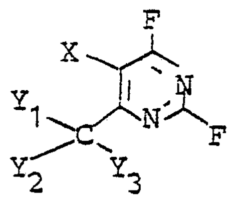

(Ia)

wobei X, $Y_1$, $Y_2$ und $Y_3$ die in Anspruch 1 angegebene Bedeutung besitzen kondensiert werden.

Häufig wird auch die Kondensation des Fluorpyrimidins mit einem Farbstoffvor- oder -zwischenprodukt zuerst durchgeführt, der dann der Aufbau des Farbstoffs z.B. durch die Azo-Kupplung folgt.

Die Umsetzung mit dem substituierten Pyrimidin erfolgt in wässrigen oder organisch-wässrigen Medien bei 0 - 70°C in Anwesenheit von säurebindenden Mitteln wie Natriumcarbonat, Natriumbicarbonat oder verdünnter Natronlauge.

Andere Umwandlungsreaktionen der Farbstoffe oder deren Vorprodukte wie Metallisierungsreaktionen, Sulfierungen oder Einführung von Acylaminogruppierungen können im allgemeinen in beliebigen Stufen der Farbstoffsynthesen

vorgenommen werden.

Die Herstellung der als Ausgangsmaterial bevorzugt eingesetzten Reaktivkomponente 2,4-Difluor-5-chlor-6-halogenalkylpyrimidin erfolgt durch Cl/F-Austausch an 6-Halogenalkyl-2,4,5-trichlorpyrimidin auf zweierlei Weise.

1) Mit NaF,
   a) ohne Lösungsmittel in 5 - 15 h bei etwa 300 - 350 °C
   b) im Lösungsmittel, bspw. Tetramethylensulfon in 5 - 10 h bei etwa 160 - 230°C

2) Mit HF wasserfrei bei 80 - 160°C und einem Druck von 15 bis 30 bar. Dabei wird der durch die Reaktion freiwerdende Chlorwasserstoff kontinuierlich in der Weise entspannt, daß der Druck anfänglich wenig (etwa bis zu 5 bar) über und gegen Ende der Reaktion nahezu oder vollständig beim Partialdruck der wasserfreien HF liegt. Verfahren und physikalische Eigenschaften sind im Beispiel näher beschrieben.

Bei diesen Verfahren können bei geeigneter Wahl der Versuchsbedingungen auch die Halogene in der Alkylseitenkette ganz oder teilweise ausgetauscht werden.

Beispiel 1

19 Teile 1,3-Diaminobenzol-6-sulfonsäure werden in 250 Teilen Wasser angerührt und durch Zugabe von ca. 40 ml einer 20 %igen Natriumcarbonatlösung gelöst.

In diese Lösung werden bei ca. 20-30° 23,5 Teile 5-Chlor-6-dichlormethyl-2,4-difluorpyrimidin gegeben. Bei 20-50° wird unter kräftigem Rühren solange die freiwerdende Flußsäure durch Zutropfen einer Natriumcarbonatlösung neutralisiert bis sich der pH-Wert von 6-7 nicht mehr ändert. Das so erhaltene Kondensationsprodukt der Formel

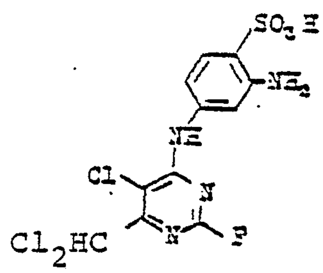

wird nach Zugabe von Eis und 7 Teilen Natriumnitrit mit 28 Teilen Salzsäure direkt diazotiert. In diese Suspension der Diazoniumverbindung werden 30 Gew.-Teile 1-[2'-Methyl-4'-sulfophenyl]-3-carboxypyrazolon-(5) gegeben und die Kupplung durch Zugabe von ca. 80 Teile einer Natriumcarbonatlösung (20 %ig) zu Ende geführt. Nach beendeter Kupplung wird die Abscheidung des Farbstoffs durch Zugabe von 200 Teilen Kaliumchlorid oder Natriumchlorid vervollständigt, anschließend wird durch Filtration isoliert und bei 50-100° getrocknet. Man erhält ein gelbes Pulver, das Baumwolle in grünstichig gelben Tönen anfärbt bzw. druckt. (CI:1; GN:Bright).

Die so erhaltenen Drucke oder Färbungen besitzen hervorragende Naßechtheiten.

Verwendet man anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in grünstichig gelben Tönen färben.

Weitere gelbe Farbstoffe mit ähnlichen Eigenschaften erhält man, wenn man die in der folgenden Tabelle in Spalte 1 genannten Diazokomponenten mit den in Spalte 3 genannten Pyrimidinen wie in Beispiel 1 beschrieben umsetzt, diazotiert und mit den in Spalte 2 genannten Kupplungskomponenten kuppelt.

Im vorliegenden Beispiel 1 sowie in den nachfolgenden Beispielen bedeutet
CI: Colour Index Hue Indication Chart-Nummer sowie
GN: Generic Code-Nummer
B = bright (klar)
D = dull (stumpf)

Tabelle 1

| | 1 | 2 | 3 | | |
|---|---|---|---|---|---|
| Beispiel | Diazokomponente | Kupplungskomponente | 2,4-Difluorpyrimidin | CI | GN |
| 2 | 2,4-Diaminobenzol-sulfonsäure | 3-Methylpyrazolon-(5) | 5-Chlor-6-dichlor-methyl | 1 | B |
| 3 | " | 1,3-Dimethylpyra-zolon-(5) | " | 1 | B |
| 4 | " | 1-Carboxymethyl-3-methyl-pyrazolon-(5) | " | 2 | B |
| 5 | " | 1-(4'-Sulfophenyl)-3-carboxypyrazolon-(5) | " | 2 | B |
| 6 | " | 1-(2',5'-Disulfophenyl)-3-methylpyrazolon-(5) | " | 1 | B |
| 7 | " | 1-(2'-Chlor-5'-sulfo-phenyl)-3-methylpyra-zolon-(5) | " | 1 | B |
| 8 | " | 1-(3'-Sulfophenyl)-3-methyl-5-aminopyrazol | " | 1 | B |
| 9 | " | 1-(2,5-Dichlor-4'-sulfophenyl)-3-me-thylpyrazolon-(5) | " | 1 | B |

Tabelle 1 (Fortsetzung)

| Beispiel | 1<br>Diazokomponente | 2<br>Kupplungskomponente | 3<br>2,4-Difluorpyrimidin | CI | GN |
|---|---|---|---|---|---|
| 10 | 2,4-Diaminobenzol-sulfonsäure | 3-Methylpyrazolon-(5) | 5-Brom-6-dichlor-methyl | 2 | B |
| 11 | " | 1,3-Dimethylpyra-zolon-(5) | " | 1 | B |
| 12 | " | 1-Carboxymethyl-3-methylpyrazolon-(5) | " | 2 | B |
| 13 | " | 1-(4'-Sulfophenyl)-3-carboxy-pyrazolon-(5) | " | 2 | B |
| 14 | " | 1-(2',5'-Disulfo-phenyl)-3-methyl-pyrazolon-(5) | " | 1 | B |
| 15 | 2,4-Diaminobenzol-sulfonsäure | 1-(2'-Chlor-5'-sul-fophenyl)-3-methyl-pyrazolon-(5) | 5-Brom-6-dichlor-methyl | 1 | B |
| 16 | " | 1-(3'-Sulfophenyl)-3-methyl-5-amino-pyrazol | " | 1 | B |

Tabelle 1 (Fortsetzung)

| | 1 | 2 | 3 | | |
|---|---|---|---|---|---|
| Beispiel | Diazokomponente | Kupplungskomponente | 2,4-Difluorpyrimidin | CI | GN |
| 17 | 2,4-Diaminobenzol-sulfonsäure | 1-Carboxymethyl-3-methyl-pyrazolon-(5) | 5-Chlor-6-dichlor-fluormethyl | 2 | B |
| 18 | " | 1-(4'-Sulfophenyl)-3-carboxypyrazolon-(5) | " | 1 | B |
| 19 | " | 1-(2'-5'-Disulfo-phenyl)-3-methyl-pyrazolon-(5) | " | 1 | B |
| 20 | " | 1-(2'-Chlor-5'-sul-fophenyl)-3-methyl-pyrazolon-(5) | " | 1 | B |
| 21 | " | 1-(3'-Sulfophenyl)-3-methyl-5-amino-pyrazol | " | 1 | B |
| 22 | " | 1-Carboxymethyl-3-methyl-pyrazolon-(5) | 6-Dichlorfluormethyl | 2 | B |

Tabelle 1 (Fortsetzung)

| | 1 | 2 | 3 | | |
|---|---|---|---|---|---|
| Beispiel | Diazokomponente | Kupplungskomponente | 2,4-Difluorpyrimidin | CI | GN |
| 23 | 2,4-Diaminobenzol-sulfonsäure | 1-(4'-Sulfophenyl)-3-carboxypyrazolon-(5) | 6-Dichlorfluormethyl | 2 | B |
| 24 | " | 1-(2'-5'-Disulfo-phenyl)-3-methyl-pyrazolon-(5) | " | 1 | B |
| 25 | " | 1-(2'-Chlor-5'-sul-fophenyl)-3-methyl-pyrazolon-(5) | " | 1 | B |
| 26 | " | 1-(3'-Sulfophenyl)-3-methyl-5-amino-pyrazol | " | 1 | B |
| 27 | " | 1-Carboxymethyl-3-methyl-pyrazolon-(5) | 5-Chlor-6-chlormethyl | 2 | B |
| 28 | " | 1-(4'-Sulfophenyl)-3-carboxypyrazolon-(5) | " | 2 | B |
| 29 | " | 1-(2'-5'-Disulfo-phenyl)-3-methyl-pyrazolon-(5) | " | 1 | B |

Tabelle 1 (Fortsetzung)

| | 1 | 2 | 3 | | |
|---|---|---|---|---|---|
| Beispiel | Diazokomponente | Kupplungskomponente | 2,4-Difluorpyrimidin | CI | GN |
| 30 | 2,4-Diaminobenzol-sulfonsäure | 1-(2'-Chlor-5'-sulfophenyl)-3-methylpyrazolon-(5) | 5-Chlor-6-chlormethyl | 1 | B |
| 31 | " | 1-(3'-Sulfophenyl)-3-methyl-5-amino-pyrazol | " | 1 | B |
| 32 | " | 1-Carboxymethyl-3-methylpyrazolon-(5) | 5-Brom-6-brommethyl | 2 | B |
| 33 | " | 1-(4'-Sulfophenyl)-3-carboxypyrazolon-(5) | " | 2 | B |
| 34 | " | 1-(2'-5'-Disulfo-phenyl)-3-methyl-pyrazolon-(5) | " | 1 | B |
| 35 | " | 1-(2'-Chlor-5'-sulfophenyl)-3-methylpyrazolon-(5) | " | 1 | B |

Beispiel 36

Die Lösung von 21 Teilen 1,3-Diamino-2-methylbenzol-5-sulfonsäure als Natriumsalz in 300 Teilen Wasser wird bei 20-50° unter kräftigem Rühren mit 23,5 Teilen 5-Chlor-6-dichlormethyl-2,4-difluorpyrimidin kondensiert. Die freiwerdende Flußsäure wird dabei durch Zutropfen einer 20 %igen Sodalösung neutralisiert. Nach beendeter Kondensation wird das Umsetzungsprodukt durch Zugabe von Eis, 7 Teilen Natriumnitrit und 28 Teilen Salzsäure bei 5-10° direkt diazotiert und anschließend durch Zugabe von 30 Gew.-Teilen 1-(2'-Methyl-4'-sulfophenyl)-3-carboxy-pyrazolon-(5) und ca. 80 Teilen einer 20 %igen Sodalösung zum Azofarbstoff der Formel

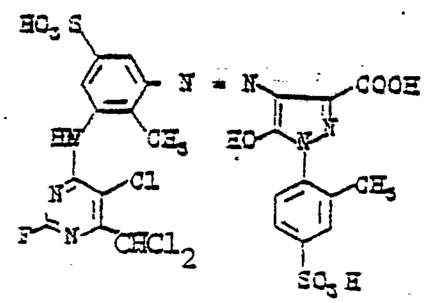

gekuppelt. Die Abscheidung des Farbstoffs wird durch Aussalzen mit 150 Teilen Natriumchlorid vervollständigt. Anschließend wird durch Filtration isoliert und bei 50-100° getrocknet. Man erhält ein gelbes Pulver, das zum Bedrücken und Färben von Baumwolle hervorragend geeignet ist. (CI = 1; GN = Bright)

Ersetzt man in diesem Beispiel die Kupplungskomponente

durch die in den Beispielen 2-9 angegebenen Pyrazolderivate, so werden ebenfalls gelbe Reaktivfarbstoffe mit sehr guten färberischen Eigenschaften erhalten.

Verwendet man anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in gelben Tönen färben. (CI = 1; GN = Bright)

Beispiel 37

19 Teile 1,4-Diaminobenzol-2-sulfonsäure werden als Natriumsalz in 250 Teilen Wasser bei 20° gelöst und mit 23,5 Teilen 5-Chlor-6-dichlormethyl-2,4-difluor-pyrimidin bei dieser Temperatur selektiv kondensiert. Durch Zugabe von 30 Teilen einer 20 %igen Sodalösung wird dabei ein pH-Wert von 5,8-6 eingehalten.

Nach beendeter Kondensation wird die Suspension durch Zugabe von Eis, 7 Teilen Natriumnitrit und 28 Teilen Salzsäure diazotiert.

Die Suspension der Diazoniumverbindung wird mit einer Lösung des Natriumsalzes von 29 Teilen 1-(4'-Sulfophenyl)-3-carboxypyrazolon-(5) in 150 Teilen Wasser

versetzt und die Kupplung durch Zugabe von 50 Teilen einer 20 %igen Sodalösung zu Ende geführt. Danach wird mit 200 Teilen Kaliumchlorid ausgesalzen, isoliert und getrocknet. Man erhält so ein gelbes Pulver, das Baumwolle in goldgelben Farbtönen färbt. Der Farbstoff entspricht der Formel

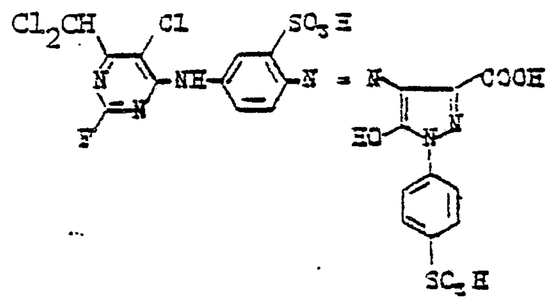

(CI = 4; GN = Bright)

Ähnliche Farbtöne werden erhalten, wenn in diesem Beispiel das 1-(4'-Sulfophenyl)-3-carboxypyrazolon-(5) durch äquimolare Mengen der im Beispiel 1-4 bzw. 6-9 angegebenen Pyrazole ersetzt wird.

Verwendet man in den obigen Farbstoffbeispielen anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in gelben Tönen färben.

Beispiel 38

0,1 Mol 1,5-Diaminobenzol-2,4-disulfonsäure werden in 250 Teilen Wasser angerührt und durch Zugabe von ca. 30 ml einer 20 %igen Natriumcarbonatlösung gelöst. In diese Lösung werden bei ca. 20°-30° 23,5 Teile 5-Chlor-5-dichlormethyl-2,4-difluorpyrimidin gegeben und bei 20-50° unter kräftigem Rühren solange die freiwerdende Flußsäure durch Zutropfen einer Natriumcarbonatlösung neutralisiert bis sich der pH-Wert von 6-7 nicht mehr ändert. Das so erhaltene Kondensationsprodukt der Formel

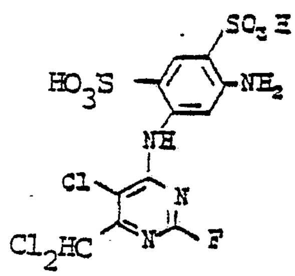

wird nach Zugabe von Eis und 7 Teilen Natriumnitrit mit 28 Teilen Salzsäure direkt diazotiert. In diese Suspension der Diazoniumverbindung werden 10 Gew.-Teile 3-Methylpyrazolon-(5) gegeben und die Kupplung durch Zugabe von ca. 80 Teilen einer Natriumcarbonatlösung (30 %ig) zu Ende geführt. Nach beendeter Kupplung wird die Abscheidung des Farbstoffs durch Zugabe von 200 Teilen Kaliumchlorid oder Natriumchlorid vervollständigt, anschließend wird durch Filtration isoliert und bei 50-100° getrocknet. Man erhält ein gelbes Pulver, das Baumwolle in gelben Tönen anfärbt bzw. druckt. (CI = 1; GN = B)

Die so erhaltenen Drucke oder Färbungen besitzen hervorragende Naßechtheiten.

Ersetzt man in diesem Beispiel die Kupplungskomponente 3-Methylpyrazolon-(5) durch äquimolare Mengen der in der folgenden Tabelle aufgeführten Pyrazole, so werden ebenfalls gelbe Reaktivfarbstoffe erhalten.

| Beispiel Nr. | Diazokomponent | Kupplungskomponent | CI | GN |
|---|---|---|---|---|
| 39 | 1,5-Diaminobenzol-2,4-disulfonsäure | 1-(2'-Chlorphenyl)-3-methylpyrazolon-(5) | 1 | B |
| 40 | " | 1,3-Dimethylpyrazolon-(5) | 1 | B |
| 41 | " | 1-Carboxymethyl-3-methylpyrazolon-(5) | 2 | B |
| 42 | " | 1-(4'-Sulfophenyl)-3-carboxypyrazolon-(5) | 2 | B |
| 43 | " | 1-(2'-5'-Disulfophenyl)-3-methylpyrazolon-(5) | 1 | B |
| 44 | " | 1-(2'-Chlor-5'-sulfophenyl)-3-methylpyrazolon-(5) | 1 | B |
| 45 | " | 1-(3'-Sulfophenyl)-3-methyl-5-aminopyrazol | 1 | B |
| 46 | " | 1(2'-Methyl-4'-sulfophenyl)-3-carboxypyrazolon-(5) | 1 | B |

Verwendet man in den Beispielen 38-46 anstelle von 5-Chlor-6-dichlormethyl-2,4-difluorpyrimidin die entsprechenden Mengen 5-Brom-6-dichlormethyl-, 5-chlor-6-dichlorfluormethyl-, 5-Chlor-6-chlormethyl-, 5-Chlor-6-

brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in gelben Tönen färben.

Beispiel 47

19 Teile 1,3-Diaminobenzol-6-sulfonsäure werden wie in Beispiel 1 beschrieben mit 23,5 Teilen 5-Chlor-6-dichlormethyl-2,4-difluorpyrimidin zur Reaktion gebracht, diazotiert und mit 22 Teilen 1,4-Dimethyl-6-oxy-2-pyridon-3-sulfonsäure gekuppelt. Der entstehende Farbstoff der Formel

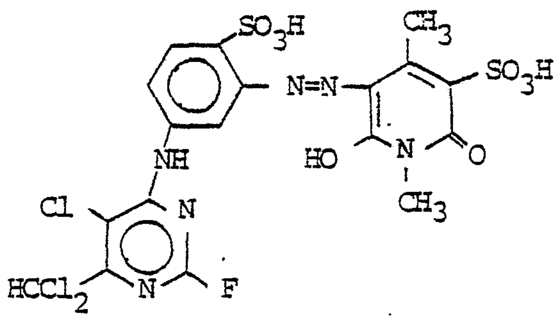

wird ausgesalzen und getrocknet. Er färbt Wolle und Baumwolle in reinen grünstichig gelben Nuancen. (CI = 1; GN=B) Weitere gelbe Farbstoffe erhält man, wenn man die in der folgenden Tabelle Spalte 1 genannten Diazokomponenten mit 5-Chlor-6-dichlormethyl-2,4-difluorpyrimidin umsetzt und danach mit den in Spalte 2 genannten Kupplungskomponenten kuppelt.

| Beispiel | Diazokomponente | Kupplungskomponente | Farbton | CI | CN |
|---|---|---|---|---|---|
| 48 | 1,3-Diaminobenzol-6-sulfonsäure | 1-Ethyl-4-methyl-6-oxy-2-pyridon-3-carbonamid | grünstichig gelb | 1 | B |
| 49 | " | 1-Ethyl-4-methyl-6-oxy-2-pyridon-3-sulfonsäure | " | 1 | B |
| 50 | " | 1-Ethyl-4-methyl-6-oxy-2-pyridon-3-methansulfonsäure | " | 1 | B |
| 51 | " | 1,4-Dimethyl-6-oxy-2-pyridon-3-methansulfonsäure | " | 1 | B |
| 52 | " | 1(2'-Ethylsulfo)-4-methyl-6-oxy-pyridon-3-carbonamid | " | 1 | B |
| 53 | 1,5-Diaminobenzol-2,4-disulfonsäure | 1-Ethyl-4-methyl-6-oxy-2-pyridon-3-carbonamid | " | 1 | B |
| 54 | " | 1-Ethyl-4-methyl-6-oxy-2-pyridon-3-sulfonsäure | " | 1 | B |
| 55 | " | 1-Ethyl-4-methyl-6-oxy-2-pyridon-3-methansulfonsäure | " | 1 | B |
| 56 | " | 1,4-Dimethyl-6-oxy-2-pyridon-3-methansulfonsäure | " | 1 | B |
| 57 | " | 1(2'-Ethylsulfo)-4-methyl-6-oxy-pyridon-3-carbonamid | " | 1 | B |
| 58 | " | 1,4-Dimethyl-6-oxy-2-pyridon-3-sulfonsäure | gelb | 1 | B |

| Beispiel | Diazokomponente | Kupplungskomponente | Farbton | CI | CN |
|---|---|---|---|---|---|
| 59 | 1,4-Diaminobenzol-6-sulfonsäure | 1,4-Dimethyl-6-oxy-2-pyridon-3-sulfonsäure | gelb | 2 | B |
| 60 | " | 1-Ethyl-4-methyl-6-oxy-2-pyridon-3-carbonamid | " | 2 | B |
| 61 | " | 1-Ethyl-4-methyl-6-oxy-2-pyridon-3-sulfonsäure | " | 2 | B |
| 62 | " | 1-Ethyl-4-methyl-6-oxy-2-pyridon-3-methansulfonsäure | " | 2 | B |
| 63 | " | 1,4-Dimethyl-6-oxy-2-pyridon-3-methansulfonsäure | " | 2 | B |
| 64 | " | 1(2'-Ethylsulfo)-4-methyl-6-oxy-pyridon-3-carbonamid | " | 2 | B |

Verwendet man in den Beispielen 47-64 anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in gelben bis grünstichig gelben Tönen färben.

Beispiel 65

20,2 Teile 3-Amino-N-methylbenzylamin-4-sulfonsäure werden in 200 ml Wasser heiß gelöst, auf 0-5° abgekühlt und mit 35 ml konzentrierter Salzsäure versetzt. Man diazotiert mit 7 Teilen Natriumnitrit und tropft die Suspension des Diazoniumsalzes in eine neutrale Lösung von 25 Teilen 1-Ethyl-4-methyl-6-oxy-2-pyridon-3-methansulfonsäure. Mit 80 Teilen einer 20 %igen Natriumcarbonatlösung wird die Kupplung zu Ende geführt. Der Farbstoff wird mit Natrium- oder Kaliumchlorid ausgesalzen und filtriert. Man löst den erhaltenen Filterkuchen in 500 ml Wasser und tropft bei 20°C langsam 23,5 Teile 5-Chlor-6-dichlormethyl-2,4-difluorpyrimidin zu. Es wird so lange gerührt, bis sich der pH-Wert von 7-8 nach Neutralisation der freiwerdenden Flußsäure durch Zutropfen von Natriumcarbonatlösung nicht mehr ändert. Der erhaltene Farbstoff der Formel

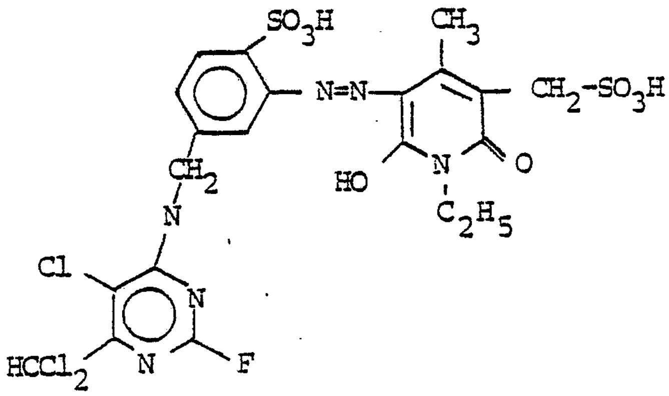

wird ausgesalzen, isoliert und getrocknet. Man erhält ein gelbes Pulver, das Baumwolle in grünstichig gelben Tönen färbt. (CI = 1; GN = B)

Weitere grünstichig gelbe Farbstoffe mit ähnlichen Eigenschaften erhält man, wenn man 1-Ethyl-4-methyl-6-oxy-2-pyridon-3-methansulfonsäure durch die in der folgenden Tabelle genannten Kupplungskomponente ersetzt und sonst wie in Beispiel 65 beschrieben verfährt.

| Beispiel | Diazokomponente | Kupplungskomponente | Farbton | CI | GN |
|---|---|---|---|---|---|
| 66 | 3-Amino-N-methyl-benzylamin-4-sulfonsäure | 1,4-Dimethyl-6-oxy-2-pyridon-3-methylsulfon-säure | grünstichig gelb | 1 | B |
| 67 | " | 1(2'-Ethylsulfo)-4-methyl-6-oxy-pyridon-3-carbonamid | " | 1 | B |
| 68 | " | 1-Ethyl-4-methyl-6-oxy-2-pyridon-3-carbonamid | " | 1 | B |

Verwendet man in den Beispielen 65-68 anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in grünstichig gelben Tönen färben.

Beispiel 69

23,9 Teile 2-Amino-5-hydroxynaphthalin-7-sulfonsäure werden in 200 ml Wasser neutral gelöst. In diese Lösung werden bei ca. 20-30° 23,5 Teile 5-Chlor-6-dichlormethyl-2,4-difluorpyrimidin gegeben. Unter kräftigem Rühren wird solange die freiwerdende Flußsäure durch Zutropfen von Natriumcarbonatlösung neutralisiert, bis sich der pH-Wert von 5,5-6,0 nicht mehr ändert.

Die Suspension des Kondensationsproduktes wird mit der nach üblichen Methoden hergestellten Suspension einer Diazoniumverbindung aus 2-Naphthylamin-1,5-disulfonsäure vereinigt und die Kupplung durch Zugabe von 10 Teilen Lithiumcarbonat zu Ende geführt. Der orangefarbene Reaktivfarbstoff der Formel

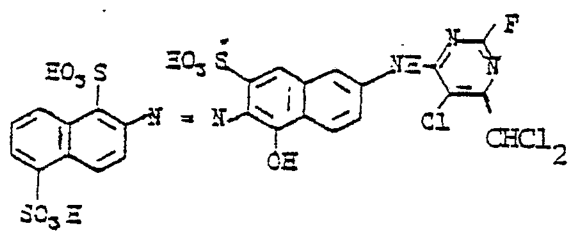

wird ausgesalzen, isoliert und getrocknet. Man erhält ein orangefarbenes Pulver, das zum Färben und Bedrucken von Baumwolle nach den für Reaktivfarbstoffen üblichen Verfahren hervorragend geeignet ist.
(CI = 6; DN = B)

Wird das in diesem Beispiel beschriebene Kondensationsprodukt aus 5-Chlor-6-dichlormethyl-2,4-difluorpyrimidin und 2-Amino-5-hydroxy-naphthalin-7-sulfonsäure nach an sich bekannten Methoden mit den Diazoniumverbindungen aus den nachfolgend aufgeführten Aminen gekuppelt, so werden ebenfalls hervorragende Reaktivfarbstoffe mit den angegebenen Farbtönen erhalten:

| Beispiel | Diazoniumverbindung aus: | Farbton | CI | GN |
|---|---|---|---|---|
| 70 | 2-Aminonaphthalin-1,5,7-trisulfonsäure | Orange | 6 | B |
| 71 | Anilin-2-sulfonsäure | Orange | 5 | B |
| 72 | 1-Amino-3-acetylaminobenzol-6-sulfonsäure | Orange | 7 | B |
| 73 | 1-Amino-5(2'-(2"-sulfophenylamino)-4'-fluortriazin-1',3',5'-yl-5')-aminobenzol-2-sulfonsäure | Orange | 6 | B |
| 74 | 1-Amino-4-methoxybenzol-2-sulfonsäure | Scharlach | 8 | B |
| 75 | 1-Amino-4-methoxybenzol-2,5-disulfonsäure | Scharlach | 8 | B |
| 76 | 2-Aminonaphthalin-3,6,8-trisulfonsäure | Scharlach | 7 | B |
| 77 | 2-Aminonaphthalin-4,6,8-trisulfonsäure | Scharlach | 7 | B |
| 78 | 2-Aminonaphthalin-1-sulfonsäure | Orange | 6 | B |

| Beispiel | Diazoniumverbindung aus | Farbton | CI | GN |
|---|---|---|---|---|
| 79 | 1-Amino-4-methoxybenzol-3-sulfonsäure | Scharlach | 8 | B |
| 80 | Anilin-4-sulfonsäure | Orange | 5 | B |
| 81 | 1-Amino-4-ethoxybenzol-2-sulfonsäure | Scharlach | 8 | B |

Verwendet man in den Beispielen 69-81 anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in den für den jeweiligen 5-Chlor-6-dichlormethyl-2-fluor-4-aminopyrimidyl-Farbstoffe angegebenen Tönen färben.

Beispiel 82

In die Lösung von 32 Gew.-Teilen 1-Amino-8-hydroxynaphthalin-3,6-disulfonsäure als Natriumsalz in 200 Teilen Wasser werden unter starkem Rühren bei 15-20° 24 Teile 5-Chlor-6-chlormethyl-2,4-difluorpyrimidin getropft und dabei die freiwerdende Säure durch verdünnte Natronlauge oder Sodalösung neutralisiert. Nach etwa einer Stunde ist die Kondensation unter diesen Bedingungen beendet. Das so erhaltene Kondensationsprodukt wird dann mit der nach üblichen Methoden zu erhaltenden Diazoniumverbindung

aus 0,1 Mol Anilin-2-sulfonsäure vereinigt und die Kupplung bei pH 7 zu Ende geführt. Nach Aussalzen, Isolieren und Trocknen erhält man ein dunkles Pulver des Farbstoffs der Formel

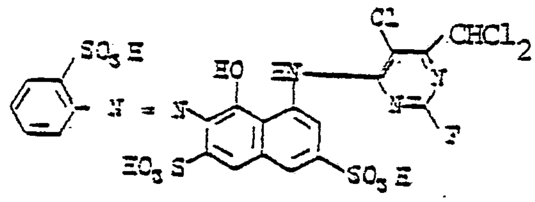

das sich in Wasser mit roter Farbe löst und Baumwolle in brillanten roten Tönen färbt. (CI = 9; GN = B)

Ersetzt man in diesem Beispiel die Diazoniumverbindung aus Anilin-2-sulfonsäure durch die äquimolaren Mengen der aus folgenden Aminen erhältlichen Diazoniumverbindungen, so werden ebenfalls brillante rote Reaktivfarbstoffe erhalten:

| Beispiel | Diazoverbindung | Farbton | CI | GN |
|---|---|---|---|---|
| 83 | Anilin | Rot | 10 | B |
| 84 | Anilin-2,5-disulfonsäure | Rot | 9 | B |
| 85 | 4-Methylanilin-2-sulfonsäure | Rot | 9 | B |
| 86 | 4-Methoxyanilin-2-sulfonsäure | blaust. Rot | 11 | B |
| 87 | 4-Methoxyanilin-2,5-disulfonsäure | blaust. Rot | 11 | B |
| 88 | 1-Amino-5(2'-(2"-sulfophenylamino) 4'-fluortriazin-1',3',5'-ly-6'-)-aminobenzol-2-sulfonsäure | Rot | 9 | B |
| 89 | 1-Amino-5-(5'-chlor-2',6'-difluor-pyrimidyl-4')-aminobenzol-2-sulfonsäure | Rot | 10 | B |

| Beispiel | Diazokomponente | Farbton | CI | GN |
|---|---|---|---|---|
| 90 | 2-Aminonaphthalin-1-sulfonsäure | blaust. Rot | 10 | B |
| 91 | 2-Aminonaphthalin-1,5-disulfonsäure | " | 10 | B |
| 92 | 2-Aminonaphthalin-1,5,7-trisulfonsäure | " | 10 | B |
| 93 | Anilin-4-sulfonsäure | Rot | 9 | B |
| 94 | Anilin-3-sulfonsäure | " | 9 | B |
| 95 | 4-Chloranilin-2-sulfonsäure | " | 9 | B |
| 96 | 2,4-Dichloranilin-6-sulfonsäure | " | 9 | B |
| 97 | 2-Chloranilin-3-sulfonsäure | " | 9 | B |
| 98 | 4-Chloranilin-3-sulfonsäure | " | 9 | B |
| 99 | 2,3,6-Trichloranilin-5-sulfonsäure | " | 8 | B |
| 100 | 2-Chloranilin-4-sulfonsäure | " | 9 | B |
| 101 | 2,5-Dichloranilin | " | 9 | B |
| 102 | 2-Methylanilin-3-sulfonsäure | " | 9 | B |
| 103 | 2-Methylanilin-4-sulfonsäure | " | 9 | B |
| 104 | 2-Chlor-6-methylanilin-4-sulfonsäure | " | 9 | B |
| 105 | 3-Methylanilin-4-sulfonsäure | " | 9 | B |
| 106 | 4-Methylanilin-3-sulfonsäure | " | 9 | B |
| 107 | 5-Methyl-4-chloranilin-2-sulfonsäure | " | 9 | B |
| 108 | 4-Acetylaminoanilin-2-sulfonsäure | " | 9 | B |
| 109 | 2,3-Dichloranilin-6-sulfonsäure | " | 8 | B |
| 110 | 2,4-Dimethylanilin-6-sulfonsäure | " | 8 | B |
| 111 | 2,4-Dimethylanilin-5-sulfonsäure | " | 9 | B |
| 112 | 2-Methyl-4-chloranilin-6-sulfonsäure | " | 8 | B |

Verwendet man in den Beispielen 82-112 anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in roten bis blaustichig roten Tönen färben.

Beispiel 113

Kondensiert man unter den in Beispiel 82 angegebenen Bedingungen je 0,1 Mol 5-Chlor-6-dichlormethyl-2,4-difluorpyrimidin und 1-Amino-8-hydroxynaphthalin-4,6-disulfonsäure und kuppelt das Kondensationsprodukt nach analogen Methoden mit der Diazoniumverbindung aus Anilin-2-sulfonsäure, so erhält man nach Aussalzen, Isolieren und Trocknen den Farbstoff der Formel

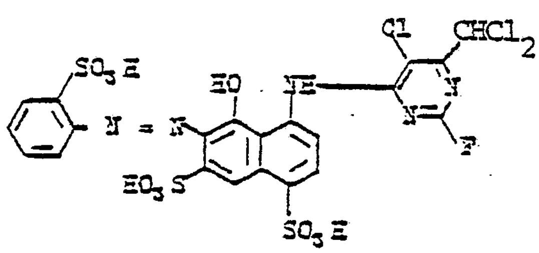

als dunkles Pulver, das sich in Wasser mit gelbstichig roter Farbe löst und zum Bedrucken und Färben von Baumwolle in brillanten roten Farbtönen hervorragend geeignet ist. (CI = 8; GN = B)

Ersetzt man in diesem Beispiel die Diazoniumverbindung aus Anilin-2-sulfonsäure durch äquimolare Mengen der Diazoniumverbindung der in Beispiel 82-112 aufgeführten

Amine, so erhält man ebenfalls brillante rote Reaktivfarbstoffe.

| Diazoverbindung | Farbton | CI | GN |
|---|---|---|---|
| Anilin | Rot | 9 | B |
| Anilin-2,5-disulfonsäure | Rot | 8 | B |
| 4-Methylanilin-2-sulfonsäure | Rot | 8 | B |
| 4-Methoxyanilin-2-sulfonsäure | Rot | 10 | B |
| 4-Methoxyanilin-2,5-disulfonsäure | Rot | 10 | B |
| 1-Amino-5(2'-(2"-sulfophenylamino)-4'-fluortriazin-1',3',5'-ly-6'-)-aminobenzol-2-sulfonsäure | Rot | 8 | B |
| 1-Amino-5-(5'-chlor-2',6'-difluor-pyrimidyl-4')-aminobenzol-2-sulfon-säure | Rot | 9 | B |
| 2-Aminonaphthalin-1-sulfonsäure | Rot | 9 | B |
| 2-Aminonaphthalin-1,5-disulfonsäure | Rot | 9 | B |
| 2-Aminonaphthalin-1,5,7-trisulfonsäure | Rot | 9 | B |
| Anilin-4-sulfonsäure | Rot | 8 | B |
| Anilin-3-sulfonsäure | Rot | 8 | B |
| 4-Chloranilin-2-sulfonsäure | Rot | 8 | B |
| 2,4-Dichloranilin-6-sulfonsäure | Rot | 8 | B |
| 2-Chloranilin-3-sulfonsäure | Rot | 8 | B |
| 4-Chloranilin-3-sulfonsäure | Rot | 8 | B |
| 2,3,6-Trichloranilin-5-sulfonsäure | Rot | 8 | B |
| 2-Chloranilin-4-sulfonsäure | Rot | 8 | B |
| 2,5-Dichloranilin | Rot | 8 | B |
| 2-Methylanilin-3-sulfonsäure | Rot | 8 | B |
| 2-Methylanilin-4-sulfonsäure | Rot | 8 | B |
| 2-Chlor-6-methylanilin-4-sulfonsäure | Rot | 8 | B |
| 3-Methylanilin-4-sulfonsäure | Rot | 8 | B |
| 4-Methylanilin-3-sulfonsäure | Rot | 8 | B |
| 5-Methyl-4-chloranilin-2-sulfonsäure | Rot | 8 | B |
| 4-Acetylaminoanilin-2-sulfonsäure | Rot | 8 | B |
| 2,3-Dichloranilin-6-sulfonsäure | Rot | 8 | B |

| Diazoverbindung | Farbton | CI | GN |
|---|---|---|---|
| 2,4-Dimethylanilin-6-sulfonsäure | Rot | 8 | B |
| 2,4-Dimethylanilin-5-sulfonsäure | Rot | 8 | B |
| 2-Methyl-4-chloranilin-6-sulfonsäure | Rot | 8 | B |

Verwendet man anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in brillant roten Tönen färben.

Beispiel 114

Zu einer Suspension von 0,1 Mol des nach üblichen Methoden hergestellten Aminoazofarbstoffs der Formel

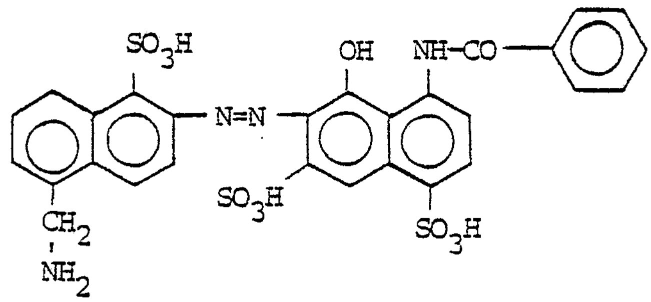

in 100 Teilen Wasser werden bei pH 7 und ca. 30° 24 Teile 5-Chlor-6-dichlormethyl-2,4-difluorpyrimidin zugetropft und dabei der pH-Wert durch Zugabe von verdünnter Natronlauge oder einer Natriumcarbonatlösung im Bereich von 6-8 gehalten.

Nach beendeter Kondensation wird ausgesalzen, isoliert und getrocknet. Der als rotes Pulver anfallende Farbstoff der Formel

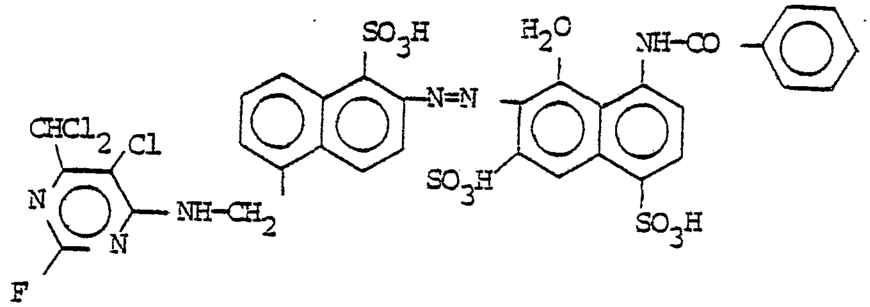

färbt Wolle und Baumwolle in brillant roten Tönen. (CI = 9; GN = B)

Weitere rote Farbstoffe mit ähnlichen Eigenschaften erhält man, wenn man aus den in der folgenden Tabelle aufgeführten Kupplungs- und Diazokomponenten nach üblichen Verfahren Aminoazofarbstoffe herstellt und diese nach dem im obigen Beispiel beschriebenen Verfahren mit 5-Chlor-6-dichlormethyl-2,4-difluorpyrimidin kondensiert.

| Beispiel | Diazokomponente | Kupplungskomponente | Farbton | CI | GN |
|---|---|---|---|---|---|
| 115 | 2-Amino-5-amino-methylennaphthalin-1-sulfonsäure | 1-Benzoylamino-8-hydroxy-naphthalin-3,6-disulfonsäure | Rot | 10 | B |
| 116 | " | 1-Acetylamino-8-hydroxy-naphthalin-3,6-disulfonsäure | " | 9 | B |
| 117 | " | 1-Acetylamino-8-hydroxy-naphthalin-4,6-disulfonsäure | " | 9 | B |
| 118 | " | 1-(2'-(2"-sulfophenylamino)-4'-fluortriazin-1',3',5'-yl-6')-8-hydroxynaphthalin-3,6-disulfonsäure | " | 9 | B |
| 119 | 3-Amino-N-methyl-benzylamin-4-sulfonsäure | 1-Acetylamino-8-hydroxy-naphthalin-3,6-disulfonsäure | Rot | 8 | B |
| 120 | " | 1-Acetylamino-8-hydroxy-naphthalin-4,6-disulfonsäure | " | 7 | B |
| 121 | " | 1-Benzoylamino-8-hydroxy-naphthalin-3,6-disulfonsäure | " | 9 | B |
| 122 | " | 1-Benzoylamino-8-hydroxy-naphthalin-4,6-disulfonsäure | " | 8 | B |

| Beispiel | Diazokomponente | Kupplungskomponente | Farbton | CI | GN |
|---|---|---|---|---|---|
| 123 | 3-Amino-N-methyl-benzylamin-4-sulfonsäure | 1-(2',3'-Dichlorchinoxalin-6'-carbonylamino)-8-hydroxynaphthalin-4,6-disulfonsäure | Rot | 8 | B |
| 124 | 2-Amino-5-aminomethylennaphthalin-1,7-disulfonsäure | " | " | 9 | B |
| 125 | " | 1-Acetylamino-8-hydroxy-naphthalin-3,6-disulfonsäure | " | 10 | B |
| 126 | " | 1-Acetylamino-8-hydroxy-naphthalin-4,6-disulfonsäure | " | 9 | B |
| 127 | " | 1-Benzoylamino-8-hydroxy-naphthalin-3,6-disulfonsäure | " | 9 | B |
| 128 | " | 1-/2'-(2"-Sulfophenylamino)-4'-fluortriazin-1',3',5'-yl-6'/-8-hydroxynaphthalin-3,6-disulfonsäure | " | 9 | B |
| 129 | " | 1-Benzoylamino-8-hydroxy-naphthalin-4,6-disulfonsäure | " | 9 | B |

| Beispiel | Diazokomponente | Kupplungskomponente | Farbton | CI | GN |
|---|---|---|---|---|---|
| 130 | 2-Amino-5-amino-methylennaphthalin-7-sulfonsäure | 1-Benzoylamino-8-hydroxy-naphthalin-4,6-disulfonsäure | Rot | 9 | B |
| 131 | " | 1-Benzoylamino-8-hydroxy-naphthalin-3,6-disulfonsäure | " | 10 | B |

Verwendet man in den Beispielen 114-131 anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in brillanten roten Tönen färben.

Beispiel 132

19 Teile 1,3-Diaminobenzol-4-sulfonsäure werden wie in Beispiel 1 beschrieben mit 23,5 Teilen 5-Chlor-6-dichlormethyl-2,4-difluorpyrimidin zur Reaktion gebracht, diazotiert und nach üblichen Methoden mit 44,5 Teilen 1-Benzoylamino-8-hydroxynaphthalin-4,6-disulfonsäure gekuppelt. Nach beendeter Kupplung wird ausgesalzen, isoliert und getrocknet. Der als rotes Pulver anfallende Farbstoff der Formel

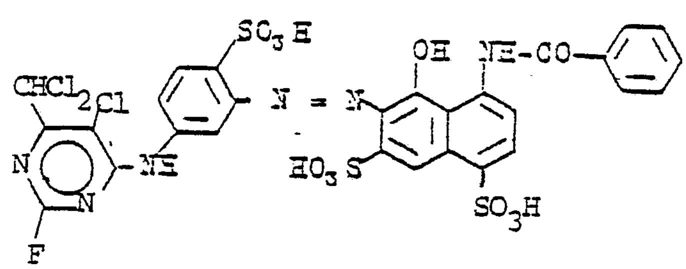

färbt Wolle und Baumwolle in brillanten roten Tönen.
(CI = 8; GN = B)

Weitere orange bis rote Farbstoffe erhält man, wenn man nach obiger Methode die in der folgenden Tabelle Spalte 2 genannten Diazokomponenten mit 5-Chlor-6-dichlormethyl-2,4-difluorpyrimidin umsetzt und danach mit den in Spalte 3 genannten Kupplungskomponenten kuppelt.

| Beispiel | Diazokomponente | Kupplungskomponente | Farbton | CI | GN |
|---|---|---|---|---|---|
| 133 | 1,3-Diaminobenzol-4-sulfonsäure | 1-Acetylamino-8-hydroxynaphthalin-3,6-disulfonsäure | Rot | 8 | B |
| 134 | " | 1-Acetylamino-8-hydroxynaphthalin-4,6-disulfonsäure | " | 7 | B |
| 135 | " | 1-Benzoylamino-8-hydroxynaphthalin-3,6-disulfonsäure | " | 9 | B |
| 136 | " | 1-(2'-(2"-Sulfophenylamino)-4'-fluortriazin-1',3',5'-yl-6')-8-hydroxynaphthalin-3,6-disulfonsäure | " | 9 | B |
| 137 | " | 1-(2',3'-Dichlorchinoxalin-6'-carbonylamino)-8-hydroxynaphthalin-4,6-disulfonsäure | " | 9 | B |
| 138 | " | 1-Hydroxy-naphthalin-4-sulfonsäure | " | 8 | B |
| 139 | " | 1-Hydroxy-naphthalin-5-sulfonsäure | " | 8 | B |

| Beispiel | Diazokomponente | Kupplungskomponente | Farbton | CI | GN |
|---|---|---|---|---|---|
| 140 | 1,3-Diaminobenzol-4-sulfonsäure | 1-Hydroxy-naphthalin-4,6-disulfonsäure | Rot | 8 | B |
| 141 | " | 1-Hydroxy-naphthalin-4,7-disulfonsäure | " | 8 | B |
| 142 | " | 1-Hydroxy-naphthalin-3,6-disulfonsäure | " | 8 | B |
| 143 | " | 2-Acetylamino-5-hydroxynaphthalin-1,7-disulfonsäure | Orange | 6 | B |
| 144 | " | 1-(5'-Chlor-6'-dichlormethyl-2'-fluor-4'-aminopyrimidyl)-8-hydroxynaphthalin-3,6-disulfonsäure | Rot | 8 | B |
| 145 | " | 2-Acetylamino-8-hydroxynaphthalin-6-sulfonsäure | Scharlach | 7 | B |
| 146 | " | 2-Acetylamino-8-hydroxynaphthalin-3,6-disulfonsäure | " | 7 | B |
| 147 | " | 2-Acetylamino-5-hydroxynaphthalin-7-sulfonsäure | Orange | 6 | B |
| 148 | " | 2-Hydroxy-naphthalin-6,8-disulfonsäure | " | 6 | B |
| 149 | " | 2-Benzoylamino-5-hydroxynaphthalin-7-sulfonsäure | " | 6 | B |

| Beispiel | Diazokomponente | Kupplungskomponente | Farbton | CI | GN |
|---|---|---|---|---|---|
| 150 | 1,4-Diaminobenzol-2-sulfonsäure | 2-Benzoylamino-5-hydroxy-naphthalin-7-sulfonsäure | Scharlach | | |
| 151 | " | 1-Acetylamino-8-hydroxynaphthalin-3,6-disulfonsäure | Rot | 10 | B |
| 152 | " | 1-Acetylamino-8-hydroxynaphthalin-4,6-disulfonsäure | " | 9 | B |
| 153 | " | 1-Benzoylamino-8-hydroxynaphthalin-3,6-disulfonsäure | " | 10 | B |
| 154 | " | 1-(2'-(2"-Sulfophenylamino)-4'-fluortriazin-1',3',5'-yl-6')-8-hydroxynaphthalin-3,6-disulfonsäure | " | 10 | B |
| 155 | 1,4-Diaminobenzol-2-sulfonsäure | 1-Benzoylamino-8-hydroxynaphthalin-4,6-disulfonsäure | Rot | 9 | B |
| 156 | " | 2-Acetylamino-8-hydroxynaphthalin-6-sulfonsäure | " | 8 | B |
| 157 | " | 2-Acetylamino-8-hydroxynaphthalin-3,6-disulfonsäure | " | 8 | B |
| 158 | " | 2-Acetylamino-5-hydroxynaphthalin-7-sulfonsäure | " | 8 | B |
| 159 | " | 1-Hydroxy-naphthalin-4-sulfonsäure | " | 8 | |

| Beispiel | Diazokomponente | Kupplungskomponente | Farbton | CI | GN |
|---|---|---|---|---|---|
| 160 | 1,4-Diaminobenzol-2-sulfonsäure | 1-Hydroxy-naphthalin-5-sulfonsäure | Rot | 8 | |
| 161 | " | 1-Hydroxy-naphthalin-4,6-disulfonsäure | " | 8 | B |
| 162 | " | 1-Hydroxy-naphthalin-4,7-disulfonsäure | " | 8 | B |
| 163 | " | 1-Hydroxy-naphthalin-3,6-disulfonsäure | " | 8 | B |
| 164 | " | 2-Acetylamino-5-hydroxynaphthalin-1,7-disulfonsäure | Scharlach | 7 | B |
| 165 | " | 2-Hydroxy-naphthalin-6,8-disulfonsäure | Orange | 6 | B |
| 166 | 1,3-Diamino-2-methylbenzol-5-sulfonsäure | " | " | | |
| 167 | " | 1-Acetylamino-8-hydroxynaphthalin-3,6-disulfonsäure | Rot | 9 | B |
| 168 | " | 1-Acetylamino-8-hydroxynaphthalin-4,6-disulfonsäure | " | 8 | B |
| 169 | " | 1-Benzoylamino-8-hydroxynaphthalin-3,6-disulfonsäure | " | 9 | B |
| 170 | " | 1-Benzoylamino-8-hydroxynaphthalin-4,6-disulfonsäure | " | 8 | B |

| Beispiel | Diazokomponente | Kupplungskomponente | Farbton | CI | GN |
|---|---|---|---|---|---|
| 171 | 1,3-Diamino-2-methylbenzol-5-sulfonsäure | 1-(2',3'-Dichlorchinoxalin-6'-carbonylamino)-8-hydroxy-naphthalin-4,6-disulfonsäure | Rot | 8 | B |
| 172 | " | 2-Acetylamino-8-hydroxynaphthalin-6-sulfonsäure | Scharlach | 7 | B |
| 173 | " | 2-Acetylamino-8-hydroxynaphthalin-3,6-disulfonsäure | " | 7 | B |
| 174 | " | 2-Acetylamino-5-hydroxynaphthalin-7-sulfonsäure | Orange | 6 | B |
| 175 | " | 1-Hydroxy-naphthalin-4-sulfonsäure | Rot | 8 | B |
| 176 | " | 1-Hydroxy-naphthalin-5-sulfonsäure | " | 8 | B |
| 177 | " | 1-Hydroxy-naphthalin-4,6-disulfonsäure | " | 8 | B |
| 178 | " | 1-Hydroxy-naphthalin-4,7-disulfonsäure | " | 8 | B |
| 179 | " | 1-Hydroxy-naphthalin-3,6-disulfonsäure | " | 8 | B |
| 180 | " | 2-Acetylamino-5-hydroxynaphthalin-1,7-disulfonsäure | Orange | 6 | B |
| 181 | " | 2-Benzoylamino-5-hydroxynaphthalin-7-sulfonsäure | " | 6 | B |

| Beispiel | Diazokomponente | Kupplungskomponente | Farbton | CI | GN |
|---|---|---|---|---|---|
| 182 | 1,5-Diaminobenzol-2,4-disulfonsäure | Benzoylamino-5-hydroxynaphthalin-7-sulfonsäure | Orange | | |
| 183 | " | 1-Acetylamino-8-hydroxynaphthalin-3,6-disulfonsäure | Rot | 9 | B |
| 184 | " | 1-Acetylamino-8-hydroxynaphthalin-4,6-disulfonsäure | " | 8 | B |
| 185 | " | 1-Benzoylamino-8-hydroxynaphthalin-3,6-disulfonsäure | " | 9 | B |
| 186 | " | 1-(2'-(2"-Sulfophenylamino)-4'-fluortriazin-1',3',5'-yl-6')-8-hydroxynaphthalin-3,6-disulfonsäure | " | 9 | B |
| 187 | 1,5-Diaminobenzol-2,4-disulfonsäure | 1-Benzoylamino-8-hydroxynaphthalin-4,6-disulfonsäure | Rot | 8 | B |
| 188 | " | 2-Acetylamino-8-hydroxynaphthalin-6-sulfonsäure | " | 8 | B |
| 189 | " | 2-Acetylamino-8-hydroxynaphthalin-3,6-disulfonsäure | " | 9 | B |
| 190 | " | 2-Acetylamino-8-hydroxynaphthalin-7-sulfonsäure | Orange | 6 | B |
| 191 | " | 1-Hydroxy-naphthalin-4-sulfonsäure | Rot | 7 | B |

| Beispiel | Diazokomponente | Kupplungskomponente | Farbton | CI | GN |
|---|---|---|---|---|---|
| 192 | 1,5-Diaminobenzzol-2,4-disulfonsäure | 1-Hydroxy-naphthalin-2-sulfonsäure | Rot | 7 | B |
| 193 | " | 1-Hydroxy-naphthalin-4,6-disulfonsäure | " | 7 | B |
| 194 | " | 1-Hydroxy-naphthalin-4,7-disulfonsäure | " | 7 | B |
| 195 | " | 1-Hydroxy-naphthalin-3,6-disulfonsäure | " | 7 | B |
| 196 | " | 2-Acetylamino-5-hydroxynaphthalin-1,7-disulfonsäure | Orange | 6 | B |
| 197 | " | 2-Hydroxynaphthalin-6,8-disulfonsäure | " | 6 | B |

Verwendet man in den Beispielen 132-197 anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in roten bis orange Tönen färben.

Beispiel 198

44 Teile 1-(4'-Aminobenzoylamino)-8-hydroxynaphthalin-3,6-disulfonsäure werden in 200 Teilen Wasser gelöst und unter starkem Rühren bei 15-20° mit 24 Teilen 5-Chlor-6-dichlormethyl-2,4-difluorpyrimidin versetzt. Dabei wird die freiwerdende Flußsäure auf pH 5-5,5 mit Sodalösung abgestumpft. Nach etwa zwei Stunden ist die Kondensation unter diesen Bedingungen beendet.

Das so erhaltene Kondensationsprodukt wird dann mit der nach üblichen Methoden zu erhaltenden Diazoniumverbindung aus 0,1 Mol Anilin-2-sulfonsäure vereinigt und die Kupplung bei pH 7 zu Ende geführt. Nach Aussalzen, Isolieren und Trocknen erhält man ein dunkles Pulver des Farbstoffs der Formel

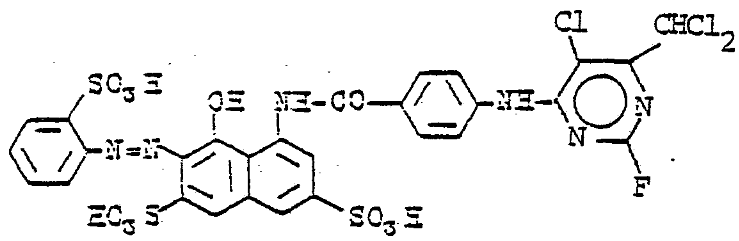

(CI = 8; GN = B)

Weitere Farbstoffe mit den angegebenen Farbtönen erhält man, wenn man nach obiger Methode die in der folgenden Tabelle Zeile 1 genannten Kupplungskomponenten mit 5-Chlor-6-dichlormethyl-2,4-difluorpyrimidin umsetzt und danach mit den in Spalte 1 genannten Diazokomponenten kuppelt.

| Kupplungs-komponente / Diazo-komponente | Farbton: -8-Hydroxynaphthalin-3,6-disulfonsäure- 1-(4'-amino-benzoylamino) | -8-Hydroxynaphthalin-3,6-disulfonsäure- 1-(3'-amino-benzoylamino) | -8-Hydroxynaphthalin-4,6-disulfonsäure- 1-(4'-amino-benzoylamino) | -8-Hydroxynaphthalin-4,6-disulfonsäure- 1-(3'-amino-benzoylamino) |
|---|---|---|---|---|
| Aminobenzol | rot | rot | rot | rot |
| 1-Aminobenzol-3-sulfonsäure | " | " | " | " |
| 1-Aminobenzol-4-sulfonsäure | " | " | " | " |
| 1-Amino-5-chlorbenzol-2-sulfonsäure | " | " | " | " |
| 1-Amino-2-carboxybenzol-4-sulfonsäure | " | " | " | " |
| 1-Amino-4-methylbenzol-2-sulfonsäure | " | " | " | " |
| 1-Amino-4-chlorbenzol-2-sulfonsäure | " | " | " | " |
| 2,4-Dichloranilin-6-sulfonsäure | " | " | " | " |
| 2-Chloranilin-3-sulfonsäure | " | " | " | " |
| 4-Chloranilin-3-sulfonsäure | " | " | " | " |
| 2-Chloranilin-4-sulfonsäure | " | " | " | " |

| Kupplungs-komponente / Diazo-komponente | Farbton: -8-Hydroxynaphthalin-3,6-disulfonsäure-1-(4'-amino-benzoylamino) | -8-Hydroxynaphthalin-3,6-disulfonsäure-1-(3'-amino-benzoylamino) | -8-Hydroxynaphthalin-4,6-disulfonsäure-1-(4'-amino-benzoylamino) | -8-Hydroxynaphthalin-4,6-disulfonsäure-1-(3'-amino-benzoylamino) |
|---|---|---|---|---|
| 2-Methylanilin-3-sulfonsäure | rot | rot | rot | rot |
| 2-Methylanilin-4-sulfonsäure | " | " | " | " |
| 2-Chlor-6-methyl-anilin-4-sulfonsäure | " | " | " | " |
| 3-Methylanilin-4-sulfonsäure | " | " | " | " |
| Anilin-2-sulfonsäure | " | " | " | " |
| 4-Methylanilin-3-sulfonsäure | " | " | " | " |
| 5-Methyl-4-chlor-anilin-2-sulfonsäure | " | " | " | " |
| 2,3-Dichloranilin-6-sulfonsäure | " | " | " | " |
| 2,4-Dimethylanilin-6-sulfonsäure | " | " | " | " |
| 2,4-Dimethylanilin-5-sulfonsäure | " | " | " | " |
| 2-Methyl-4-chloranilin-6-sulfonsäure | " | " | " | " |

| Kupplungskomponente / Diazokomponente | Farbton: -8-Hydroxynaphthalin-3,6-disulfonsäure- 1-(4'-amino-benzoylamino) | 1-(3'-amino-benzoylamino) | -8-Hydroxynaphthalin-4,6-disulfonsäure- 1-(4'-amino-benzoylamino) | 1-(3'-amino-benzoylamino) |
|---|---|---|---|---|
| Anilin-2,5-disulfonsäure | rot | rot | rot | rot |
| 1-Amino-5-acetylamino-benzol-2-sulfonsäure | blaust. rot | blaust. rot | " | " |
| 1-Amino-5-(2'-/2"-sulfophenylamino/-4'-fluor-triazin-1',3',5'-yl-6')-aminobenzol-2-sulfonsäure | " | " | " | " |
| 1-Amino-5-(5'-chlor-2',6'-difluordiazin-1',3'-yl-4'-amino)-benzol-2-sulfonsäure | " | " | " | " |
| 1-Amino-5-(2',3'-dichlorchinoxalin-6-carboxylamino)-benzol-2-sulfonsäure | " | " | " | " |
| 2-Amino-naphthalin-1-sulfonsäure | " | " | blaust. rot | blaust. rot |
| 2-Amino-naphthalin-1,5-disulfonsäure | " | " | " | " |
| 2-Amino-naphthalin-1,7-disulfonsäure | " | " | " | " |

| Kupplungskomponente / Diazokomponente | Farbton: -8-Hydroxynaphthalin-3,6-disulfonsäure- 1-(4'-aminobenzoylamino) | -8-Hydroxynaphthalin-3,6-disulfonsäure- 1-(3'-aminobenzoylamino) | -8-Hydroxynaphthalin-4,6-disulfonsäure- 1-(4'-aminobenzoylamino) | -8-Hydroxynaphthalin-4,6-disulfonsäure- 1-(3'-aminobenzoylamino) |
|---|---|---|---|---|
| 1-Amino-naphthalin-3,6-disulfonsäure | blaust. rot | blaust. rot | blautst. rot | blaust. rot |
| 2-Amino-naphthalin-3,7-disulfonsäure | " | " | " | " |
| 2-Amino-naphthalin-4,8-disulfonsäure | " | " | " | " |
| 2-Amino-naphthalin-1,5,7-trisulfonsäure | " | " | " | " |
| 1-Amino-4-chlorbenzol-2-sulfonsäure | " | " | " | " |
| 1-Amino-4-acetylamino-benzol-2-sulfonsäure | Violett | Violett | " | " |
| 1-Amino-4-methoxybenzol-2-sulfonsäure | Violett | Violett | rotst.Violett | rotst.Violett |
| 1-Amino-5-(5'-chlor-6'-dichlorfluormethyl-2'-fluor-4'-aminopyrimidyl)-benzol-2-sulfonsäure | Rot | Rot | Rot | Rot |

Verwendet man anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in roten bis violetten Tönen färben.

Beispiel 199

In eine Suspension von 0,1 Mol des Diazoniumsalzes von 1-Amino-5-(5'-chlor-6-dichlormethyl-2-fluor-pyrimidyl-4)-aminobenzol-2-sulfonsäure (hergestellt nach Beispiel 1) wird eine mit Natriumacetat gepufferte Lösung von 24 g 2-Amino-8-hydroxy-naphthalin-6-sulfonsäure in 80 ml Wasser bei pH 2-3 gegeben. Es erfolgt Kupplung zu dem Farbstoff der Formel

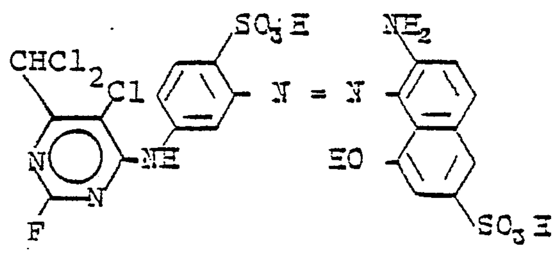

Nach beendeter Kupplung wird der Farbstoff ausgesalzen, isoliert und bei 30-40°C im Vakuum getrocknet. Er löst sich in Wasser und färbt Wolle in roten Tönen mit guten Naß und Lichtechtheiten. (CI = 8; GN = B).

Weitere orange bis blaustichig rote Farbstoffe erhält man, wenn man die in der folgenden Tabelle Spalte 2 genannten Diazokomponenten mit 5-Chlor-6-dichlormethyl-2,4-difluorpyrimidin nach der in Beispiel 1 beschriebenen Methode umsetzt und diazotiert und danach mit den in Spalte 3 genannten Kupplungskomponenten nach bekannten Methoden kuppelt.

| Beispiel | Diazokomponente | Kupplungskomponente | Farbton | CI | GN |
|---|---|---|---|---|---|
| 200 | 1,3-Diaminobenzol-4-sulfonsäure | 2-N-Methylamino-8-hydroxynaphthalin-6-sulfonsäure | blaust. rot | 9 | B |
| 201 | " | 2-Amino-8-hydroxynaphthalin--5-sulfonsäure | rot | 8 | B |
| 202 | " | 2-Amino-8-hydroxynaphthalin-3,6-disulfonsäure | blaust. rot | 9 | B |
| 203 | " | 2-Aminonaphthalin-5-sulfonsäure | Orange | 6 | B |
| 204 | " | 2-Aminonaphthalin-6-sulfonsäure | " | 6 | B |
| 205 | " | 2-Aminonaphthalin-7-sulfonsäure | " | 6 | B |
| 206 | " | 7-Aminonaphthalin-1-sulfonsäure | " | 6 | B |
| 207 | " | 2-Aminonaphthalin-3,6-disulfonsäure | " | 6 | B |
| 208 | " | 2-Aminonaphthalin-3,7-disulfonsäure | " | 6 | B |
| 209 | " | 6-Aminonaphthalin-1,3-disulfonsäure | " | 6 | B |
| 210 | " | 2-N-Methylaminonaphthalin-7-sulfonsäure | rotst. Orange | 6 | B |
| 211 | " | 2-Amino-8-hydroxynaphthalin-5,7-disulfonsäure | blaust Rot | 9 | B. |

| Beispiel | Diazokomponente | Kupplungskomponente | Farbton | CI | GN |
|---|---|---|---|---|---|
| 212 | 1,4-Diaminobenzol-2-sulfonsäure | 2-Amino-8-hydroxynaphthalin-6-sulfonsäure | blaust. Rot | 9 | B |
| 213 | " | 2-N-Methylamino-8-hydroxynaphthalin-6-sulfonsäure | " | 9 | B |
| 214 | " | 2-Amino-8-hydroxynaphthalin-5-sulfonsäure | " | 9 | B |
| 215 | " | 2-Amino-8-hydroxynaphthalin-3,6-disulfonsäure | " | 9 | B |
| 216 | " | 2-Aminonaphthalin-5-sulfonsäure | rotst. Orange | 6 | B |
| 217 | " | 2-Aminonaphthalin-6-sulfonsäure | " | 6 | B |
| 218 | " | 2-Aminonaphthalin-7-sulfonsäure | " | 6 | B |
| 219 | " | 7-Aminonaphthalin-1-sulfonsäure | " | 6 | B |
| 220 | " | 2-Aminonaphthalin-3,6-disulfonsäure | " | 6 | B |
| 221 | " | 2-Aminonaphthalin-3,7-disulfonsäure | " | 6 | B |
| 222 | " | 6-Aminonaphthalin-1,3-disulfonsäure | " | 6 | B |
| 223 | " | 2-N-Methylaminonaphthalin-7-sulfonsäure | rot | 8 | B |
| 224 | " | 2-Amino-8-hydroxynaphthalin-5,7-disulfonsäure | blaust. Rot | 9 | B |

| Beispiel | Diazokomponente | Kupplungskomponente | Farbton | CI | GN |
|---|---|---|---|---|---|
| 225 | 2,5-Diamino-4-methyl-3'-sulfo-1,1'-diphenylsulfon | 2-Amino-8-hydroxynaphthalin-6-sulfonsäure | blaust. Rot | 9 | B |
| 226 | " | 2-N-Methylamino-8-hydroxynaphthalin-6-sulfonsäure | " | 9 | B |
| 227 | " | 2-Amino-8-hydroxynaphthalin-5-sulfonsäure | " | 9 | B |
| 228 | 2,5-Diamino-4-methyl-3'-sulfo-1,1'-diphenylsulfon | 2-Amino-8-hydroxynaphthalin-3,6-disulfonsäure | blaust. Rot | 9 | B |
| 229 | " | 2-Aminonaphthalin-5-sulfonsäure | gelbst. Rot | 7 | B |
| 230 | " | 2-Aminonaphthalin-6-sulfonsäure | " | 7 | B |
| 231 | " | 2-Aminonaphthalin-7-sulfonsäure | " | 7 | B |
| 232 | " | 7-Aminonaphthalin-1-sulfonsäure | " | 7 | B |
| 233 | " | 2-Aminonaphthalin-3,6-disulfonsäure | " | 7 | B |
| 234 | " | 2-Aminonaphthalin-3,7-disulfonsäure | " | 7 | B |
| 235 | " | 6-Aminonaphthalin-1,3-disulfonsäure | " | 7 | B |

| Beispiel | Diazokomponente | Kupplungskomponente | Farbton | CI | GN |
|---|---|---|---|---|---|
| 236 | 2,5-Diamino-4-methyl-3'-sulfo-1,1'-diphenylsulfon | 2-N-Methylaminonaphthalin-7-sulfonsäure | Rot | 8 | B |
| 237 | " | 2-Amino-8-hydroxynaphthalin-5,7-disulfonsäure | blaust. Rot | 9 | B |

Verwendet man in den Beispielen 199-237 anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Borm-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in orange bis blaustichig roten Tönen färben.

Beispiel 238

Zu einer Suspension von 0,1 Mol des nach üblichen Methoden hergestellten Aminoazofarbstoffs der Formel

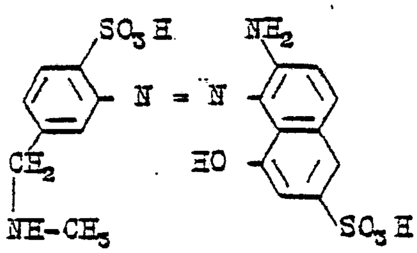

in 100 ml Wasser werden bei pH 7 und ca. 30°C 24 Teile 5-Chlor-6-dichlormethyl-2,4-difluorpyrimidin zugetropft und dabei der pH-Wert durch Zugabe von verdünnter Natronlauge oder einer Natriumcarbonat-Lösung im Bereich von 6-8 gehalten. Nach beendeter Kondensation wird ausgesalzen, isoliert und getrocknet. Der als rotes Pulver anfallende Farbstoff der Formel

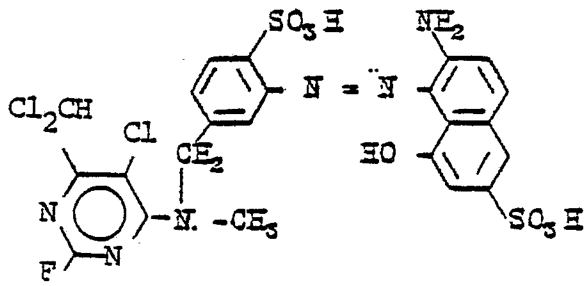

färbt Wolle und Baumwolle in roten echten Tönen.
(CI = 8; GN = B)

Weitere orange bis rote Farbstoffe mit ähnlichen Eigenschaften erhält man, wenn man aus den in der folgenden Tabelle aufgeführten Kupplungs- und Diazokomponenten nach üblichen Verfahren Aminoazofarbstoffe herstellt und diese nach dem im obigen Beispiel beschriebenen Verfahren mit 5-Chlor-6-dichlormethyl-2,4-difluorpyrimidin kondensiert.

| Beispiel | Diazokomponente | Kupplungskomponent | Farbton | CI | GN |
|---|---|---|---|---|---|
| 239 | 3-Amino-N-methyl-benzylamin-4-sulfonsäure | 2-N-Methylamino-8-hydroxynaphthalin-6-sulfonsäure | blaust. Rot | 9 | B |
| 240 | " | 2-Amino-8-hydroxynaphthalin-5-sulfonsäure | Rot | 8 | B |
| 241 | " | 2-Amino-8-hydroxynaphthalin-3,6-disulfonsäure | blaust. Rot | 9 | B |
| 242 | " | 2-Aminonaphthalin-5-sulfonsäure | Orange | 6 | B |
| 243 | " | 2-Aminonaphthalin-6-sulfonsäure | " | 6 | B |
| 244 | " | 2-Aminonaphthalin-7-sulfonsäure | " | 6 | B |
| 245 | " | 7-Aminonaphthalin-1-sulfonsäure | " | 6 | B |
| 246 | " | 2-Aminonaphthalin-3,6-disulfonsäure | " | 6 | B |
| 247 | " | 2-Aminonaphthalin-3,7-disulfonsäure | " | 6 | B |
| 248 | " | 6-Aminonaphthalin-1,3-disulfonsäure | " | 6 | B |
| 249 | " | 2-N-Methylaminonaphthalin-7-sulfonsäure | rotst. Orange | 7 | B |
| 250 | " | 2-Amino-8-hydroxynaphthalin-5,7-disulfonsäure | blaust. Rot | 9 | B |

| Beispiel | Diazokomponente | Kupplungskomponent | Farbton | CI | GN |
|---|---|---|---|---|---|
| 251 | 2-Amino-5-amino-methylnaphthalin-1-sulfonsäure | 2-N-Methylamino-8-hydroxynaphthalin-6-sulfonsäure | blaust. Rot | | |
| 252 | " | 2-Amino-8-hydroxynaphthalin-5-sulfonsäure | Rot | 8 | B |
| 253 | " | 2-Amino-8-hydroxynaphthalin-3,6-disulfonsäure | blaust. Rot | 9 | B |
| 254 | " | 2-Aminonaphthalin-5-sulfonsäure | Orange | 6 | B |
| 255 | 2-Amino-5-amino-methylennaphthalin-1-sulfonsäure | 2-Aminonaphthalin-6-sulfonsäure | Orange | 6 | B |
| 256 | " | 2-Aminonaphthalin-7-sulfonsäure | " | 6 | B |
| 257 | " | 7-Aminonaphthalin-1-sulfonsäure | " | 6 | B |
| 258 | " | 2-Aminonaphthalin-3,6-disulfonsäure | " | 6 | B |
| 259 | " | 2-Aminonaphthalin-3,7-disulfonsäure | " | 6 | B |
| 260 | " | 6-Aminonaphthalin-1,3-disulfonsäure | " | 6 | B |
| 261 | " | 2-Amino-8-hydroxynaphthalin-6-sulfonsäure | Rot | 8 | B |

| Beispiel | Diazokomponente | Kupplungskomponent | Farbton | CI | GN |
|---|---|---|---|---|---|
| 262 | 2-Amino-5-amino-methylennaphthalin-1,7-disulfonsäure | 2-Amino-8-hydroxynaphthalin-6-sulfonsäure | Rot | 8 | B |
| 263 | " | 2-N-Methylamino-8-hydroxynaphthalin-6-sulfonsäure | blaust. Rot | 9 | B |
| 264 | " | 2-Amino-8-hydroxynaphthalin-3,6-disulfonsäure | Rot | 8 | B |
| 265 | " | 2-Aminonaphthalin-5-sulfonsäure | Orange | 6 | B |
| 266 | " | 2-Aminonaphthalin-6-sulfonsäure | " | 6 | B |
| 267 | " | 2-Aminonaphthalin-7-sulfonsäure | " | 6 | B |
| 268 | " | 7-Aminonaphthalin-1-sulfonsäure | " | 6 | B |
| 269 | " | 2-Aminonaphthalin-3,6-disulfonsäure | " | 6 | B |
| 270 | 2-Amino-5-aminomethylennaphthalin-1,7-disulfonsäure | 2-Aminonaphthalin-3,7-disulfonsäure | Orange | 6 | B |
| 271 | " | 6-Aminonaphthalin-1,3-disulfonsäure | " | 6 | B |

| Beispiel | Diazokomponente | Kupplungskomponent | Farbton | CI | GN |
|---|---|---|---|---|---|
| 272 | 2-Amino-5-aminomethylennaphthalin-7-sulfonsäure | 2-Amino-8-hydroxynaphthalin-6-sulfonsäure | Rot | 8 | B |
| 273 | " | 2-N-Methylamino-8-hydroxynaphthalin-6-sulfonsäure | blaust. Rot | 9 | B |
| 274 | " | 2-Amino-8-hydroxynaphthalin-3,6-disulfonsäure | " | 9 | B |
| 275 | " | 2-Aminonaphthalin-5-sulfonsäure | Orange | 6 | B |
| 276 | " | 2-Aminonaphthalin-6-sulfonsäure | " | 6 | B |
| 277 | " | 2-Aminonaphthalin-7-sulfonsäure | " | 6 | B |
| 278 | " | 7-Aminonaphthalin-1-sulfonsäure | " | 6 | B |
| 279 | " | 2-Aminonaphthalin-3,6-disulfonsäure | " | 6 | B |
| 280 | " | 2-Aminonaphthalin-3,7-disulfonsäure | " | 6 | B |
| 281 | " | 6-Aminonaphthalin-1,3-disulfonsäure | " | 6 | B |

Verwendet man in den Beispielen 238-281 anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl-, oder 5-Brom-6-trichlormethyl-2,4-difluorpyrimidin und verfährt sonst wie oben beschrieben, so erhält man Farbstoffe die Wolle umd Baumwolle in orange bis rote Töne färben.

Beispiel 282

Zu einer Suspension von 0,1 Mol des nach üblichen Methoden hergestellten Aminoazofarbstoffs der Formel

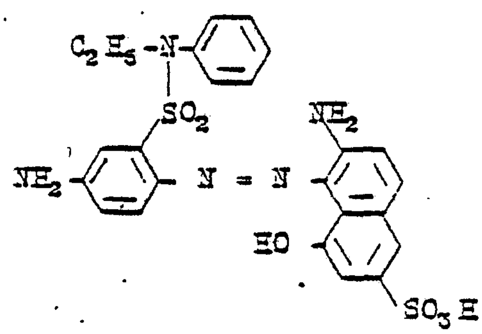

in 1200 ml Wasser werden bei pH 6 und ca. 30°C 24 Teile 5-Chlor-6-dichlormethyl-2,4-difluorpyrimidin zugetropft und dabei der pH-Wert durch Zugabe von verdünnter Natronlauge oder einer Natriumcarbonat-Lösung im Bereich von 6-7 gehalten. Nach beendeter Kondensation wird ausgesalzen, isoliert und getrocknet. Der als rotes Pulver anfallende Farbstoff der Formel

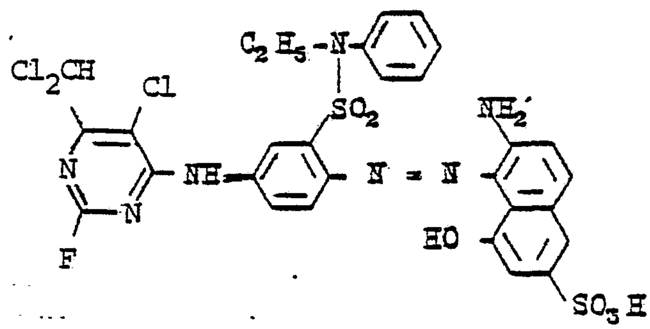

färbt Wolle und Baumwolle in roten echten Tönen.
(CI = 7; GN = B)

Weitere orange bis rote Farbstoffe mit ähnlichen Eigenschaften erhält man, wenn im obigen Beispiel 2-Amino-8-hydroxynaphthalin-6-sulfonsäure durch die in der nachfolgenden Tabelle aufgeführten Kupplungskomponenten ersetzt und zwar nach an sich bekannten Methoden und die so erhaltenen Farbstoffe mit 5-Chlor-6-dichlormethyl-2,4-difluorpyrimidin kondensiert.

| Beispiel | Kupplungskomponente | Farbton | CI | GN |
|---|---|---|---|---|
| 239 | 2-N-Methylamino-8-hydroxynaphthalin-6-sulfonsäure | blaust. Rot | 10 | |
| 240 | 2-Amino-8-hydroxynaphthalin-5-sulfonsäure | " | 9 | |
| 241 | 2-Amino-8-hydroxynaphthalin-3,6-disulfonsäure | " | 10 | D |
| 242 | 2-Aminonaphthalin-5-sulfonsäure | gelbst. Rot | 7 | |
| 243 | 2-Aminonaphthalin-6-sulfonsäure | " | 7 | |
| 244 | 2-Aminonaphthalin-7-sulfonsäure | " | 7 | |
| 245 | 2-Aminonaphthalin-1-sulfonsäure | " | 7 | |
| 246 | 2-Aminonaphthalin-3,6-disulfonsäure | " | 7 | |
| 247 | 2-Aminonaphthalin-3,7-disulfonsäure | " | 7 | |
| 248 | 6-Aminonaphthalin-1,3-disulfonsäure | " | 7 | |
| 249 | 5-Aminonaphthalin-1,3-disulfonsäure | " | 7 | |
| 250 | 2-N-Methylaminonaphthalin-7-sulfonsäure | Rot | 8 | |
| 251 | 2-Amino-8-hydroxynaphthalin-5,7-disulfonsäure | blaust. Rot | 10 | |

Verwendet man in den Beispielen 238-251 anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in orange bis blaustichig roten Tönen färben.

Beispiel 252

Zu einer Suspension von 0,1 Mol des nach üblichen Methoden hergestellten Aminoazofarbstoffs der Formel

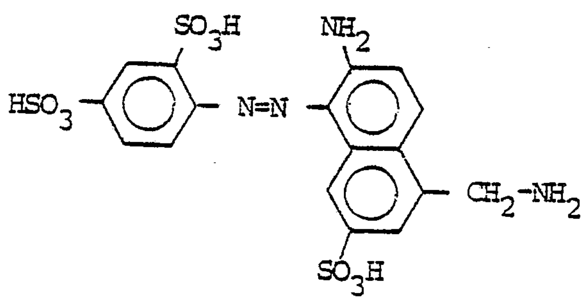

in 1000 ml Wasser werden bei pH 7 und ca. 30°C 24 Teile 5-Chlor-6-dichlormethyl-2,4-difluorpyrimidin zugetropft und dabei der pH-Wert durch Zugabe von verdünnter Natronlauge oder einer Natriumcarbonat-Lösung im Bereich von 6-8 gehalten. Nach beendeter Kondensation wird ausgesalzen, isoliert und getrocknet. Der als rotes Pulver anfallende Farbstoff der Formel

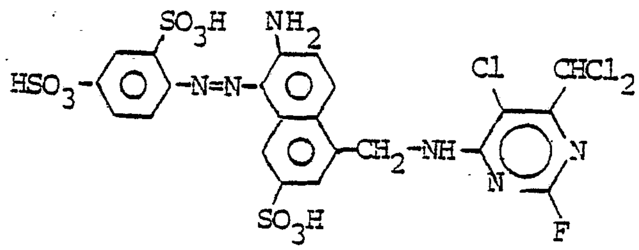

färbt Wolle und Baumwolle in echten roten Tönen.
(CI = 7; GN = B)

Weitere orange bis rote Farbstoffe mit ähnlichen Eigenschaften erhält man, wenn man aus den in der folgenden Tabelle aufgeführten Kupplungs- und Diazokomponente nach üblichen Verfahren Aminoazofarbstoffe herstellt und diese nach dem im obigen Beispiel beschriebenen Verfahren mit 5-Chlor-6-dichlormethyl-2,4-difluorpyrimidin kondensiert.

| Beispiel | Diazokomponente | Kupplungskomponente | Farbton | CI | GN |
|---|---|---|---|---|---|
| 253 | 1-Aminobenzol-2,5-disulfonsäure | 2-Amino-5-aminomethylen-naphthalin-1,7-disulfonsäure | gelbst. Rot | 7 | B |
| 254 | 1-Amino-2,5-dichlorbenzol-4-sulfonsäure | " | Orange | 6 | B |
| 255 | 1-Amino-5-benzoylamino-benzol-2-sulfonsäure | " | " | 6 | B |
| 256 | 1-Amino-4-benzoylamino-benzol-2-sulfonsäure | " | " | 6 | B |
| 257 | 1-Amino-4-acetylamino-benzol-2,5-disulfonsäure | " | Scharlach | 7 | B |
| 258 | 1-Amino-5-acetylamino-benzol-2,4-disulfonsäure | " | Rot | 8 | B |
| 259 | 1-Aminobenzol-2-sulfonsäure | " | Orange | 6 | B |
| 260 | 1-Aminobenzol-2,4-disulfonsäure | 2-Amino-5-aminomethylen-naphthalin-1-sulfonsäure | Rot | 8 | B |
| 261 | 1-Aminobenzol-2,5-disulfonsäure | " | gelbst. Rot | 7 | B |
| 262 | 1-Amino-2,5-dichlor-benzol-4-sulfonsäure | " | Orange | 6 | B |
| 263 | 1-Amino-5-benzoylamino-benzol-2-sulfonsäure | " | " | 6 | B |
| 264 | 1-Amino-4-benzoylamino-benzol-2-sulfonsäure | " | " | 6 | B |

| Beispiel | Diazokomponente | Kupplungskomponente | Farbton | CI | GN |
|---|---|---|---|---|---|
| 265 | 1-Amino-4-acetylamino-benzol-2,5-disulfon-säure | 2-Amino-5-aminomethylen-naphthalin-1-sulfonsäure | Scharlach | 7 | B |
| 266 | 1-Amino-5-acetylamino-benzol-2,4-disulfon-säure | " | Rot | 8 | B |
| 267 | 1-Aminobenzol-2-sulfon-säure | " | " | 8 | B |

Beispiel 267

Zur Lösung von 0,1 Mol des nach üblichen Methoden hergestellten Aminoazofarbstoffs der Formel

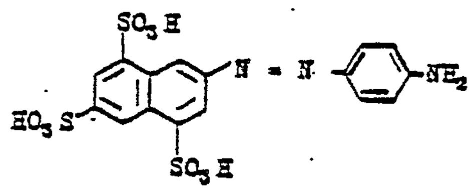

in 1000 Teilen Wasser werden bei pH 7 und ca. 30° 24 Teile 6-Dichlormethyl-5-chlor-2,4-difluorpyrimidin zugetropft und dabei der pH-Wert durch Zugabe von verdünnter Natronlauge oder einer Natriumcarbonatlösung im Bereich von 6-8 gehalten.

Nach beendeter Kondensation wird ausgesalzen, isoliert und getrocknet. Der als gelbes Pulver anfallende Farbstoff der Formel

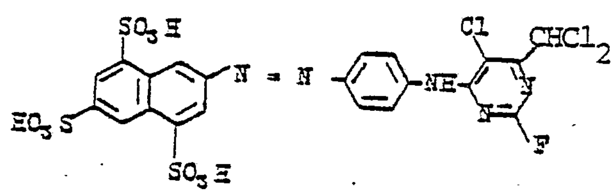

färbt Baumwolle in licht- und chlorechten gelben Farbtönen. (CI = 3; GN = B)

Wird in diesem Beispiel die 2-Aminonaphthalin-4,6,8-trisulfonsäure durch 2-Aminonaphthalin-4,8-disulfonsäure,

2-Aminonaphthalin-6,8-disulfonsäure, 2-Aminonaphthalin-3,6,8-trisulfonsäure, Aminobenzol-2,4-disulfonsäure oder Aminobenzol-2,5-disulfonsäure ersetzt, so werden ebenfalls gelbe Reaktivfarbstoffe hervorragender Licht- und Naßechtheiten erhalten.

In analoger Weise können gelbe Reaktivfarbstoffe dieses Typs erhalten werden, wenn in diesem Beispiel das als Kupplungskomponente eingesetzte Anilin durch 3-Methylanilin, 2-Methoxyanilin, 2,5-Dimethoxyanilin, 3-Acetylaminoanilin, 3-Ureidoanilin oder durch 2-Methoxy-5-methylanilin ersetzt wird.

Verwendet man anstelle von 5-Chlor-6-chlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in gelben Tönen färben.

Beispiel 268

Zur Lösung der nach üblichen Methoden aus 0,1 Mol 2-Aminonaphthalin-4,6,8-trisulfonsäure zu erhaltenden Diazoniumverbindung werden 0,1 Mol des nach Beispiel 1 zu erhaltenden Kondensationsproduktes aus 19 Teilen 1,3-Diaminobenzol-6-sulfonsäure und 23,5 Teilen 5-Chlor-6-dichlormethyl-2,4-difluorpyrimidin gegeben und die Kupplung durch Neutralisation mit einer verdünnten Sodalösung zu Ende geführt.

Nach Aussalzen, Filtrieren und Trocknen wird der Farbstoff der Formel

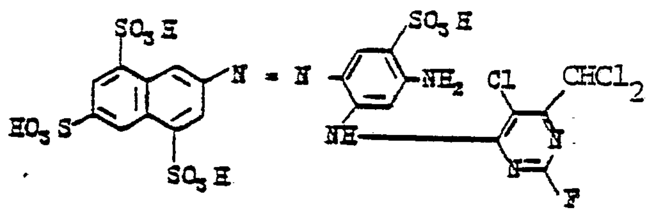

in Form eines gelben Pulvers erhalten. Er eignet sich in hervorragender Weise zum Färben und Bedrucken von Baumwolle in goldgelben Farbtönen. (CI = 4; GN = B)

Wird in diesem Beispiel die 2-Aminonaphthalin-4,6,8-trisulfonsäure durch 2-Aminonaphthalin-4,6,8-disulfonsäure, 2-Aminonaphthalin-6,8-disulfonsäure, 2-Aminonaphthalin-3,6,8-trisulfonsäure, Aminobenzol-2,4-disulfonsäure oder Aminobenzol-2,5-disulfonsäure ersetzt, so werden ebenfalls gelbe Reaktivfarbstoffe mit hervorragenden Naßechtheiten erhalten.

Verwendet man anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in goldgelben Tönen färben.

Beispiel 269

In die Lösung des Natriumsalzes von 32 Gew.-Teilen 1-Amino-8-hydroxynaphthalin-3,6-disulfonsäure in 200 Teilen Wasser werden bei pH 5,5-6 wie in Beispiel 82 angegeben 24 Teile 5-Chlor-6-dichlormethyl-2,4-difluorpyrimidin gegeben und bei 15-20° kondensiert. Die Lösung des Kondensationsproduktes wird dann mit der Diazoniumverbindung aus 0,1 Mol 2-Aminonaphthalin-4,6,8-trisulfonsäure in 200 Teilen Wasser vereinigt und durch Zugabe von Alkali die Kupplung bei pH 7 durchgeführt.

Nach beendeter Kupplung wird die rote Farbstofflösung mit 35 Gew.-Teilen Kupfersulfat versetzt und bei 15° und pH 6,4 eine Mischung aus 50 Teilen einer 30 %igen Wasserstoffperoxidlösung und 130 Teilen einer 20 %igen Natriumacetatlösung zugetropft. Durch Zugabe von ca. 200 g Eis wird dabei die Temperatur im Bereich von 10-15° gehalten. Nach beendeter oxydativer Kupferung wird der Kupferkomplexfarbstoff der Formel

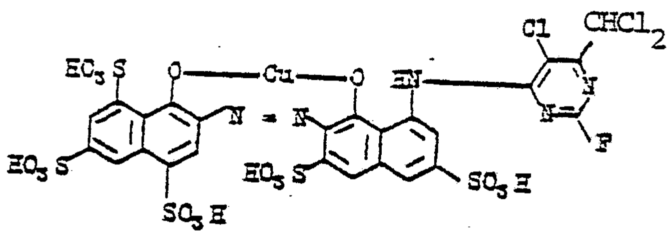

ausgesalzen, isoliert und getrocknet. Man erhält ein dunkles Pulver, das sich in Wasser mit blauer Farbe löst und zum Bedrucken und Färben von Baumwolle in marineblauen Farbtönen mit guter Lichtechtheit geeignet ist. (CI = 38)

Die Diazoniumverbindung aus 2-Naphthylamin-4,6,8-trisulfonsäure kann in diesem Beispiel durch die Diazoniumverbindungen aus den Aminen der folgenden Tabelle ersetzt werden. Die Ausführung der oxydativen Kupferung erfolgt analog.

| Diazoniumverbindung | Farbton | CI | GN |
|---|---|---|---|
| Anilin-4-sulfonsäure | rotst. Blau | 13 | D |
| Anilin-3,5-disulfonsäure | " | 13 | D |
| Anilin | " | 13 | D |
| 2-Aminonaphthalin-4,8-disulfonsäure | Blau | 14 | D |
| 2-Aminonaphthalin-6,8-disulfonsäure | Blau | 14 | D |
| 2-Aminonaphthalin-1,5,7-trisulfonsäure | Blau | 14 | D |

Verwendet man anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-

chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Baumwolle in violetten bis blauen Tönen färben.

Beispiel 270

In die nach dem in Beispiel 69 beschriebenen Verfahren zur Herstellung des Kondensationsproduktes aus 2-Amino-5-hydroxynaphthalin-7-sulfonsäure und 5-Chlor-6-dichlormethyl-2,4-difluorpyrimidin erhaltene Lösung wird eine Suspension der Diazoniumverbindung aus 0,1 Mol 2-Aminonaphthalin-4,6,8-trisulfonsäure in 250 Volumenteilen Wasser gegeben und die Kupplung durch Zugabe von 20 Teilen Natriumbicarbonat zu Ende geführt. Die so erhaltene Kupplungslösung wird mit 250 Teilen einer 20 %igen Natriumacetatlösung und 500 Teilen einer 3 %igen Wasserstoffperoxidlösung versetzt. Die oxidative Kupferung wird dann durch Zutropfen von 250 Teilen einer 20 %igen Kupfersulfatlösung erreicht. Nach beendeter Kupferung wird der Farbstoff der Formel

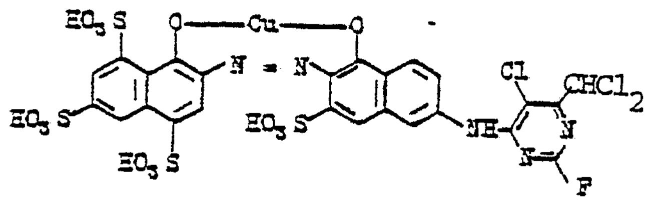

mit einem Gemisch aus 400 Teilen Natriumchlorid und 100

Teilen Kaliumchlorid ausgesalzen anschließend isoliert und getrocknet. Man erhält ein dunkles Pulver, das sich in Wasser mit violetter Farbe löst und Baumwolle in klaren violetten Farbtönen färbt. (CI = 36; GN = B)

Ersetzt man in diesem Beispiel die Diazoniumverbindung aus 2-Aminonaphthalin-4,6,8-trisulfonsäure durch Diazoniumverbindungen aus folgenden aromatischen Aminen, so erhält man ebenfalls violette Reaktivfarbstoffe:

| | CI | GN |
|---|---|---|
| Anilin-4-sulfonsäure | 35 | B |
| Anilin-3-sulfonsäure | 35 | B |
| Anilin-3,5-disulfonsäure | 35 | B |
| 2-Aminonaphthalin-4,8-disulfonsäure | 36 | B |
| 2-Aminonaphthalin-6,8-disulfonsäure | 37 | B |
| 1-Aminonaphthalin-4,6-disulfonsäure | 36 | B |
| 1-Aminonaphthalin-4,7-disulfonsäure | 36 | B |
| 2-Aminonaphthalin-3,6,8-trisulfonsäure | 36 | B |

Verwendet man anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Baumwolle in violetten Tönen färben.

Beispiel 271

0,1 Mol der Aminoazoverbindung der Formel

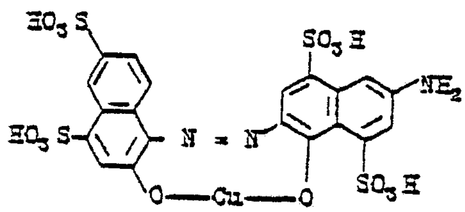

die durch Kupplung von 1-Aminonaphthalin-4,6-disulfonsäure mit 2-Acetylamino-5-hydroxynaphthalin-4,8-disulfonsäure anschließender oxydativer Kupferung und Verseifung der Acetylaminogruppe erhalten werden kann, werden in 1500 Volumenteilen Wasser bei pH 6,5 gelöst und bei ca. 40° mit 25 Teilen 5-Chlor-6-dichlormethyl-2,4-difluorpyrimidin portionsweise versetzt, wobei die freiwerdende Säure so mit einer Sodalösung neutralisiert wird, daß während der Kondensationszeit von etwa 2 Stunden ein pH von 6,5 gehalten wird.

Nach beendeter Umsetzung wird der Farbstoff der Formel

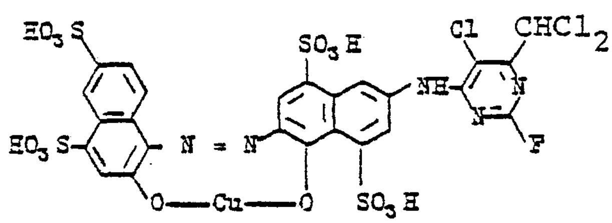

ausgesalzen, isoliert und bei ca. 50° im Vakuum getrocknet.

Man erhält ein dunkles Pulver, das sich in Wasser mit blauer Farbe löst und Baumwolle in rotstichig blauen Farbtönen färbt. Der gleiche Farbstoff kann durch Kupplung der Diazoniumverbindung aus 1-Aminonaphthalin-4,6-disulfonsäure mit dem Kondensationsprodukt aus 2-Amino-5-hydroxynaphthalin-4,8-disulfonsäure und 6-Dichlormethyl-2,4-difluor-5-chlor-pyrimidin und anschließender oxydativer Kupferung erhalten werden. (CI = 38)

Farbstoffe, die Baumwolle in ähnlichen blauen Farbtönen färben, können erhalten werden, wenn man in diesem Beispiel die Diazoniumverbindung aus 1-Aminonaphthalin-4,6-disulfonsäure durch die der folgenden Amine ersetzt:

| | CI | GN |
|---|---|---|
| 1-Aminonaphthalin-4,7-disulfonsäure | 38 | - |
| 1-Aminonaphthalin-4-sulfonsäure | 38 | - |
| 2-Aminonaphthalin-6,8-disulfonsäure | 38 | - |
| 2-Aminonaphthalin-4,6,8-trisulfonsäure | 38 | - |
| 2-Aminonaphthalin-3,6-disulfonsäure | 38 | - |
| 1-Amino-8-hydroxynaphthalin-4,6-disulfonsäure bzw. -3,6-disulfonsäure. | 39 | B |

Verwendet man anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in blauen Tönen färben.

Beispiel 272

0,1 Mol des 1:2-Chromkomplexes der Formel

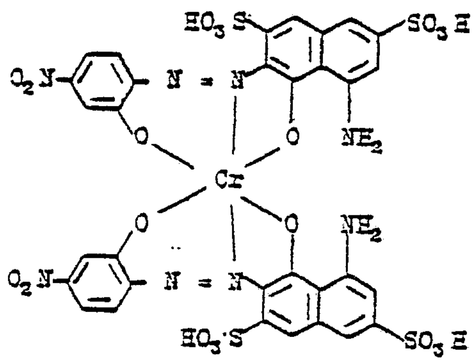

der durch Kupplung von diazoliertem 5-Nitro-2-aminophenol mit 1-Amino-8-hydroxynaphthalin-3,6-disulfonsäure und anschließender Umwandlung in den 1:2-Cr-Komplex erhalten werden kann, werden in 1000 Teilen Wasser bei pH 7 gelöst und mit 52 Teilen 5-Chlor-6-dichlormethyl-2,4-difluorpyrimidin bei 40° kondensiert. Die Neutralisation der freiwerdenden Säure erfolgt dabei durch Zutropfen einer verdünnten Sodalösung. Nach beendeter Kondensation wird der Farbstoff ausgesalzen, isoliert und getrocknet. Er fällt in Form eines dunklen Pulvers an, das Baumwolle in grünstichig grauen Farbtönen färbt. (CI = 46)

Ersetzt man in diesem Beispiel den reinen Chromkomplex durch eine 1:1-Mischung aus dem Cr-Komplex und dem entsprechenden Co-Komplex, so erhält man eine Farbstoffmischung, die Baumwolle in neutralen, lichtechten Schwarz- bzw. Grautönen färbt. (CI = 47)

Verwendet man anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in grünstichig bis neutralen lichtechten Schwarz- bzw. Grautönen färbt.

Beispiel 273

0,1 Mol des Co-Komplexes der Formel

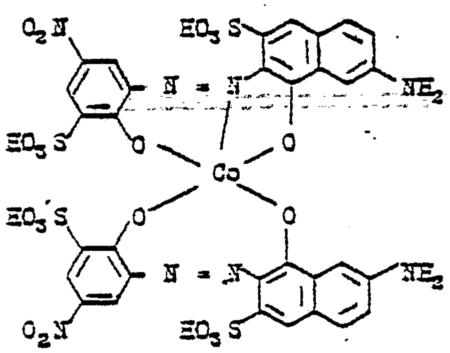

werden in 800 Volumenteilen Wasser bei pH 6 gelöst und mit 50 Teilen 5-Chlor-6-dichlormethyl-2,4-difluorpyrimidin unter kräftigem Rühren kondensiert. Die freiwerdende Säure wird durch Zutropfen einer 20 %igen Sodalösung neutralisiert. Nach beendeter Kondensation wird der erhaltene Reaktivfarbstoff ausgesalzen, isoliert und getrocknet. Er fällt in Form eines dunklen Pulvers an, das sich in Wasser mit brauner Farbe löst und Baumwolle in lichtechten Brauntönen färbt. (CI = 24)

Verwendet man anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in lichtechten Brauntönen färben.

Beispiel 274

0,1 Mol des durch alkalische Kupplung von diazotierter 4-Chlor-2-amino-1-hydroxybenzol-6-sulfonsäure mit 1-Amino-8-hydroxynaphthalin-3,6-disulfonsäure erhaltenen Farbstoffe werden in 300 Teilen Wasser bei 70-80° und einem pH-Wert von 8-9 mit 0,1 Mol des 1:1-Chromkomplexes des Farbstoffs aus 6-Nitro-1-diazo-2-hydroxynaphthalin-4-sulfonsäure und 2-Hydroxynaphthalin versetzt. Nach kurzer Zeit hat sich eine dunkle blaue Lösung gebildet. Der so erhaltene 1:2-Cr-Mischkomplex wird in ca. 2 Stunden bei 40-50° und einem pH von 6,5-7,5 mit 30 Teilen 5-Chlor-6-dichlormethyl-2,4-difluorpyrimidin acyliert. Hierbei wird der pH durch Zutropfen einer 20 %igen Sodalösung konstant gehalten. Der Reaktivfarbstoff wird mit 20 % Natriumchlorid ausgesalzen, isoliert und getrocknet. Der Farbstoff der Formel

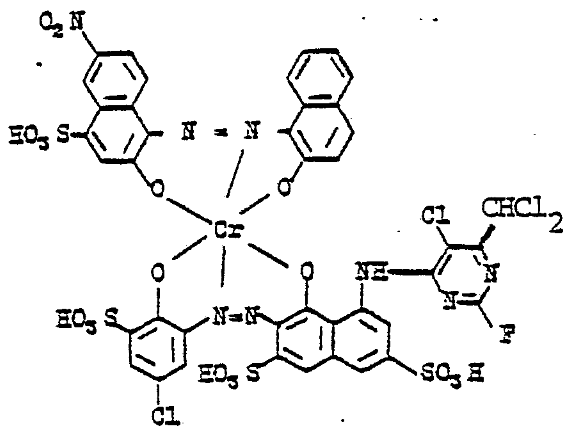

färbt Baumwolle in lichtechten blaugrauen Farbtönen.

Verwendet man anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Baumwolle in lichtechten blaugrauen Tönen färben. (CI = 45)

Beispiel 275

0,1 Mol des Kondensationsproduktes aus 1,3-Diaminobenzol-6-sulfonsäure und 5-Chlor-6-dichlormethyl-2,4-difluorpyrimidin wurde wie in Beispiel 1 beschrieben diazotiert und die Suspension bei pH 2-3 mit 0,1 Mol 1-Amino-8-hydroxynaphthalin-3,6-disulfonsäure gekuppelt. Nach beendeter Kupplung wird die dunkelrote Farbstofflösung mit der Suspension der Diazotierung aus Anilin-2,5-disulfonsäure versetzt. Zur zweiten Kupplung wird der pH-Wert

des Reaktionsgemisches durch Zutropfen einer 20 %igen Sodalösung auf 7-8 gestellt. Nach beendeter Kupplung wird der Farbstoff der Formel

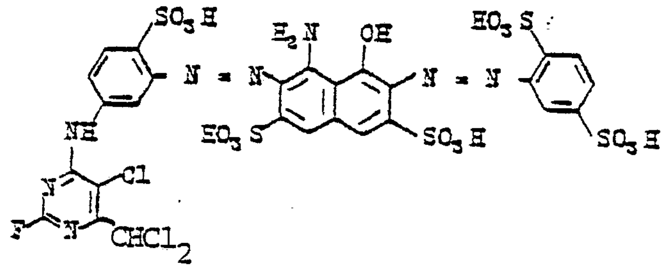

ausgesalzen, isoliert und getrocknet. Er stellt dann ein dunkles Pulver dar, das sich in Wasser mit blauer Farbe löst und Baumwolle in dunkelblauen bis schwarzen Farbtönen färbt. (CI = 39; GN = D)

Verwendet man anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in dunkelblauen bis schwarzen Tönen färben.

Verwendet man in den oben beschriebenen Farbstoffen anstelle von 1-Amino-8-hydroxynaphthalin-3,6-disulfonsäure 1-Amino-8-hydroxynaphthalin-4,6-disulfonsäure, so erhält man Farbstoffe, die Wolle und Baumwolle in schwarzen Tönen färben. (CI = 39; GN = D)

Beispiel 276

Die in schwach saurem pH-Bereich ausgeführte Kupplung von 0,1 Mol der Diazoniumverbindung aus Anilin-2,5-disulfonsäure mit 0,1 Mol 1-Amino-8-hydroxynaphthalin-3,6-disulfonsäure führt zu einer dunkelroten Lösung des Monoazofarbstoffs, der bei pH 6 - 7 mit 0,1 Mol der Diazoniumverbindung aus dem Kondensationsprodukt von 1,3-Diaminobenzol-6-sulfonsäure und 5-Chlor-6-dichlormethyl-2,4-difluorpyrimidin zu einem dunkelblauen Farbstoff gekuppelt werden kann. Nach Aussalzen, Isolieren und Trocknen fällt der Farbstoff der Formel

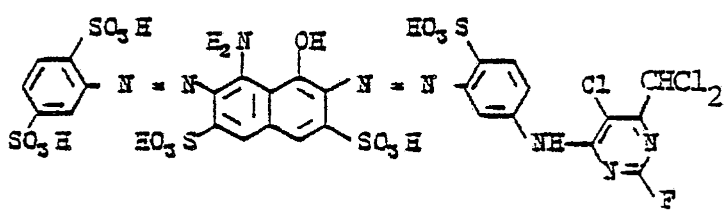

als dunkles Pulver an, das sich in Wasser mit blauer Farbe löst und Baumwolle in dunkelblauen bis schwarzen Farbtönen färbt. (CI = 39; GN = D)

Verwendet man anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe, die Wolle und Baumwolle in dunkelblauen bis schwarzen Tönen färben.

Verwendet man in den oben beschriebenen Farbstoffen anstelle von 1-Amino-8-hydroxynaphthalin-3,6-disulfonsäure 1-Amino-8-hydroxynaphthalin-4,6-disulfonsäure, so erhält man Farbstoffe, die Wolle und Baumwolle in schwarzen Tönen färben. (CI = 39; GN = D)

Beispiel 277

0,1 Mol der Aminoazoverbindung der Formel

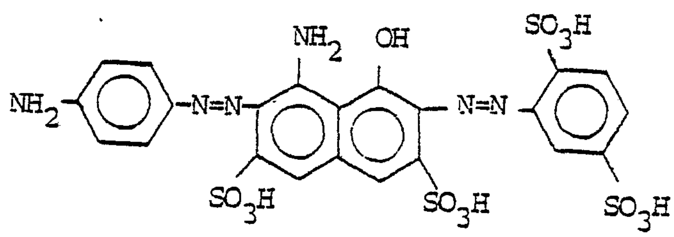

die durch in schwach saurem pH-Bereich ausgeführte Kupplung des Diazoniumsalzes von 0,1 Mol 4-Nitranilin und anschließender in neutralem pH-Bereich ausgeführter Kupplung des Diazoniumsalzes von 0,1 Mol Anilin-2,5-disulfonsäure mit 0,1 Mol 1-Amino-8-hydroxynaphthalin-3,6-disulfonsäure und anschließender Reduktion der Nitrogruppe mit Natriumsulfid auf an sich bekanntem Weg erhalten werden kann, werden in 1500 Volumenteilen Wasser bei pH 6,5 gelöst und bei ca. 40° mit 25 Teilen 5-Chlor-6-dichlormethyl-2,4-difluorpyrimidin portionsweise versetzt, wobei die freiwerdende Säure so mit einer Sodalösung neutralisiert wird, daß während der Kondensationszeit von etwa 2 Stunden ein pH von 6,5 gehalten wird.

Nach beendeter Umsetzung wird der Farbstoff der Formel

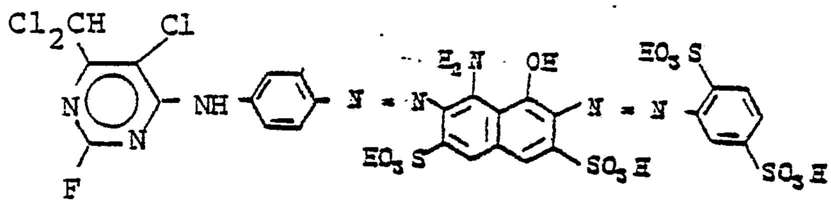

ausgesalzen, isoliert und getrocknet. Er stellt dann ein dunkles Pulver dar, das sich in Wasser mit grüner Farbe löst und Baumwolle in grünstichig schwarzen Farbtönen färbt. (CI = 41; GN = D)

Verwendet man in den oben beschriebenen Farbstoffen anstelle von 1-Amino-8-hydroxynaphthalin-3,6-disulfonsäure 1-Amino-8-hydroxynaphthalin-4,6-disulfonsäure, so erhält man Farbstoffe, die Wolle und Baumwolle in schwarzen Tönen färben. (CI = 41; GN = D)

Beispiel 278

0,1 Mol 1-Amino-4(3'-amino-2'-methylanilino)-anthrachinon-2,5'-disulfonsäure wird in 500 ml Wasser mit Natriumhydroxid neutral gelöst. Bei 20-30° tropft man 24 Teile 5-Chlor-6-dichlormethyl-2,4-difluorpyrimidin zu und hält den pH-Wert durch Zugabe von 10 %iger Sodalösung auf 6-7. Nach beendeter Acylierung wird mit Natriumchlorid ausgesalzen, isoliert und getrocknet. Der Farbstoff der Formel

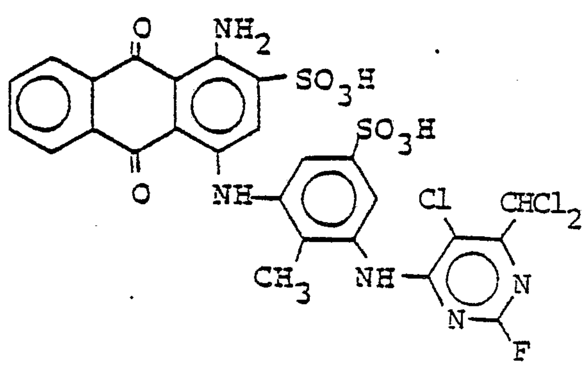

färbt Wolle und Baumwolle in klaren blauen Tönen mit guten Naß- und Lichtechtheiten. (CI = 14; GN = B)

Farbstoffe, die Baumwolle und Wolle in blauen Farbtönen färben, können erhalten werden, wenn man nach obigem Beispiel die in der folgenden Tabelle aufgeführten Anthrachinonderivate mit 5-Chlor-6-dichlormethyl-2,4-difluorpyrimidin kondensiert.

| Beispiel | Aminoanthrachinon-Derivat | Farbton | CI | GN |
|---|---|---|---|---|
| 279 | 1-Amino-4-(3'-amino-2',6'-dimethyl-anilino)-anthrachinon-2,5'-disulfonsäure | rotst. Blau | 13 | B |
| 280 | 1-Amino-4-(3'-amino-2',4',6'-trimethyl-anilino)-anthrachinon-2,5'-disulfonsäure | rotst. Blau | 13 | B |
| 281 | 1-Amino-4-(3'-amino-anilino)-anthrachinon-2,4-disulfonsäure | Blau | 14 | B |
| 282 | 1-Amino-4-(4'-amino-2'-carboxy-anilino)-anthrachinon-2-sulfonsäure | grünst. Blau | 15 | B |
| 283 | 1-Amino-4-(4'-aminoanilino)anthrachinon-2,3'-disulfonsäure | " | 15 | B |
| 284 | 1-Amino-4-(4'-aminoanilino)-anthrachinon-2,2'-disulfonsäure | " | 15 | B |
| 285 | 1-Amino-4-(3'-aminoanilino)-anthrachinon-2,4',6'-trisulfonsäure | Blau | 14 | B |

Verwendet man in den Beispielen 278-285 anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in rotstichig bis grünstichig blauen Tönen färben.

Beispiel 286

0,1 Mol 1-Amino-4-(4'-N-methylamino-methylanilino)-anthrachinon-2,2'-disulfonsäure werden in 550 Teilen Wasser gelöst und mit Natronlauge auf pH 7 gestellt. Dann tropft man bei 10-20° 25 Teile 6-Dichlormethyl-2,4-difluor-5-chlor-pyrimidin zu und hält mit 2n Sodalösung einen pH-Wert von 7,5 aufrecht. Das nach beendeter Kondensation in blauen Nadeln ausfallende Kondensationsprodukt wird abgesaugt und getrocknet. Der erhaltene Farbstoff der Formel

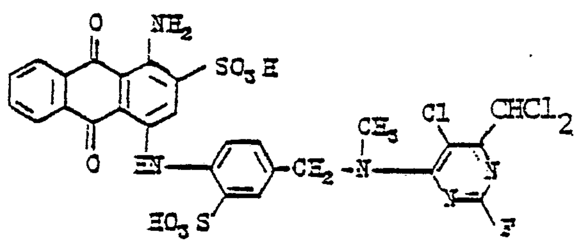

färbt Baumwolle in klaren blauen Farbtönen.
(CI = 14; GN = B)

Farbstoffe, die Baumwolle und Wolle in blauen Farbtönen färben, können erhalten werden, wenn man nach obigem Beispiel die in der folgenden Tabelle aufgeführten Anthrachinonderivate mit 5-Chlor-6-dichlormethyl-2,4-difluorpyrimidin kondensiert.

| Beispiel | Aminoanthrachinon-Derivat | Farbton | CI | GN |
|---|---|---|---|---|
| 287 | 1-Amino-4-(2'-aminomethyl-4'-methyl-anilino)-anthrachinon-2,6'-disulfonsäure | rotst. Blau | 13 | B |
| 288 | 1-Amino-4-(2',6'-dimethyl-3-N-methyl-amino-methylanilino)-anthrachinon-2,4'-disulfonsäure | " | 13 | B |
| 289 | 1-Amino-4-(2',6'-dimethyl-3-N-methyl-aminomethylanilino)-anthrachinon-2,5'-disulfonsäure | " | 13 | B |
| 290 | 1-Amino-4-(4'-N-methylaminomethyl-anilino)-anthrachinon-2,3'-disulfonsäure | Blau | 14 | B |
| 291 | 1-Amino-4-(4'-methoxy-3'-methylamino-methylanilino)-anthrachinon-2,5'-disulfonsäure | grünst. Blau | 15 | B |
| 292 | 1-Amino-4-(4'-methoxy-5'-methylamino-methylanilino)-anthrachinon-2,2'-disulfonsäure | " | 15 | B |

Verwendet man in den Beispielen 286-292 anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in rotstichig bis grünstichig blauen Tönen färben.

Beispiel 293

0,1 Mol 1-Amino-4-(3'-amino-anilino)-anthrachinon-2-sulfonsäure werden in einer Mischung aus 800 Teilen

Wasser und 400 Teilen Dioxan unter Zusatz von 5 Teilen Natriumcarbonat gelöst und bei 30-40° mit 0,1 Mol 5-Chlor-6-dichlormethyl-2,4-difluorpyrimidin kondensiert. Dabei wird - unter kräftigem Rühren - der pH-Wert von 6-6,5 durch Zutropfen einer 2n Sodalösung aufrecht erhalten. Nach beendeter Kondensation wird der Farbstoff isoliert, getrocknet und anschließend in einem Schwefelsäure/Oleum-Gemisch nach üblichen Methoden nachsulfiert. Man erhält nach Aufarbeitung des Reaktionsgemisches einen Reaktivfarbstoff der wahrscheinlichen Formel

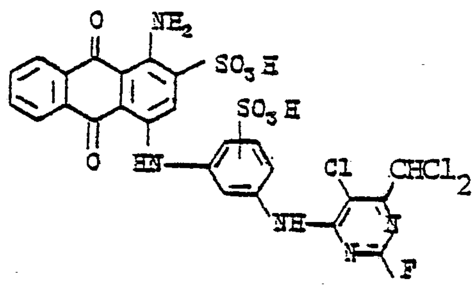

der Baumwolle in lichtechten, klaren blauen Farbtönen guter Naßechtheit färbt. (CI = 14; GN = B)

Verwendet man anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in klaren blauen Tönen färben.

Beispiel 294

0,1 Mol 1-Amino-4-(4'-carboxyethylamino)-phenylamino-

anthrachinon-2-sulfonsäure werden in 400 Teilen Wasser suspendiert und mit Natronlauge bei pH 6,5-7 gelöst. Dann tropft man bei 20-30°C 25 Teile 5-Chlor-6-dichlormethyl-2,4-difluorpyrimidin zu und hält mit verdünnter Sodalösung einen pH-Wert von 5,5-6,5 aufrecht. Nach beendeter Kondensation wird der Farbstoff mit Natriumchlorid ausgesalzen, filtriert und 10 %iger Kochsalzlösung gewaschen und getrocknet. Der erhaltene Farbstoff der Formel

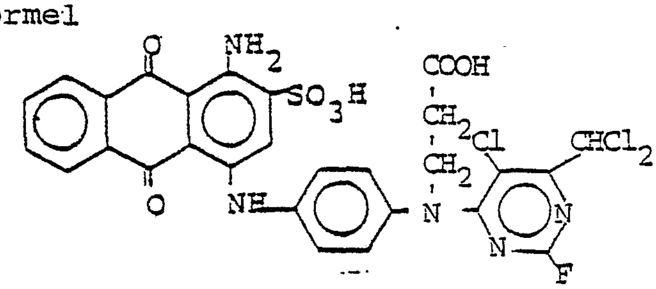

färbt Baumwolle und Wolle in lichtechten, klaren grünstichig blauen Farbtönen. (CI = 15; GN = B)

Farbstoffe, die Wolle und Baumwolle in klaren blauen Tönen färben erhält man, wenn man anstelle des obengenannten Aminoanthrachinons 1-Amino-4-(3'-carboxyethylamino)-phenylaminoanthrachinon-2-sulfonsäure oder 1-Amino-4-(3'-carboxyethylamino-4'-methyl-5'-sulfo)-phenylamino-anthrachinon-2-sulfonsäure verwendet aber sonst wie oben beschrieben verfährt. (CI = 14; GN = B)

Verwendet man anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie

oben beschrieben, so erhält man Farbstoffe die Wolle und Baumwolle in blauen bzw. grünstichig blauen Tönen färben.

Beispiel 295

0,1 Mol des in üblicher Weise durch Einwirkung von Chlorsulfonsäure und Thionylchlorid auf Kupferphthalocyanin frisch hergestellten Kupferphthalocyanin-tetrasulfochlorids werden in Form des feuchten, gut gewaschenen Saugkuchens in 1000 Teilen Wasser und 500 Teilen Eis suspendiert und bei pH 8-9 mit 0,1 Mol des Kondensationsproduktes aus 1,3-Diaminobenzol und 5-Chlor-6-dichlormethyl-2,4-difluorpyrimidins bei 15-20° zur Umsetzung gebracht. Durch Zutropfen von 20 %iger Sodalösung wird dabei ein pH-Wert von ca. 8,5 aufrechterhalten. Nach beendeter Kondensation wird der Reaktivfarbstoff der Formel

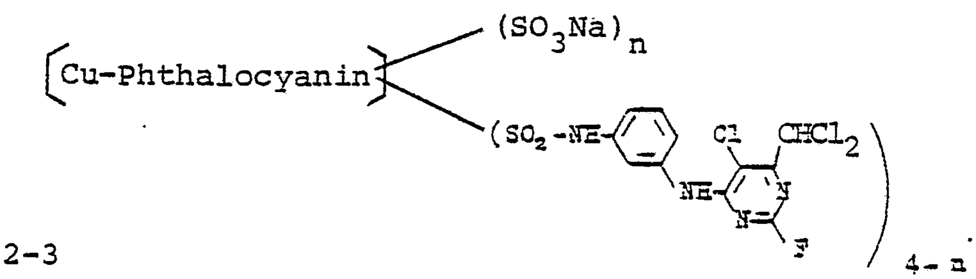

n = ~ 2-3

ausgesalzen, gewaschen und bei 50-100! im Vakuum getrocknet. Er stellt ein dunkelblaues, in Wasser mit blauer Farbe lösliches Pulver dar, das Baumwolle in klaren grünstichig blauen Farbtönen färbt. (CI = 15; GN = B)

Verwendet man anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in klaren grünstichig blauen Farbtönen färben.

Beispiel 296

0,01 Mol des Triphendioxazins der Formel

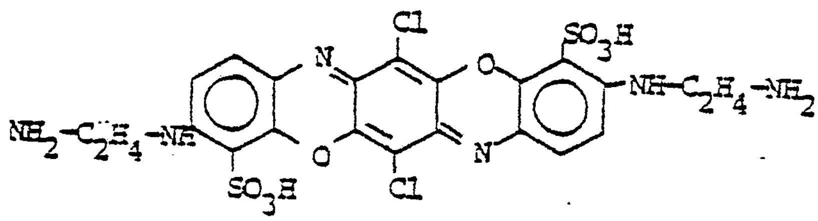

werden in 2 l Wasser bei Raumtemperatur gelöst und bei einem pH-Wert von 7-7,5 mit 5,5 g 5-Chlor-6-dichlormethyl-2,4-difluorpyrimidin umgesetzt, der pH-Wert wird mit Sodalösung gehalten. Es wird 1 Stunde nachgerührt, das ausgefallene Produkt abfiltriert und bei 60°C getrocknet. Man erhält ein dunkelblaues Pulver, das Baumwolle und Wolle in leuchtend blauen Tönen färbt. (CI = 14; GN = B)

Verwendet man anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in leuchtend blauen Tönen färben.

Nach gleichen Verfahren und mit den genannten Difluorpyrimidinen liefern auch die folgenden Verbindungen im ersten Falle leuchtend blaue (CI = 14; GN = B), im zweiten Fall leuchtend grünstichig (CI = 15; GN = B) blaue Färbungen auf Baumwolle und Wolle

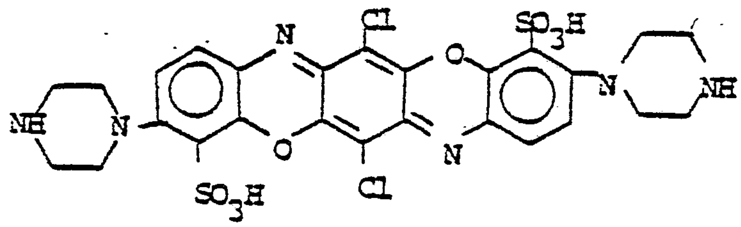

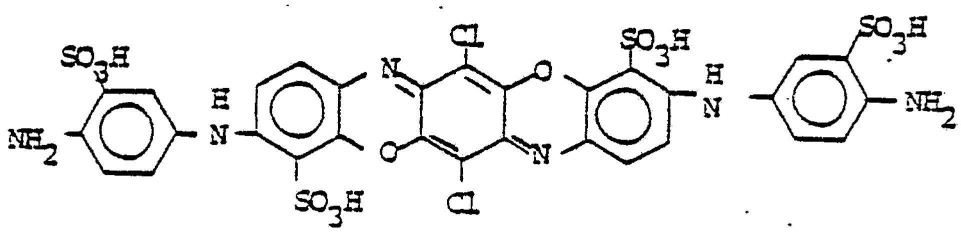

Beispiel 297

0,1 Mol des Kupferkomplexes von N-(2-Carboxy-5-sulfophenyl)-N-(2'-hydroxy-3'-amino-5'-sulfophenyl)-ms-phenylformazan-di-Natriumsalz werden in 500 ml Wasser gelöst und bei 20-30° mit 25 Teile 5-Chlor-6-dichlormethyl-2,4-difluorpyrimidin versetzt.

Durch Zugabe von Sodalösung wird ein pH-Wert von 6-6,5 aufrecht erhalten. Man salzt mit Natriumchlorid aus,

saugt den ausgefallenen Farbstoff ab und trocknet bei 30-40° im Vakuum.

Der erhaltene Farbstoff der Formel

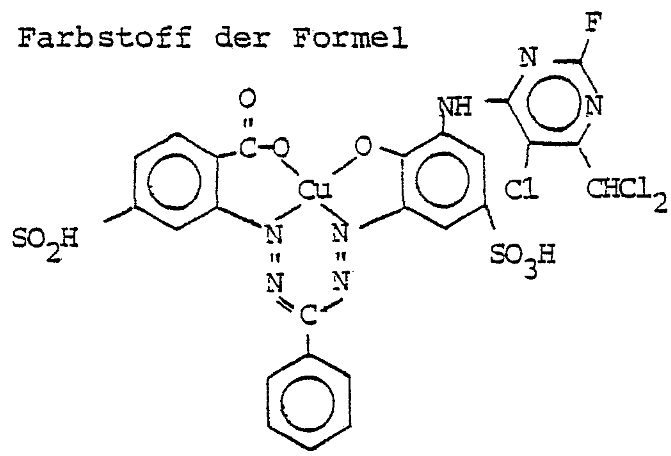

färbt Zellulose- und Polyamidfasern in echten blauen Tönen. (CI = 14; GN = D)

Verwendet man anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in echten blauen Tönen färben.

Verwendet man die in der folgenden Tabelle aufgeführten Kupferkomplexe und die oben angegebenen Difluorpyrimidine und verfährt wie oben angegeben so erhält man weitere Farbstoffe, die Zellulose- und Polyamidfasern in echten blauen Tönen färben.

| Aminoformazan | Nuance auf Baumwolle | CI | GN |
|---|---|---|---|
| N-(2-Hydroxy-3-amino-5-sulfophenyl)-N'-(2'-carboxy-4'-sulfophenyl)-ms-(2"-chlor-5"-sulfophenyl)-Formazan, Cu-Komplex, | blau | 14 | D |
| N-(2-Hydroxy-5-amino-3-sulfophenyl)-N'-(2',5'-disulfophenyl)-ms-phenylformazan, Cu-Komplex, | blau | 14 | D |
| N-(2-Carboxy-4-aminophenyl)-N'-(2'-hydroxy-5'-methylsulfonyl-3'-sulfophenyl)-ms-(2"-sulfophenyl)-formazan, Cu-Komplex, | blau | 14 | D |
| N-(2-Carboxy-5-sulfophenyl)-N'-(2'-hydroxy-3',5'-disulfophenyl)-ms-(3"-aminophenyl)-formazan, Cu-Komplex, | grünstichig blau | 15 | D |
| N-(4-Amino-2-sulfophenyl)-N'-(2'-hydroxy-4'-sulfophenyl)-ms-(4"-chlor-3"-sulfophenyl)-formazan, Cu-Komplex, | blau | 14 | D |
| N-(2-Hydroxy-3-amino-5-sulfophenyl)-N'-(2'-hydroxy-4'-sulfophenyl)-ms-(2"-sulfophenyl), Cu-Komplex, | marineblau | 28 | |
| N-(2-Carboxy-5-sulfophenyl)-N'-(2'-hydroxy-5'-amino-3'-sulfophenyl)-ms-(4"-sulfophenyl)-formazan, Cu-Komplex, | blau | 14 | D |
| N-(2-Carboxy-5-sulfophenyl)-N'-(2'-hydroxy-3'-amino-5'-sulfophenyl)-ms-phenylformazan, Cu-Komplex | blau | 14 | D |
| N-(2-Carboxy-4-sulfophenyl)-N'-(2'-hydroxy-3'-amino-5'-sulfophenyl)-ms-phenylformazan, Cu-Komplex, | blau | 14 | D |
| N-(2,4-Disulfophenyl)-N'-(2'-hydroxy-4,6-disulfophenyl)-ms-(3"-aminophenyl)-formazan, Cu-Komplex, | blau | 14 | D |

| Aminoformazan | Nuance auf Baumwolle | CI | GN |
|---|---|---|---|
| N-(2-Carboxy-5-sulfophenyl)-N'-(2'-hydroxy-3'-amino-5'-sulfophenyl)-ms-(4"-methoxyphenyl)-formazan, Cu-Komplex, | blau | 14 | D |
| N-(2-Carboxy-5-sulfophenyl)-N'-(2'-hydroxy-4'-methylsulfonyl-6'-sulfophenyl)-ms-(3"-aminophenyl)-formazan, Cu-Komplex, | blau | 14 | D |
| N-(2-Hydroxy-5-sulfophenyl)-N'-(2'-hydroxy-3',5'-disulfophenyl-ms-(4"-aminophenyl)-formazan, Cu-Komplex, | blau | 14 | D |
| N-(2-Carboxy-5-sulfophenyl)-N'-(2'-hydroxy-3'-amino-5'-sulfophenyl)-ms-(4"-methyl-3"-bromphenyl)-formazan, Cu-Komplex, | blau | 14 | D |
| N-(2-Carboxy-4-aminophenyl)-N'-(2'-hydroxy-4'-sulfonaphth-1'-yl)-ms-(2"-sulfophenyl)-formazan, Cu-Komplex, | blau | 14 | D |

Beispiel 298

0,1 Mol der bekannten Kupferkomplexverbindung der Formel

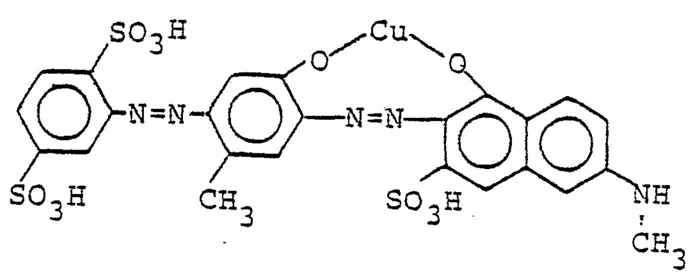

werden in 1500 Volumenteilen Wasser bei pH 6,5 gelöst und bei 30 - 40° mit 25 Teilen 5-Chlor-6-dichlormethyl-2,4-difluorpyrimidin portionsweise versetzt, wobei die freiwerdende Säure so mit Sodalösung neutralisiert wird, daß während der Kondensationszeit von ca. 2 Stunden ein pH-Wert von 6,5 gehalten wird.

Nach beendeter Umsetzung wird der Farbstoff der Formel

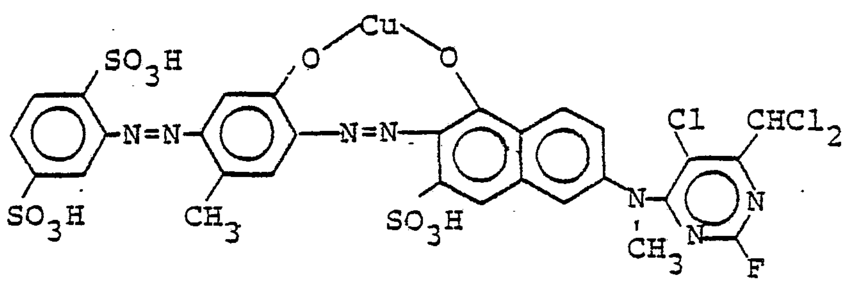

ausgesalzen, isoliert und bei 50° im Vakuum getrocknet.

Man erhält ein dunkles Pulver, das sich in Wasser mit blauer Farbe löst und Baumwolle in marineblauen Tönen färbt. (CI = 28)

Farbstoffe, die Baumwolle in ähnlichen marineblauen Farbtönen färben, können erhalten werden, wenn man in obigem Beispiel die Kupplungskomponente 6-N-Methylamino-1-hydroxynaphthalin-3-sulfonsäure durch die folgenden Aminohydroxynaphthalinsulfonsäuren ersetzt:

| | CI |
|---|---|
| 6-Amino-1-hydroxynaphthalin-3-sulfonsäure | 28 |
| 6-N-Methylamino-1-hydroxynaphthalin-3,5-disulfonsäure | 28 |

| | CI |
|---|---|
| 6-Amino-1-hydroxynaphthalin-3,5-disulfonsäure | 28 |
| 6-Amino-1-hydroxynaphthalin-4,8-disulfonsäure | 28 |
| 7-Amino-1-hydroxynaphthalin-3,6-disulfonsäure | 28 |
| 5-Amino-1-hydroxynaphthalin-3-sulfonsäure | 28 |

Weitere Farbstoffe, die Baumwolle in marineblauen Farbtönen färben, können erhalten werden, wenn man die folgenden bekannten Kupferkomplexverbindungen der Formeln

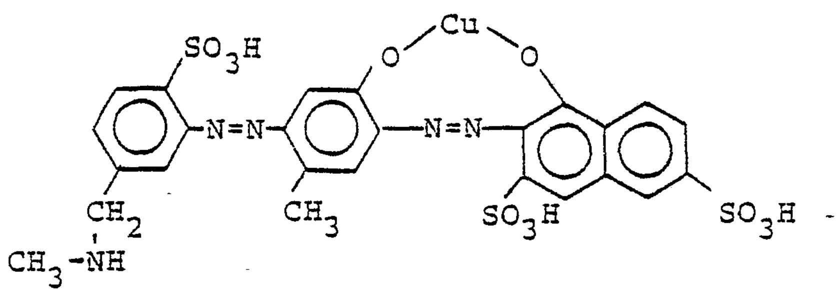

(CI = 28)

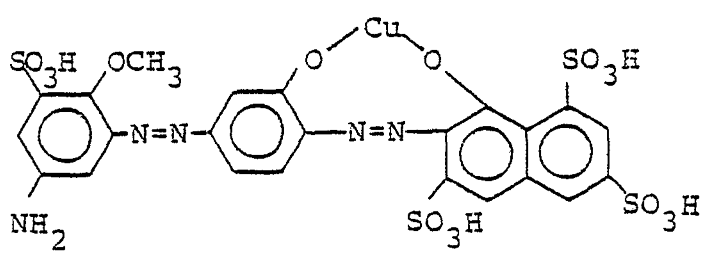

(CI = 28)

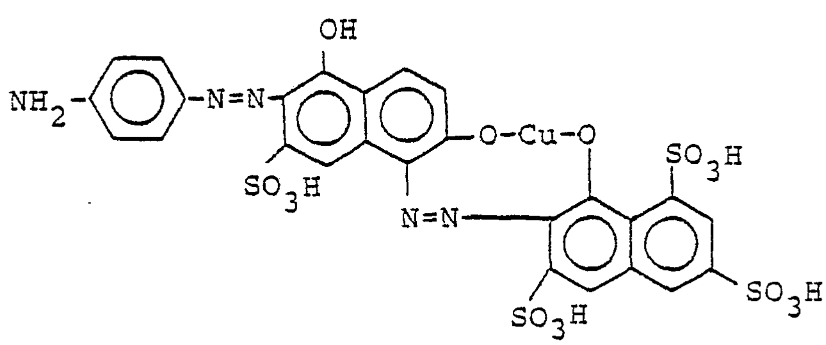

(CI = 28)

mit 5-Chlor-6-dichlormethyl-2,4-difluorpyrimidin umsetzt.

Verwendet man in den oben beschriebenen Farbstoffen anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe, die Baumwolle in marineblauen Farbtönen färben.

Beispiel 299

0,1 Mol der bekannten Aminodisazoverbindung der Formel

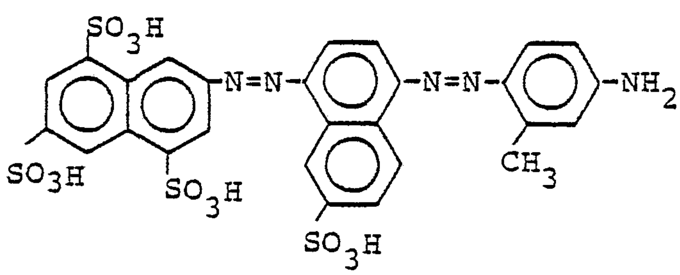

werden in 1500 Volumenteilen Wasser bei pH 6,5 gelöst und bei 30 - 40° mit 25 Teilen 5-Chlor-6-dichlormethyl-2,4-difluorpyrimidin portionsweise versetzt, wobei die freiwerdende Säure so mit Sodalösung neutralisiert wird, daß während der Kondensationszeit von ca. 2 Stunden ein pH-Wert von 6,5 gehalten wird.

Nach beendeter Umsetzung wird der Farbstoff der Formel

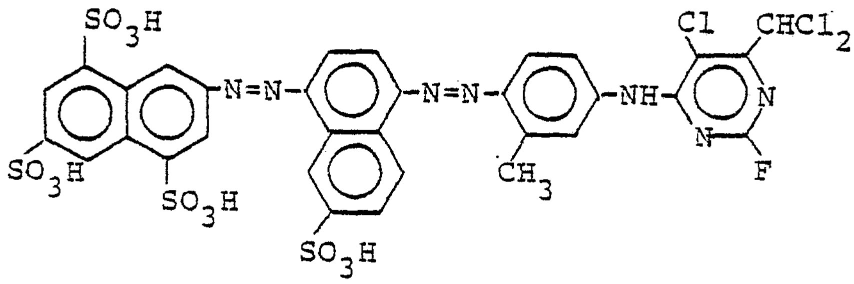

ausgesalzen, isoliert und bei 50° im Vakuum getrocknet.

Man erhält ein braunes Pulver, das sich in Wasser mit dunkelbrauner Farbe löst und Baumwolle in braunen Tönen färbt. (CI = 24)

Farbstoffe, die Baumwolle in orange bis braunen Farbtönen färben, können erhalten werden, wenn man die Aminodisazofarbstoffe der folgenden Tabelle mit 5-Chlor-6-dichlor-2,4-difluorpyrimidin umsetzt.

| Aminodisazofarbstoff | Farbton der Reaktivfärbung | CI | GN |
|---|---|---|---|
| 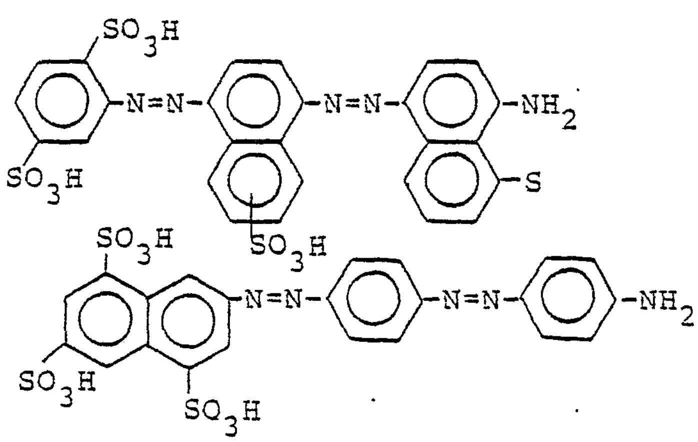 | Braun | 28 | - |
| | Orange | 5 | D |

| Aminodisazofarbstoff | Farbton der Reaktivfärbung | CI GN |
|---|---|---|
| 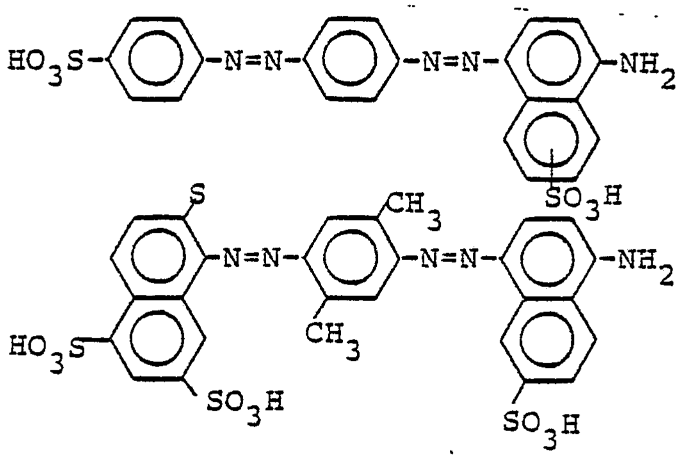 | Orange-Braun | 22 |
| | Braun | 24 |
| 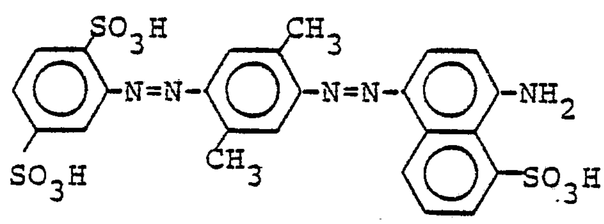 | Orange | 5 D |
| 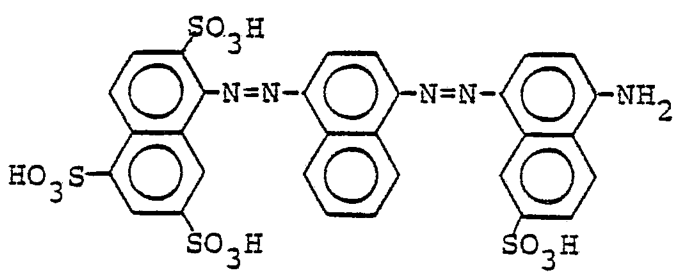 | Braun | 24 |
| 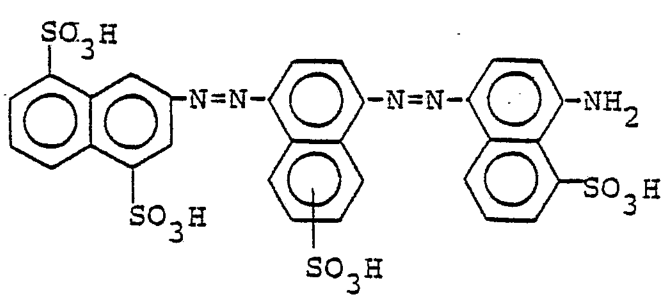 | Braun | 24 |

Verwendet man in den oben beschriebenen Farbstoffen anstelle von 5-Chlor-6-dichlormethyl-2,4-difluorpyrimidin die entsprechenden Mengen 5-Brom-6-dichlormethyl-, 5-Chlor-6-dichlorfluormethyl-, 5-Chlor-6-chlormethyl-, 5-Chlor-6-brommethyl-, 5-Brom-6-brommethyl-, 5-Brom-6-dichlorfluormethyl-, 6-Dichlorfluormethyl-, 5-Chlor-6-trichlormethyl- oder 5-Brom-6-trichlormethyl-2,4-difluorpyrimidin und verfährt sonst wie oben beschrieben, so erhält man Farbstoffe die Wolle und Baumwolle in orange bis braunen Tönen färben.

Beispiel 300

In einem 4 l-Vierhalskolben mit Rührer, Rückflußkühler, Thermometer und Gaseinleitungsrohr werden 1975 g (≙ 10 Mol) 6-Methyl-2,4,5-trichlorpyrimidin und 200 ml Phosphoroxychlorid vorgelegt. Man erwärmt die Mischung auf 90°C und leitet über eine Zeit von ca. 8 h und unter kräftigem Rühren 1200 g Chlorgas ein. Danach wird das Reaktionsgemisch unter teilweisem Abdestillieren des Phosphoroxychlorids auf 120°C erwärmt. Es werden über 4 h weitere 350 g Chlorgas in das Reaktionsgemisch eingeleitet. Danach ist die Reaktion beendet. Der Endpunkt kann gaschromatographisch ermittelt werden. Man destilliert das restliche Phosphoroxychlorid ab, und unterwirft den Rückstand einer Vakuumdestillation.

Ausbeute: 2040 g 6-Dichlormethyl-2,4,5-trichlorpyrimidin ($Kp_{0.2mbar}$ = 92°C).

Verwendet man anstelle von 6-Methyl-2,4,5-trischlorpyrimidin 2304 g 5-Brom-2,4-dichlor-6-methylpyrimidin und verfährt sonst wie oben beschrieben, so erhält man 2370 g 5-Brom-6-dichlormethyl-2,4-dichlorpyrimidin ($Kp_{0,01\ mbar}$ = 82°).

Beispiel 301

In einen 2 l-Vierhalskolben mit Rührer, Rückflußkühler, Thermometer und Gaseinleitungsrohr werden 987,5 g (≙ 5 Mol) 6-Methyl-2,4,5-trichlorpyrimidin vorgelegt. Man erwärmt auf ca. 90 - 100° und leitet über eine Zeit von ca. 8 h 800 g Chlorgas ein. Danach erhitzt man unter Einleiten von Chlorgas das Reaktionsgemisch innerhalb von 4 h auf 190 - 200°. Diese Temperatur wird 24 h lang gehalten, während man weitere 400 g Chlorgas einleitet. Der Fortgang der Reaktion wird gaschromatographisch verfolgt. Nach beendeter Umsetzung wird das Reaktionsgemisch im Vakuum destilliert. Man erhält zwei Fraktionen: Der Vorlauf, 40 g, ($Kp_{0.3mbar}$ bis 98°C) enthält überwiegend die Zwischenstufe 6-Dichlormethyl-2,4,5-trichlorpyrimidin. Als Hauptfraktion erhält man 1280 g 6-Trichlormethyl-2,4,5-trichlorpyrimidin ($Kp_{0.3mbar}$ = 98°C).

Beispiel 302

In einem 4 l-Vierhalskolben mit Rührer, Rückflußkühler, Thermometer und Gaseinleitungsrohr wird eine Suspension aus 504 g (≙ 4 Mol) 6-Methyl-2,4-dihydroxypy-

rimidin, 1,4 l Phosphoroxychlorid und 700 ml (≙ 1100 g ≙ 8 Mol) Phosphortrichlorid 1 h auf 80° erwärmt. Bei dieser Temperatur leitet man über 6 h 1000 g Chlorgas ein. Dabei löst sich die Suspension langsam auf. Man erwärmt jetzt langsam auf 110 - 115°C und leitet während 4 h weitere 450 g Chlorgas ein. Danach ist die Reaktion beendet. Der Endpunkt kann gaschromtographisch ermittelt werden. Zur Aufarbeitung des Reaktionsgemisches wird Phosphoroxychlorid abdestilliert. Der Rückstand wird einer Vakuumdestillation unterworfen. Man erhält 50 g Vorlauf ($Kp_{0.5mbar}$ bis 109°C), der überwiegend 6-Dichlormethyl-2,4-dichlorpyrimidin enthält und 917,5 g Hauptlauf ($Kp_{0.5mbar}$ 109°C) bestehend aus 2,4-Dichlor-6-trichlormethylpyrimidin.

Beispiel 303

In einem Stahlautoklaven werden 400 ml HF (wasserfrei) vorgelegt. Man läßt bei ca. 5°C 301 g 2,4,5-Trichlor-6-trichlormethylpyrimidin zutropfen. Dann wird die Apparatur verschlossen und ein Stickstoff-Schutzdruck von 3 bar aufgedrückt. Unter Rühren wird bis 60°C und dann langsam im Maß der Chlorwasserstoffentwicklung bis auf 100°C aufgeheizt. Der entstehende Chlorwasserstoff wird bei einem Druck entspannt, der 3 bis 5 bar über dem jeweiligen Siedepunkt der HF liegt. Bei der Reaktionstemperatur von 100°C entspricht dies z.B. einem Entspannungsdruck von 12 bis 14 bar. Nach dem Ausbleiben der HCl-Entwicklung wird auf ca. 8 bis 9 bar

entspannt und noch 30 Minuten nachgerührt. Dann wird abgekühlt, der Reaktor ganz entspannt und die überschüssige HF abdestilliert. Der Rückstand wird in Eiswasser gegossen, der organische Anteil wird mit Methylenchlorid extrahiert, getrocknet und destilliert. Man erhält 128 g 2,4-Difluor-5-chlor-6-trichlorpyrimidin (Kp 105°C/15 mbar in $n_D^{20}$: 1,5234.

Beispiel 304

In einem Rührgefäß aus Glas mit Rührer, Thermometer und Rückflußkühler werden 600 g 2,4,5-Trichlor-6-trichlormethylpyrimidin in 720 ml Tetramethylensulfon vorgelegt und nach Zugabe von 225 g Natriumfluorid unter Rühren aufgeheizt. Man läßt 4 Stunden bei 200°C reagieren. Die Aufarbeitung erfolgt durch Destillation. Man erhält 291 g 2,4-Difluor-5-chlor-6-trichlormethyl-pyrimidin (Siedepunkt 102°C bei 12 mbar Druck $n_D^{20}$: 1,5225.
Laut Gaschromatogramm beträgt die Reinheit 99,1 %.

Beispiel 305

In einer Apparatur gemäß Beispiel 303 werden 1170 g 2,4,5-Trichlor-6-dichlormethylpyrimidin in 2,6 l Fluorwasserstoffsäure vorgelegt und in einer Stunde bis auf 120°C aufgeheizt. Der Entspannungsdruck für den entstehenden Chlorwasserstoff beträgt 16 bar. Nach Ende der kräftigen HCl-Entwicklung wird auf 140°C geheizt. Der zunächst bei 25 bar gehaltene Innendruck wird im Maß der nachlassenden HCl-Entwicklung auf 18 bar reduziert. Dann kühlt man den Ansatz ab und arbeitet durch Destillation auf. Man erhält 832 g 2,4-Difluor-5-chlor-6-dichlormethylpyrimidin vom Siedepunkt 96°C bei 20 mbar $n_D^{20}$: 1,5142.
Laut Gaschromatogramm liegt eine Reinheit von 96,9 % vor.

Beispiel 305a

In einen 4 l-Vierhalskolben mit Rührer, Rückflußkühler, Thermometer und Gaseinleitungsrohr werden 1975 g (≙ 10 Mol) 6-Methyl-2,4,5-trichlorpyrimidin und 200 ml Phosphoroxychlorid vorgelegt. Man erwärmt die Mischung auf 90°C und leitet über eine Zeit von ca. 8 h und unter kräftigem Rühren 1200 g Chlorgas ein. Danach wird das Reaktionsgemisch unter teilweisem Abdestillieren des Phosphoroxychlorids auf 120° erwärmt. Es werden über 4 h weitere 350 g Chlorgas in das Reaktionsgemisch eingeleitet. Danach ist die Reaktion beendet. Der Endpunkt kann gaschromatographisch ermittelt werden. Man destilliert das restliche Phosphoroxychlorid ab und unterwirft den Rückstand einer Vakuumdestillation.

Ausbeute: 2040 g 6-Dichlormethyl-2,4,5-trichlorpyrimidin ($Kp_{0.2mbar}$ = 92°C).

Verwendet man anstelle von 6-Methyl-2,4,5-trichlorpyrimidin 2304 g 5-Brom-2,4-dichlor-6-methylpyrimidin

und verfährt sonst wie oben beschrieben, so erhält man 2370 g 5-Brom-6-dichlormethyl-2,4-dichlorpyrimidin ($Kp_{0.01\ mbar}$ = 82°).

Beispiel 306

In einer Apparatur gemäß Beispiel 304 werden 360 g 2,4,5-Trichlor-6-dichlormethylpyrimidin mit 270 g Natriumfluorid in 290 ml Tetramethylensulfon 2 Stunden bei 170°C fluoriert. Nach Destillation und Restdestillation erhält man 44 g reines 2,4-Difluor-5-chlor-6-dichlormethylpyrimidin vom Siedepunkt 94 bis 95°C bei einem Druck von 18 mbar $n_D^{20}$: 1,5145.

Beispiel 307

266,5 g 2,4-Dichlor-6-trichlormethylpyrimidin werden nach dem Verfahren des Beispiels 304 mit 126 g Natriumfluorid in 360 ml Tetramethylensulfon 3 Stunden bei 180°C fluoriert. Man erhält 103 g 2,4-Difluor-6-trichlormethylpyrimidin (Siedepunkt: 88°C bei 14 mbar, Schmelzpunkt 36 bis 37°C).

Beispiel 308

450 g 2,4-Dichlor-5-brom-6-dichlormethylpyrimidin werden in einer Apparatur gemäß Beispiel 303 mit 870 ml Fluorwasserstoffsäure (wasserfrei) 2 Stunden lang bei 120°C und anschließend 2 Stunden bei 140°C fluoriert.

Man erhält durch fraktionierte Destillation 194 g 2,4-Difluor-5-brom-6-dichlormethylpyrimidin vom Siedepunkt 101 bis 103°C bei 16 mbar und $n_D^{20}$: 1,5402. Die Substanz besitzt laut Gaschromatogramm einen Reinheitsgrad von 97 %.

Beispiel 309

In einer Apparatur gemäß Beispiel 303 werden 355 g eines Rohgemisches, welches 35 % der Verbindung 2,4,5-Trichlor-6-dibrommethylpyrimidin enthält mit 400 ml HF zunächst bei 120°C und 15 bar und anschließend bei 140°C und 25 bar fluoriert. Gegen Ende der Chlorwasserstoffentwicklung wird bei 140°C langsam bis auf 18 bar entspannt. Dann läßt man abkühlen, entspannt völlig und arbeitet durch Destillation und Redestillation auf. Man erhält 57 g 2,4-Difluor-5-chlor-6-dibrommethylpyrimidin vom Siedepunkt 115°C bei 10 mbar und $n_D^{20}$: 1,5631. Die Substanz hat laut Gaschromatogramm einen Reinheitswert von 95 %.

Beispiel 310

In einer Apparatur gemäß Beispiel 303 werden nach dem gleichen Verfahren wie dort beschrieben mit überschüssiger HF 245 g 2,4,5-Trichlor-6-chlormethylpyrimidin bei einer Temperatur von 80 bis 120°C fluoriert. Durch Destillation erhält man 133 g 2,4-Difluor-5-chlor-6-chlormethylpyrimidin als farblose Flüssigkeit vom Siedepunkt 86 bis 87°C bei 18 mbar und $n_D^{20}$: 1,5025. Reinheitsgrad laut Gaschromatogramm: 96,6 %.

Beispiel 311

Ändert man bei Einsatz der gleichen Komponenten die Bedingungen des Beispiels 303 in der Weise ab, daß man den Ansatz zunächst auf 120°C hochheizt und den Entspannungsdruck auf 15 Bar einstellt, eine Stunde bei 120°C reagieren läßt und anschließend noch zwei Stunden bei 140°C/25 Bar nachrührt und dann bei der gleichen Temperatur langsam bis auf 18 Bar entspannt, dann erhält man nach der Aufarbeitung in einer Ausbeute von 85 % der Theorie 2,4-Difluor-5-chlor-6-dichlorfluormethyl-pyrimidin als Flüssigkeit vom Siedepunkt 78°C/17 mbar, $n_D^{20}$ : 1,4875.

Beispiel 312

In Analogie zu den Bedingungen aus Beispiel 311 erhält man beim Einsatz von 2,4-Dichlor-6-trichlormethyl-pyrimidin als Ausgangskomponente die Verbindung 2,4-Difluor-6-dichlorfluormethylpyrimidin als Endprodukt. Letztgenannte Verbindung hat einen Siedepunkt von 63°C bei 18 mbar und einem Brechungsindex $n_D^{20}$: von 1,4622.

Patentansprüche

1. Reaktivfarbstoffe der Formel

$$D-\left[W-\underset{R}{\overset{}{N}}-A\right]_n \qquad (I)$$

worin

D für einen organischen Farbstoffrest steht,

W = direkte Bindung oder ein Brückenglied zu einem aromatischen, carbocyclischen oder aromatisch heterocyclischen Kohlenstoffatom des Restes D,

R = Wasserstoff oder $C_1$-$C_4$-Alkyl

A =

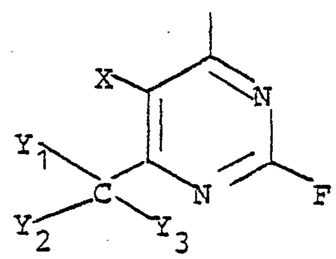

wobei X = Wasserstoff oder Substituent

$Y_1$ und $Y_2$ gleich oder verschieden sind und für Wasserstoff, Chlor oder Brom stehen

und $Y_3$ = Cl, Br oder F

und n = 1 oder 2.

2. Reaktivfarbstoffe gemäß Anspruch 1, dadurch gekennzeichnet, daß

X = Wasserstoff oder Halogen.

3. Reaktivfarbstoffe gemäß Anspruch 1, dadurch gekennzeichnet, daß A für einen der folgenden Reste steht:

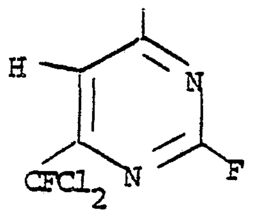 , 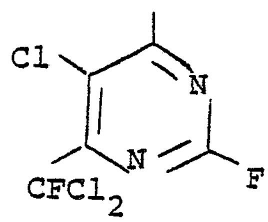 , 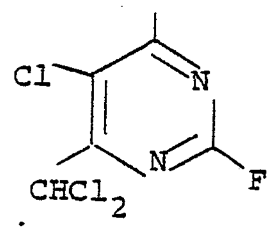

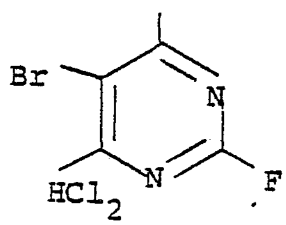 oder 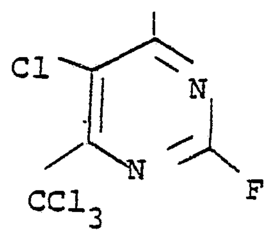

4. Reaktivfarbstoffe gemäß Anspruch 1, dadurch gekennzeichnet, daß A für den Rest

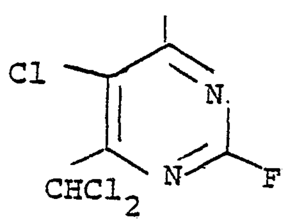

steht.

5. Farbstoffe gemäß Anspruch 1 bis 4 der Formeln:

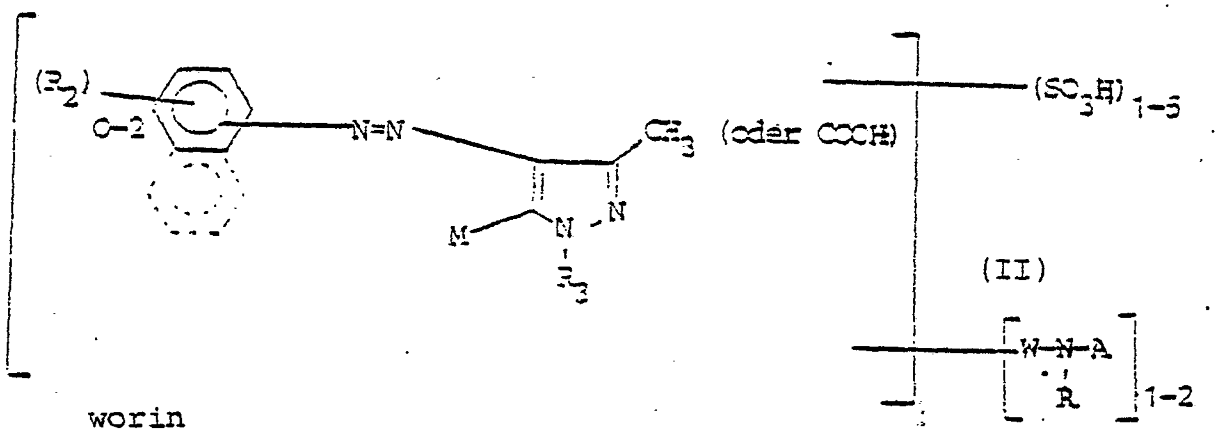

(II)

worin

M = OH, $NH_2$, $NR_3R_4$, $OR_3$,

$R_2$ = Alkyl, Alkoxy, Halogen, insbesondere Cl,

$R_3$ und $R_4$ = Wasserstoff, Alkyl, Aryl, Hetaryl, Aralkyl, oder wobei $R_3$ und $R_4$ zusammen für gegebenenfalls durch NH oder O unterbrochenes $C_4$-$C_6$-Alkylen stehen,

und worin R, W, A die in Anspruch 1 angegebene Bedeutung besitzen,

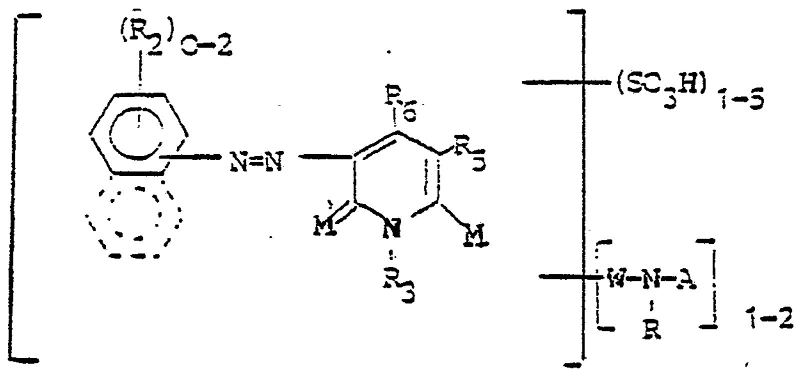

(III)

$R_5$= $-COOR_3$, $-CONR_3R_4$, -CN, $-CH_2-SO_3H$ oder $-CH_2-NR_3R_4$
$R_6$= -M, $-R_3$ oder $-R_5$
M'= O, $NR_3$

wobei $R_3$ und $R_4$=Wasserstoff, Alkyl, Aryl, Hetaryl, Aralkyl oder wobei $R_3$ und $R_4$ zusammen für gegebenenfalls durch NH oder O unterbrochenes $C_4$-$C_6$-Alkylen stehen,

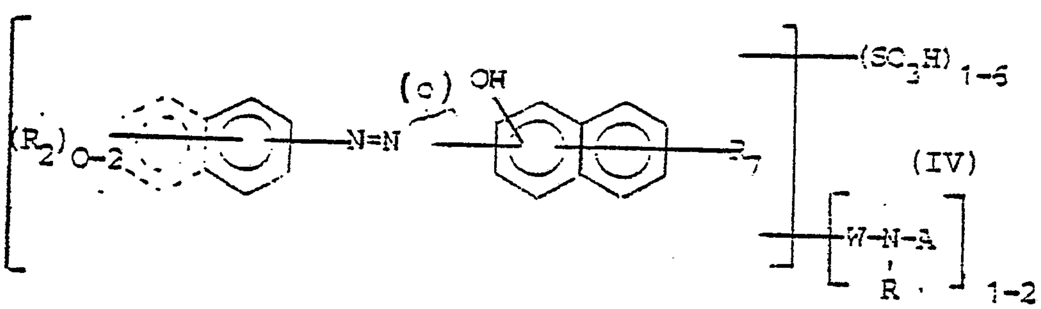

(IV)

$R_7$= Wasserstoff, $-NR_3-CO-R_8$, $-NR_3-SO_2-R_8$
o = ortho-ständige Stellung der jeweiligen Reste

wobei $R_3$ = Wasserstoff, Alkyl, Aryl, Hetaryl, Aralkyl,
$R_8$ = Alkyl, Aryl, Hetaryl oder Aralkyl,

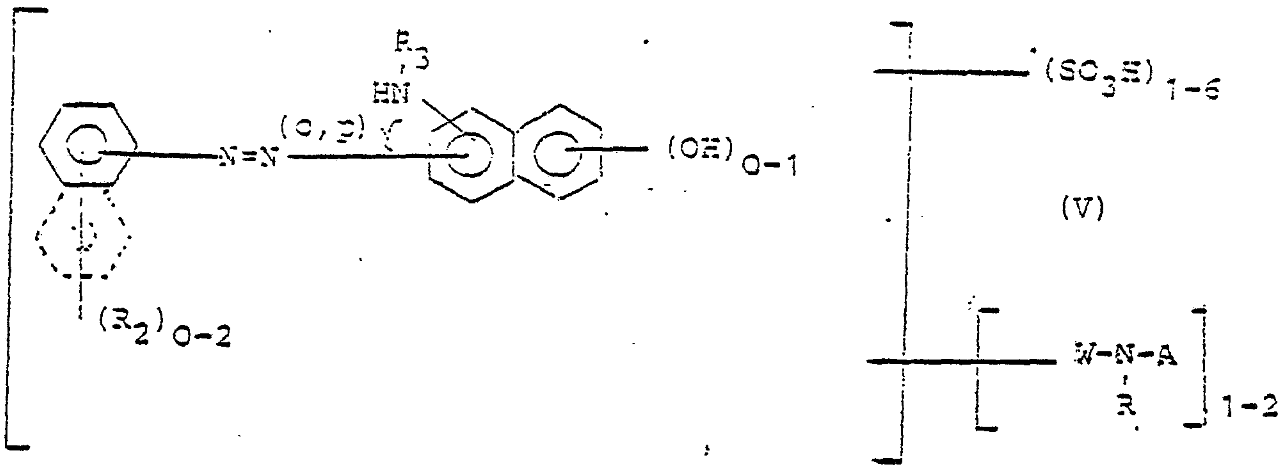

(V)

(o,p) = ortho- oder paraständige Stellung der jeweiligen Reste

wobei $R_2$ = Alkyl, Alkoxy, Halogen (insbesondere Cl)
$R_3$ = Wasserstoff, Alkyl, Aryl, Hetaryl oder Aralkyl

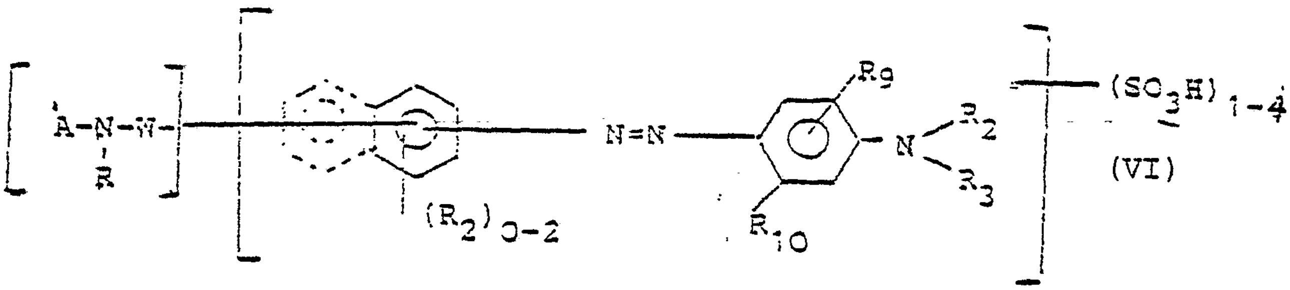

(VI)

$R_9$ und $R_{10}$ = Wasserstoff, $R_8$, $-OR_3$, Halogen, $-NR_3R_4$, $-COOR_3$, $-NR_3-CO-R_4$, $-O-CO-R_4$, $-NR_3-SO_2-R_4$, $-O-SO_2-R_4$, $-NR_3-CO-NR_3R_4$

wobei $R_3$ und $R_4$ = Wasserstoff, Alkyl, Aryl, Hetaryl, Aralkyl oder wobei $R_3$ und $R_4$ zusammen für gegebenenfalls durch NH oder O unterbrochenes $C_4$-$C_6$-Alkylen stehen
und $R_8$ = Alkyl, Aryl, Hetaryl oder Aralkyl

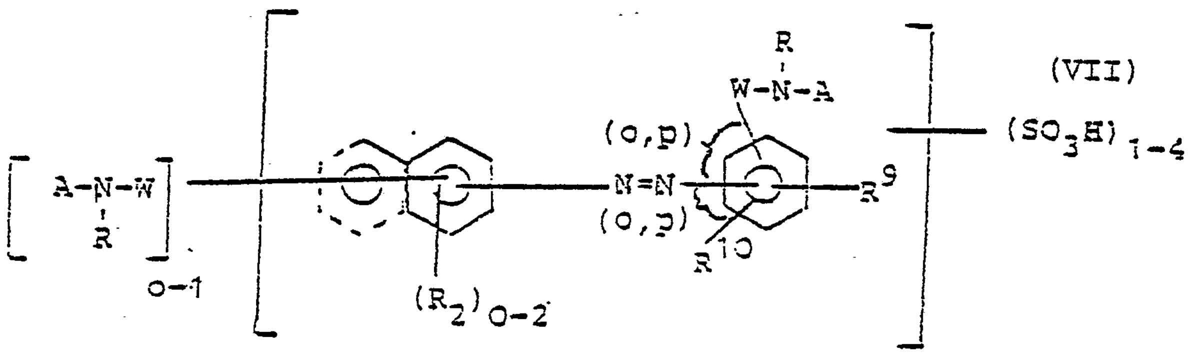

(VII)

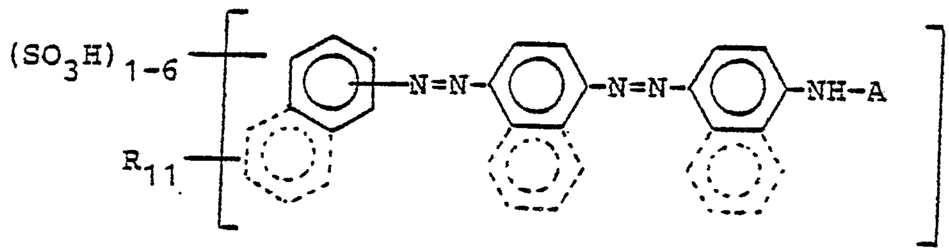

(VIIa)

$R_{11} = R_9, C_1-C_4$-Alkyl

wobei (o,p) = Ortho- oder paraständige Stellung der jeweiligen Reste

$R_2$ = Alkyl, Alkoxy, Halogen und wobei $R_9$ und $R_{10}$ die unter Formel VI angegebene Bedeutung haben.

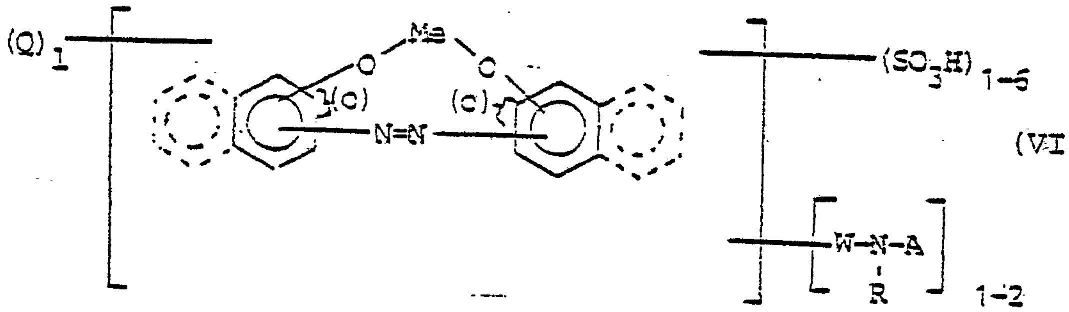

(VIII)

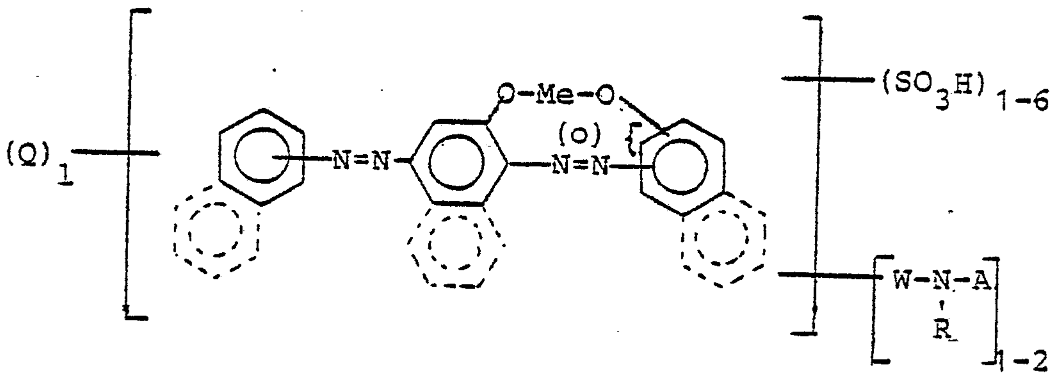

(VIIIa)

wobei (o) = orthoständige Stellung der jeweiligen Reste

Me = Cu, Cr

Q = Alkyl, Alkoxy, Halogen, $-NO_2$, Acylamino wie $CH_3-CO-NR_3-$, $-COOR_3$, $-CONR_3R_4$, $-SO_2-NR_3R_4$

l = 0-4

und wobei $R_3$ und $R_4$ die unter Formel (VI) angegebene Bedeutung haben.

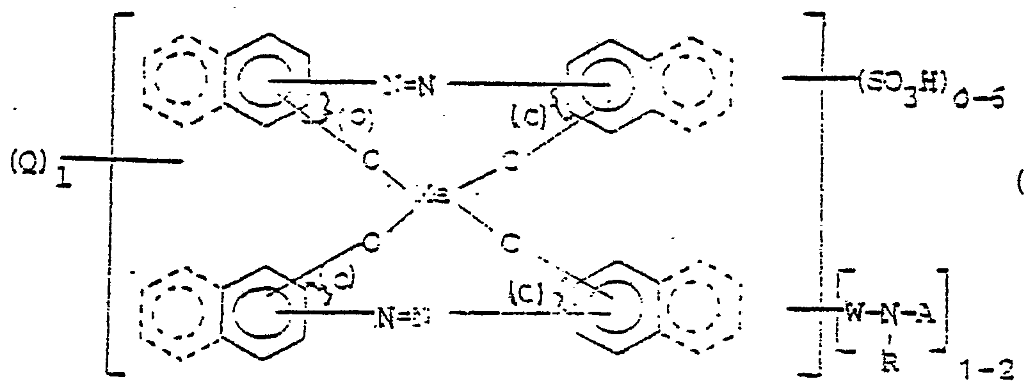

(IX)

Me. = Co, Cr

l = 0-8

(o) = orthoständige Stellung der Substituenten

Q = Alkyl, Alkoxy, Halogen, $NO_2$, Acylamino wie $CH_3$-CO-$NR_3$-, -$COOR_3$, -$CONR_3R_4$, -$SO_2$-$NR_3R_4$

und wobei $R_3$ und $R_4$ die unter Formel (VI) angegebene Bedeutung haben.

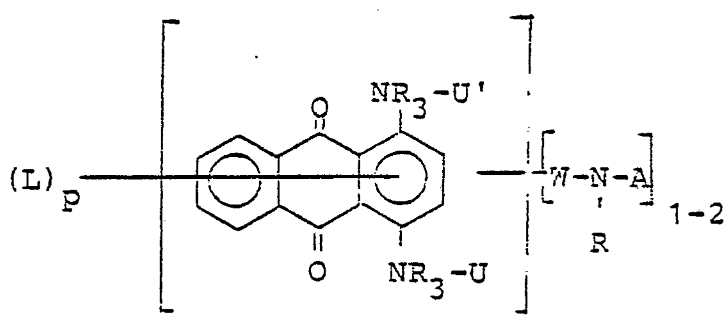

(X)

p = 0 - 4,

L = Substituent, wie Sulfo oder Carboxy,

U = gegebenenfalls durch O oder S unterbrochener, aliphatischer, cycloaliphatischer, araliphatischer oder aromatischer Rest

U' = Wasserstoff oder U

$R_3$ = Wasserstoff, Alkyl, Aryl, Hetaryl oder Aralkyl

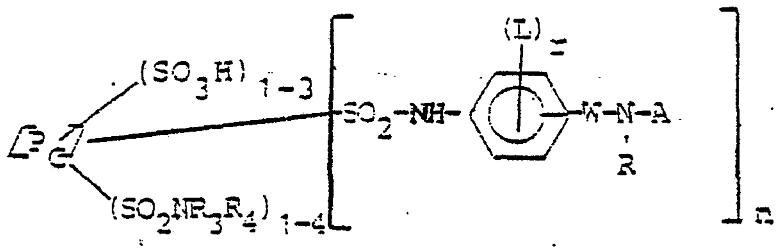

(XI)

$P_C$= Cu/Ni-Phthalocyanin-Rest
L = Substituent, insbesondere Sulfo oder Carboxy
r = 0 - 4

und wobei $R_3$ und $R_4$ die unter Formel (VI) angegebene Bedeutung haben.

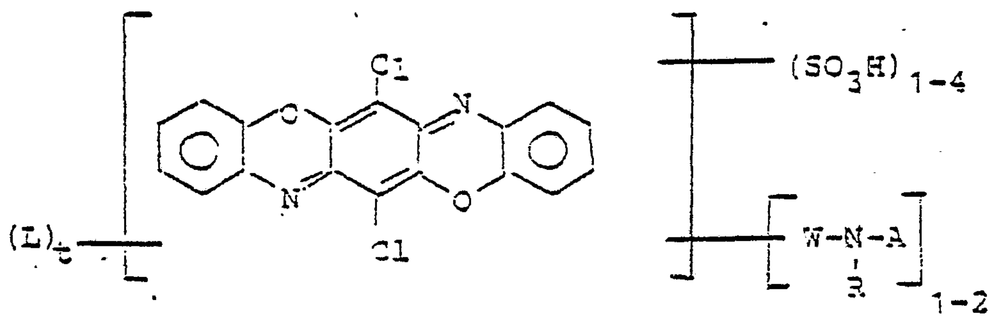

(XII)

L = Substituent, insbesondere Halogen, $C_{1-4}$-Alkyl, $C_{1-4}$-Alkoxy oder Carboxy
t = 0 bis 3

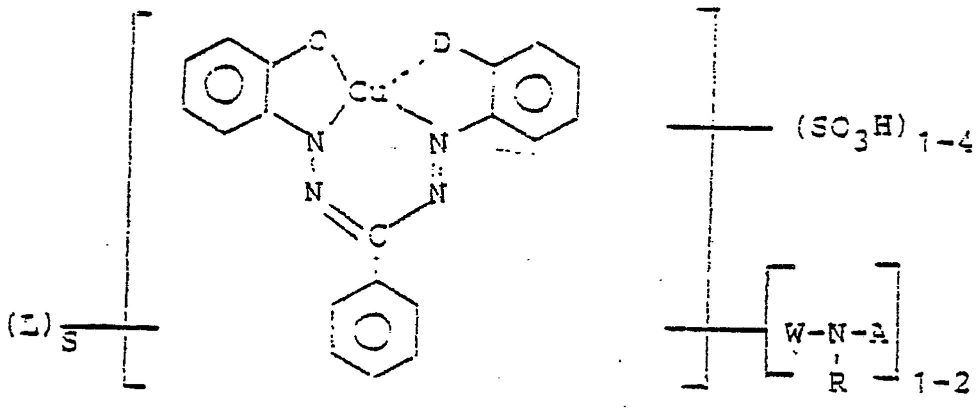

(XIII)

L = Substituent, insbesondere Halogen $C_{1-4}$-Alkyl, $C_{1-4}$Alkoxy, Alkylsulfonyl, Aminosulfonyl, unkondensierte Phenylreste oder Carboxy

S = 0-6

D = zur Komplexbildung befähigte Gruppe wie -OH, -COOH oder $-SO_3H$

6. Farbstoffe gemäß Anspruch 1 bis 5 der Formeln:

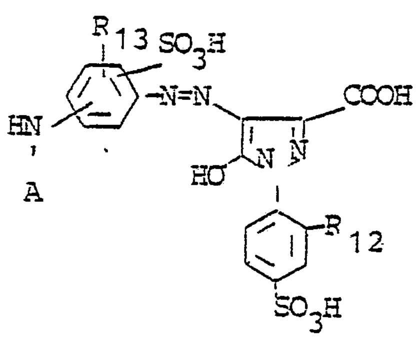

XIV

wobei

$R_{12}$ = H, $CH_3$, Cl

$R_{13}$ = H, $CH_3$, Cl

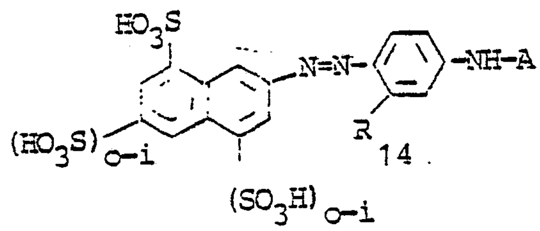

(XV)

wobei $R_{14}$ = H, $CH_3$, $OCH_3$, $NHCOCH_3$, $NH-CONH_2$

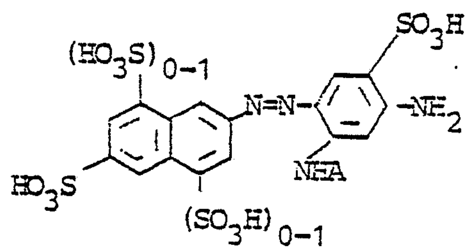

(XVI)

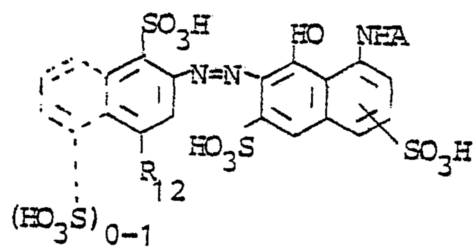

(XVII)

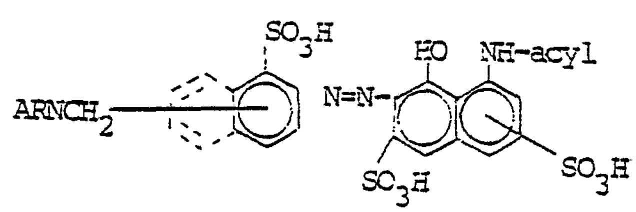

(XVIII)

worin acyl = Acylrest, insbesondere Formyl, $C_1$-$C_4$-Alkylcarbonyl, Arylcarbonyl und bevorzugt gegebenenfalls durch $C_1$-$C_4$-Alkyl, OH, $SO_3H$, COOH substituiertes Phenylcarbonyl.

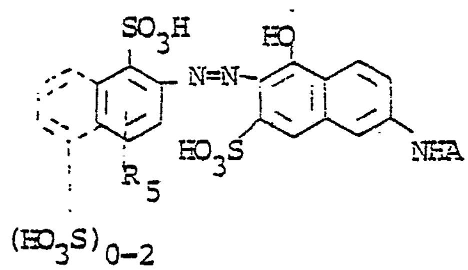

(XIX)

$R_{15}$ = $R_{13}$, $CH_3$, $C_1$-$C_4$-Alkoxy, Acylamino

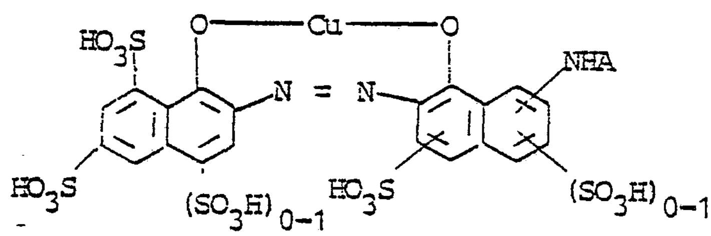

(XX)

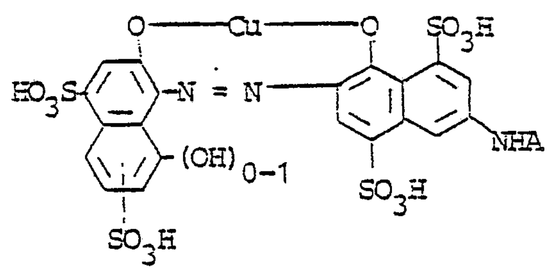

(XXI)

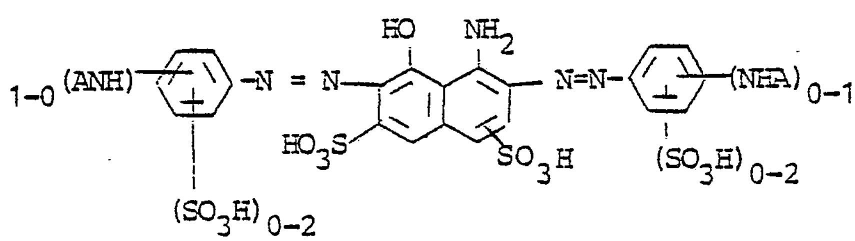

(XXII)

wobei in der Formel XXII der Rest ANH- im Molekül nur einmal vorhanden sein soll,

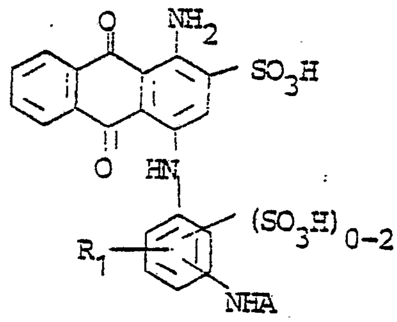

(XXIII)

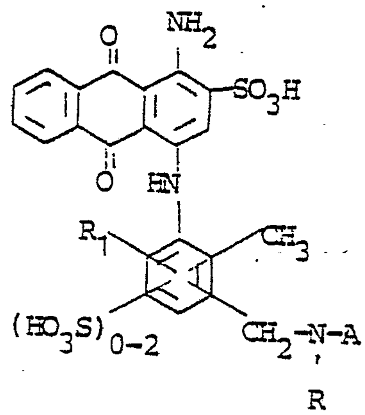

(XXIV)

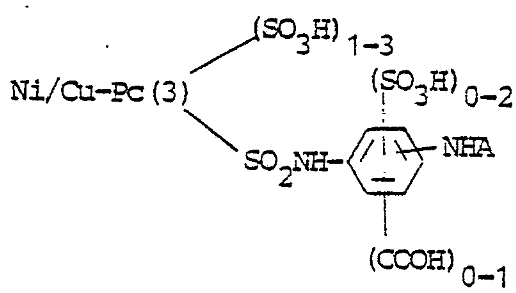

(XXV)

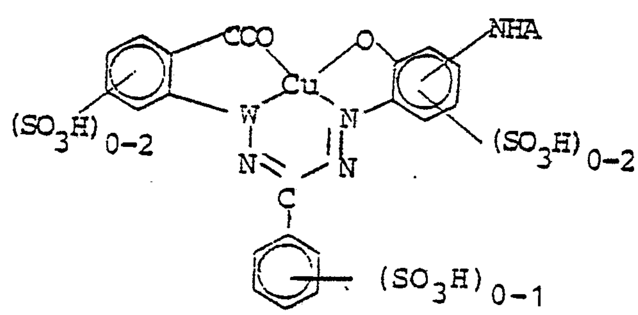

(XXVI)

7. Verfahren zur Herstellung von Reaktivfarbstoffen der Formel

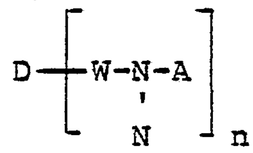

(I)

worin D, W, R, A und n die im Anspruch 1 angegebene Bedeutung besitzen,

dadurch gekennzeichnet, daß man einen Farbstoff der Formel

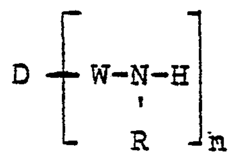

mit der äquimolaren Menge eines 2,4-Difluorpyrimindin-Derivats der Formel

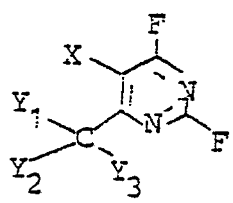

wobei X, $Y_1$, $Y_2$ und $Y_3$ die im Anspruch 1 angegebene Bedeutung besitzen, im wäßrigen oder organisch-wäßrigen Medium bei 0-70°C in Anwesenheit von säurebindenden Mitteln umsetzt und den Reaktivfarbstoff gemäß Formel (I) nach an sich bekannten Methoden isoliert.

8. Verwendung der Farbstoffe der Ansprüche 1 bis 6 zum Färben und Bedrucken von hydroxylgruppenhaltigen und/oder stickstoffhaltigen Materialien.

9. Halogenpyrimidin-Verbindungen der allgemeinen Formel

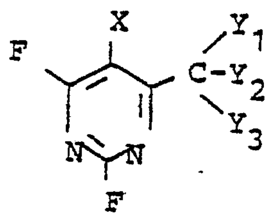

(Ia)

in welcher

X Wasserstoff, Chlor oder Brom,

$Y_1$, $Y_2$ Wasserstoff, Chlor oder Brom und

$Y_3$ Chlor, Brom oder Fluor

bedeuten.

10. Halogenpyrimidin-Verbindungen gemäß Anspruch 1 der Formeln

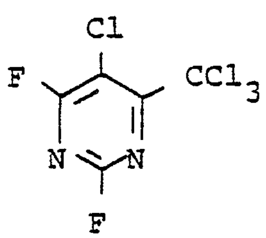

,

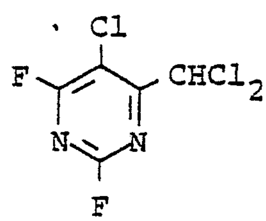

,

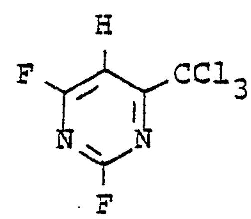

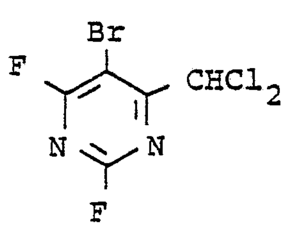

,

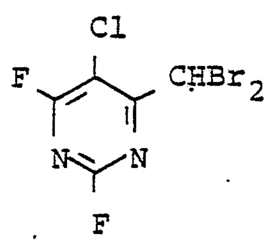

,

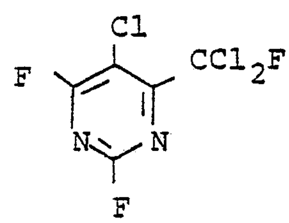

11. Verfahren zur Herstellung von Halogenpyrimidin-Verbindungen der allgemeinen Formel

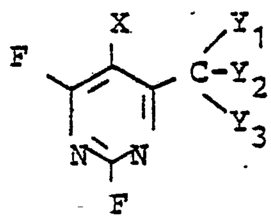

(Ia)

in welcher

X Wasserstoff, Chlor oder Brom,

$Y_1$,$Y_2$ Wasserstoff, Chlor oder Brom und

$Y_3$ Chlor, Brom oder Fluor

bedeutet,

dadurch gekennzeichnet, daß man Chlorpyrimidin-Derivate der Formel

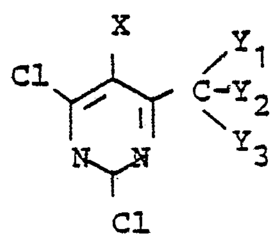

(IIa)

in welcher

X,$Y_1$,$Y_2$ und $Y_3$ die oben angegebene Bedeutung haben,

mit Fluorwasserstoff in wasserfreier Flußsäure unter Druck umgesetzt oder mit einem Alkalifluorid in einem polaren organischen Löaungsmittel umgesetzt werden.

12. Verbindungen der Formel

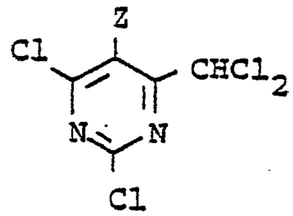

worin

Z H, Cl oder Br

bedeutet.